# EUROPEAN PATENT APPLICATION

(11) **EP 4 257 612 A1**
(43) Date of publication of application: **11.10.2023**
(21) Application number: 21900076.7
(22) Date of filing: 02.12.2021
(51) Int. Cl.: C07K 16/46, C12N 15/13, A61K 39/395, A61K 47/68, A61K 45/00, A61K 51/10, A61P 35/00

(54) **DEVELOPMENT OF NEW TUMOR ENGAGER THERAPEUTIC DRUG AND USE THEREOF**

(30) Priority: 02.12.2020 CN 202011402162
(71) Applicant: Keymed Biosciences Co.,Ltd., Chengdu, Sichuan 610219 (CN)
(72) Inventor: CHEN, Bo, Chengdu, Sichuan 610219 (CN); XU, Gang, Chengdu, Sichuan 610219 (CN); SONG, Qin, Chengdu, Sichuan 610219 (CN)
(74) Representative: Elzaburu S.L.P.
(86) International application number: PCT/CN2021/135153
(87) International publication number: WO 2022/117050

(57) **Abstract**

The present disclosure relates to the development of a new tumor engager therapeutic drug and the use thereof. The therapeutic drug contains a bispecific antibody that binds to GPC3 and CD3, wherein the bispecific antibody contains a first binding domain that binds to GPC3 on the surface of a target cell and a second binding domain that binds to CD3 on the surface of a T cell. The bispecific antibody can inhibit tumor growth at a very low dose and effectively inhibit the growth of a transplanted tumor in an immune reconstitution mouse. A toxicological study conducted in cynomolgus monkeys also shows that animals have good tolerance to the bispecific antibody, and the efficacy and safety of the bispecific antibody are superior to those of similar antibodies.

## Description

### Field of the invention

The present disclosure relates to a bispecific antibody and use thereof, in particular to the development of a new tumor engager therapeutic drug and the use thereof.

### Background

Glypican-3 (GPC3, also known as DGSX, GTR2-2, MXR7, OCI-5, SDYS, SGB, SGBS or SGBS1) is a phosphatidylinositol proteoglycan, belonging to the heparan sulfate proteoglycans (HSPGs) family, that is anchored to the outer surface of cell membranes by the glycosyl phosphatidyl inositol (GPI). The core of the GPC3 protein is composed of 580 amino acids with a molecular weight of about 65 kDa. Protease Furin cleaves between 358Arg-359Ser (355R-356Q-357Y-358R-359S) to form two subunits. Two heparan sulfate polysaccharide chains are connected to the membrane-proximal C-terminal subunit, which is an important component of the extracellular matrix and may be involved in the activation of cells through multiple signaling pathways such as Wnt, Hh (Hedgehog), and fibroblast growth factor (FGF) (Li N et al, Glypicans as Cancer Therapeutic Targets, Trends Cancer. 2018). GPC3 is expressed in various fetal tissues (liver, lung, kidney, and placenta). After birth, the GPC3 gene is no longer expressed due to DNA methylation modification, but in many tumor tissues, such as 66-90% of hepatocellular carcinoma, 54-65% of lung squamous carcinoma, 16-64% of small cell lung cancer, 20-30% of digestive tract tumors (esophageal cancer, gastric cancer, cardiac cancer) and some other types of cancer cells, the GPC3 gene resumes expression (Kawaida M et al, Diffuse and canalicular patterns of glypican-3 expression reflect malignancy of hepatocellular carcinoma, Pathol Int. 2019 Mar;69(3):125-134; Yu X et al, Differential expression of glypican-3 (GPC3) in lung squamous cell carcinoma and lung adenocarcinoma and its clinical significance, Genet Mol Res. 2015 Aug 28;14(3):10185-92; Ishiguro T et al, An anti-glypican 3/CD3 bispecific T cell-redirecting antibody for the treatment of solid tumors, Sci Transl Med. 2017 Oct 4;9(410):eaal4291; and Baumhoer D et al, Glypican 3 expression in human nonneoplastic, preneoplastic, and neoplastic tissues: a tissue microarray analysis of 4,387 tissue samples, Am J Clin Pathol. 2008 Jun;129(6):899-906). Diffuse expression of GPC3 on pathological sections is correlated with low differentiation of HCC cells and disease prognosis. Recent reports have shown that GPC3 is a tumor stem cell marker, which can effectively kill tumor stem cells and inhibit tumor growth in mice via GPC3-specific cytotoxic T lymphocytes (CTL) (Okada M et al, Selective elimination of undifferentiated human pluripotent stem cells using pluripotent state-specific immunogenic antigen Glypican-3, Biochem Biophys Res Commun. 2019 Apr 9;511(3):711-717).

A T-cell bispecific antibody (or T-cell adaptor) is a specially constructed antibody molecule that recognizes a target cell surface antigen (antigen arm) at one end and binds to a T-cell CD3 receptor (CD3 arm) at the other end. Using antibodies that target the ε chain of CD3, CD3 on T cells is allowed to aggregate in a manner similar to TCR/peptide/HLA, thereby activating T cells and killing tumors. The BCMA x CD3 bispecific molecule is a T-cell bispecific (TCB) antibody that targets BCMA expressed on myeloma cells and the CD3ε chain (CD3e) present in T cells. The mechanism of action of the BCMA x CD3 bispecific molecule involves binding to both BCMA + myeloma cells and CD3+ T cells, resulting in T cell activation and T cell-mediated cell killing.

Codrituzumab is the first therapeutic monoclonal antibody targeting GPC3 and has shown significant anti-tumor activity in a mouse tumor model. However, in Phase II clinical trial (NCT01507168), Codrituzumab showed no significant difference in disease progression and overall survival compared to the control group (Abou-Alfa GK et al, Randomized phase II placebo controlled study of codrituzumab in previously treated patients with advanced hepatocellular carcinoma, J Hepatol. 2016 Aug;65(2):289-95). Studies have shown that Codrituzumab may bring clinical benefits to patients with high GPC3 expression in tumors or high CD16 affinity mutations (Chen G et al,Combining expression of GPC3 in tumors and CD16 on NK cells from peripheral blood to identify patients responding to codrituzumab, Oncotarget. 2018 Jan 2;9(12):10436-10444). ERY974 is an anti-GPC3/CD3 bispecific antibody that can effectively kill various GPC3 positive tumor cells through T cell-mediated killing effects, with activity superior to monoclonal antibodies. However, due to its strong side effects, clinical research was suspended in 2018; in 2019, after phase I clinical trials were restarted, the combination of IL-6 antibody Tocilizumab was used to avoid cytokine release syndrome.

### Summary

In order to solve the problems of the prior art, the present disclosure provides a new GPC3 antibody having a high affinity to the GPC3 protein. Also, the present disclosure provides new GPC3-CD3 κλ bispecific antibodies formed by GPC3 antibodies and CD3 antibodies. GPC3 antibodies can mediate antibody-dependent cell-mediated cytotoxicity (ADCC) against target cells, and GPC3 antibodies can inhibit GPC3-induced endocytosis. GPC3-CD3 κλ bispecific antibody adopts full-length IgG configuration. Through introducing charge and affinity optimization, the κλ bispecific antibody preferentially localizes to GPC3+ tumor tissue. It can recruit activated T cells at low concentrations and produce effective killing to target cells, while it does not activate T cells in the absence of target cells. At the same time, κλ bispecific antibodies do not bind to receptors such as FcγRI, Fcγ RIIA, and Fcγ RIIIA, reducing the risk of cytokine storms. The pharmacodynamic test showed that the novel GPC3-CD3 κλ bispecific antibody could inhibit the growth of the tumor at a very low dose and effectively inhibit the growth of transplanted tumors in an immune reconstitution mouse. A toxicological study conducted in cynomolgus monkeys also showed that the animals have good tolerance to the new GPC3-CD3 κλ bispecific antibody, and the efficacy and safety of the new GPC3-CD3 κλ bispecific antibody are superior to those of similar antibodies.

In one aspect, the present disclosure provides an antibody or antigen-binding portion thereof that binds GPC3.

In one aspect, the present disclosure provides a bispecific antibody or antigen-binding portion thereof.

In one aspect, the present disclosure provides a nucleic acid encoding the bispecific antibody or antigen-binding portion thereof according to the previous aspects.

In one aspect, the present disclosure provides a vector comprising the nucleic acid according to the previous aspects.

In one aspect, the present disclosure provides a cell comprising the nucleic acid or vector according to the previous aspects.

An antibody or antigen binding portion thereof according to any one of the previous aspects, wherein the antibody or antigen binding portion thereof is humanized.

In an aspect, the present disclosure provides a pharmaceutical composition or kit containing the antibody or the antigen-binding portion thereof or the nucleic acid encoding the antibody or the antigen-binding portion thereof of any one of the aforementioned aspects and a pharmaceutically acceptable carrier.

In an aspect, the present disclosure provides an antibody-drug conjugate containing the antibody or the antigen-binding portion thereof of any one of the aforementioned aspects, or a bispecific or multispecific molecule that is covalently attached to a therapeutic moiety.

In an aspect, the present disclosure provides a method for treating a GPC3-associated disease, which includes the following steps: administering a therapeutically effective amount of antibody or antigen-binding fragment thereof, nucleic acid, vector, cell and/or pharmaceutical composition of any one of the aforementioned aspects to a mammal.

In an aspect, the present disclosure provides use of the antibody or the antigen-binding fragment thereof, the nucleic acid, the vector, the cell and/or the pharmaceutical composition of any one of the aforementioned aspects in the preparation of a drug or kit for treating a GPC3-associated disease in a mammal.

The antibody of the present disclosure can be used in a variety of applications such as the detection of GPC3 protein, and diagnosis, treatment, or prevention of GPC3-associated diseases.

### Brief Description of the Drawing

FIG. 1 shows non-reduced SDS-PAGE results for recombinant GPC3 proteins.
FIG. 2 shows flow cytometry results for GPC3 stably-transfected cells.
FIG. 3 shows SDS-PAGE results for recombinant CD3εγ-Fc proteins.
FIG. 4 shows flow cytometry results for GPC3 antibodies and human GPC3 stably-transfected cells.
FIG. 5 shows the binding of antibody 49G8 to different species of GPC3 antigens.
FIG. 6 shows the ADCC effect mediated by antibody 49G8.
FIG. 7 shows the binding of the humanized antibodies to the GPC3 antigens.
FIG. 8 shows the binding of the humanized antibodies to the GPC3 stably-transfected cells.
FIG. 9 shows the Octet affinity assay for the humanized antibodies.
FIG. 10 shows the SPR affinity assay for humanized antibodies.
FIG. 11 shows the results of endocytosis of the humanized antibodies.
FIG. 12 shows the binding of the CD3 humanized antibodies to human CD3 εγ antigens.
FIG. 13 shows the binding of the CD3 humanized antibodies to Jurkat cells.
FIG. 14 shows a comparison of the affinity of CD3 humanized antibodies to human and monkey CD3 εγ antigens.
FIG. 15 shows the binding of GPC3 x CD3 κλ bispecific antibodies to GPC3 stably-transfected cells.
FIG. 16 shows the binding of GPC3 x CD3 κλ bispecific antibodies to HepG2 cells.
FIG. 17 shows the binding of GPC3 x CD3 κλ bispecific antibodies to Jurkat cells.
FIG. 18 shows the binding of GPC3 x CD3 κλ bispecific antibodies to peripheral blood T cells.
FIG. 19 shows the TDCC effect mediated by the GPC3 x CD3 κλ bispecific antibodies, with FIG. 19A showing killing of HepG2 cells and FIG. 19B showing activation of T cells.
FIG. 20 shows the effect of the GPC3 x CD3 κλ bispecific antibodies on the T cell NFAT signaling pathway.
FIG. 21 shows the activation of PBMC by the GPC3 x CD3 κλ bispecific antibodies.
FIG. 22 shows the binding of the GPC3 x CD3 κλ bispecific antibodies to FcyR activating receptors.
FIG. 23 shows the inhibitory effect of the GPC3 x CD3 κλ bispecific antibody in an immune reconstitution mouse HepG2 xenograft model.
FIG. 24 shows the inhibitory effect of the GPC3 x CD3 κλ bispecific antibody in a CD3 humanized murine Hepa1-6/hGPC3 xenograft model.

### Detailed description of the invention.

### I. Definitions

In the present disclosure, unless otherwise specified, scientific and technical terms used herein have the meanings commonly understood by those skilled in the art. Moreover, protein and nucleic acid chemistry, molecular biology, cell and tissue culture, microbiology, and immunology-related terms and laboratory procedures used herein are terms and conventional procedures widely used in corresponding arts. Meanwhile, in order to better understand the present disclosure, definitions and explanations of relevant terms are provided below.

As used herein, the term "GPC3" may refer to the concept of GPC3 itself and any variants, isoforms, and paralogs thereof, which are present together in animals and preferably in humans.

The term "human GPC3" refers to the GPC3 of human origin.

The terms "anti-GPC3 antibody" and "GPC3-binding antibody" refer to an antibody that can bind to GPC3 with sufficient affinity such that the antibody is useful in targeting GPC3 as a diagnostic and/or therapeutic agent. In an embodiment, the binding ability of the anti-GPC3 antibody to unrelated non-GPC3 protein is less than about 10% of the binding ability of the antibody to GPC3 that is determined by, for example, radioimmunoassay (RIA). In certain embodiments, the GPC3-binding antibody has a dissociation constant (Kd) ≤ 1 µM, ≤100 nM, ≤10 nM, ≤1 nM, ≤0.1 nM, ≤0.01 nM, or ≤0.001 nM (e.g., 10⁻⁸ M or less, e.g., 10⁻⁸ M to 10⁻¹³ M, e.g., 10⁻⁹M to 10⁻¹³M). In certain embodiments, anti-GPC3 antibodies bind to GPC3 epitopes conserved among GPC3 from different species.

"CD3" refers to any natural CD3 of any vertebrate origin including mammals such as primates (e.g., humans), non-human primates (e.g., Macaca fascicularis), and rodents (e.g., mice and rats), unless otherwise specified. The term encompasses "full-length", unprocessed CD3, and any form of processed CD3 from a cell. The term also encompasses naturally occurring variants of CD3, such as splice variants and allelic variants. In one embodiment, the CD3 is human CD3, in particular the epsilon subunit (CD3ε) of human CD3. An amino acid sequence of human CD3ε is shown in UniProt (www.uniprot.org) accession number P07766 (version 144) or NCBI (www.ncbi.nlm.nih.gov/) RefSeq NP_000724.1. An amino acid sequence of cynomolgus monkey[Macaca fascicularis] CD3ε is shown in NCBI GenBank NO. BAB71849.1.

The term "cell surface" is used according to its normal meaning in the art and thus includes the outside of a cell which is accessible through binding to proteins and other molecules.

As used herein, unless otherwise specified, the term "about" or "approximate" means within plus or minus 10% of a given value or range. In the case that integers are required, the term means rounding up or down to the nearest integer within plus or minus 10% of a given value or range.

With respect to antibody chain polypeptide sequences, the phrase substantially identical is to be understood as at least 60%, 65%, 70%, 75%, 80%, 85%, 90%, 95%, 96%, 97%, 98%, 99% or higher sequence identity of an antibody chain to a reference polypeptide sequence. With respect to nucleic acid sequences, the term is to be understood as at least greater than 60%, 65%, 70%, 75%, 80%, 85%, 90%, 95%, 96%, 97%, 98%, 99% or higher sequence identity of a nucleotide sequence to a reference nucleic acid sequence.

The sequence "identity" has a meaning recognized in the art, and the percentage of sequence identity between two nucleic acids or polypeptide molecules or regions can be calculated by disclosed technologies. The sequence identity can be measured along the full length of polynucleotides or polypeptides or regions of the molecules. There are many methods for measuring the identity between two polynucleotides or polypeptides, but the term identity is well known to the skilled person (Carrillo, H. & Lipman, D., SIAM J Applied Math 48:1073 (1988)).

A "substitution" variant refers to a variant obtained by removing at least one amino acid residue from a natural sequence and inserting a different amino acid at the same site. The substitution may be a single substitution, that is, only one amino acid in the molecule is substituted, or the substitution may be multiple substitutions, that is, two or more amino acids in the same molecule are substituted. The multiple substitutions may be at consecutive sites. Similarly, an amino acid can be substituted with multiple residues, and thus such a variant includes substitution and insertion. An insertion variant is a variant obtained by inserting one or more amino acids immediately adjacent to an amino acid at a specific site on a natural sequence. Immediately adjacent to an amino acid means linking with the α-carboxyl or α-amino functional group of the amino acid. A "deletion" variant refers to a variant obtained by removing one or more amino acids from a natural amino acid sequence. Usually, a deletion variant has one or two amino acid deletions in a specific region of its molecule.

With respect to variable domains of antibodies, the term "variable" refers to certain portions of relevant molecules that have extensive sequence differences between antibodies, and are used for the specific recognition and binding to a specific antibody against its specific target. However, the variability is not uniformly distributed throughout the variable domains of antibodies. The variability is concentrated in three segments known as complementarity-determining regions (CDRs; i.e., CDR1, CDR2, and CDR3) or hypervariable regions, which are located within variable domains of light chains and heavy chains. A more conservative portion within the variable domain is referred to as a framework region (FR) or framework sequence. Each variable domain of a natural heavy chain or light chain includes four FRs, which mainly adopt a β-folded configuration and are linked together through three CDRs, the CDRs form a loop, and the loop is linked with the β-folded configuration and forms a part of the β-folded configuration in certain cases. CDRs of each chain are usually linked adjacently by an FR region and contribute to the formation of a target binding site (epitope or determinant) of an antibody by virtue of CDRs from other chains. As used herein, the numbering of amino acid residues of immunoglobulin is based on the numbering system of amino acid residues of immunoglobulin developed by Kabat et al., unless otherwise specified. A CDR may have the ability to specifically bind to a cognate epitope.

As used herein, an "antibody fragment" or "antigen-binding fragment" of an antibody refers to any portion of a full-length antibody, which is shorter than the full-length antibody, at least contains a partial variable region (e.g., one or more CDRs and/or one or more antibody-binding sites) of the antibody that binds to an antigen, and thus retains the binding specificity and at least partial specific binding ability of the full-length antibody. Therefore, the antigen-binding fragment refers to an antibody fragment that contains an antigen-binding portion binding to the same antigen as the antibody from which the antibody fragment is derived. The antibody fragment includes an antibody derivative that is produced by enzymatic treatment of the full-length antibody, and a synthesized derivative such as a recombinant derivative. The antibody includes the antibody fragment. Examples of the antibody fragment include but are not limited to, Fab, Fab', F(ab')2, single-stranded Fv(scFv), Fv, dsFv, double antibody, Fd, and an Fd' fragment, and other fragments, including a modified fragment. The fragment may include multiple chains that are linked together through, for example, disulfide bonds and/or peptide adaptors. The antibody fragment generally contains at least or about 50 amino acids, typically at least or about 200 amino acids. The antigen-binding fragment includes any antibody fragment, which is inserted into an antibody framework (e.g., by replacing a corresponding region) to obtain an antibody specifically binding to (that is, with a binding constant Ka of about 10⁷-10⁸M⁻¹) an antigen. A "functional fragment" or "analog of an anti-GPC3 antibody" is a fragment or analog that prevents or substantially reduces the ability of the receptor to bind a ligand or initiate signal transduction. As used herein, the functional fragment generally has the same meaning as an antibody fragment, and with respect to an antibody, it may refer to a fragment that can prevent or substantially reduce the ability of the receptor to bind to a ligand or initiate signal signaling, such as Fv, Fab, F(ab')₂, etc. The Fv fragment consists of a dimer (V_{H}-V_{L} dimer) formed by the non-covalent binding of a variable domain of a heavy chain and a variable region of a light chain. In this configuration, three CDRs of each variable domain interact to define a target binding site on the surface of the V_{H}-V_{L} dimer, as in the case of an intact antibody. The six CDRs together confer the target binding specificity to the intact antibody. However, even a single variable domain (or half of Fv containing 3 target-specific CDRs only) can still have the ability to recognize and bind to a target.

As used herein, the term "bispecific antibody (BsAb)" refers to an antibody and/or antigen-binding molecule that can specifically bind to two different antigenic determinants, and usually, the bispecific antibody and/or antigen-binding molecule contains two antigen-binding sites each of which is specific to different antigenic determinants. In certain embodiments, the bispecific antibody and/or antigen-binding molecule can bind to two antigenic determinants at the same time, particularly two antigenic determinants that are expressed on two different types of cells.

As used herein, a "monoclonal antibody" refers to a population of identical antibodies, meaning that each individual antibody molecule in the population of monoclonal antibodies is identical to the other antibody molecules. This characteristic is in contrast to that of a polyclonal population of antibodies, which contain antibodies with a variety of different sequences. Monoclonal antibodies can be prepared by many well-known methods (Smith et al. (2004) J.Clin. Pathol.57, 912-917; and Nelson et al., J Clin Pathol (2000), 53, 111-117). For example, monoclonal antibodies can be prepared by immortalizing B cells, for example, fusing with myeloma cells to produce a hybridoma cell line or infecting B cells with a virus such as EBV Recombination can also be used to prepare antibodies in vitro from clonal populations of host cells by transforming the host cells with plasmids carrying artificial sequences of nucleotides encoding the antibodies As used herein, the term "hybridoma" or "hybridoma cell" refers to a cell or cell line (usually myeloma or lymphoma cells) produced by fusing lymphocytes that produce antibodies with cancer cells that do not produce antibodies. As is known to those of ordinary skill in the art, a hybridoma can proliferate and continuously supply and produce specific monoclonal antibodies. The method for producing a hybridoma is known in the art. When the term "hybridoma" or "hybridoma cell" is referred to, it also includes a subclone and progeny cells of the hybridoma.

As used herein, a full-length antibody is an antibody that has two full-length heavy chains (e.g., VH-CH1-CH2-CH3 and VH-CH1-CH2-CH3-CH4), two full-length light chains (VL-CL), and hinge regions, such as an antibody produced naturally by antibody-secreting B cells and a synthesized antibody with the same domains.

The term "chimeric antibody" refers to an antibody that contains a variable region sequence derived from a species and a constant region sequence derived from another species. For example, the variable region sequence is derived from a mouse antibody, and the constant region sequence is derived from a human antibody.

A "humanized" antibody refers to a non-human (e.g., mouse) antibody form, which is a chimeric immunoglobulin, immunoglobulin chain, or a fragment thereof (e.g., Fv, Fab, Fab', F(ab')2 or other antigen-binding subsequences of an antibody), containing the minimal sequence derived from non-human immunoglobulin. Preferably, the humanized antibody is a human immunoglobulin (recipient antibody), and residues in a complementary determining region (CDR) of the recipient antibody are substituted with CDR residues in a non-human species, such as a mouse, a rat, and a rabbit, that has the desired specificity, affinity, and ability.

In addition, during humanization, amino acid residues within CDR1, CDR2, and/or CDR3 regions of VH and/or VL may be mutated, so as to improve one or more binding characteristics (e.g., the affinity). A mutation can be introduced, for example, by performing PCR-mediated mutation, the influence of which on antibody binding or other functional characteristics can be assessed using the in vitro or in vivo assay described herein. Usually, a conservative mutation is introduced. Such a mutation may be an amino acid substitution, addition, or deletion. In addition, there are usually no more than one or two mutations within a CDR. Therefore, the humanized antibody of the present disclosure also covers an antibody of which a CDR contains 1 or 2 amino acid mutations.

As used herein, the term "CDR" refers to a complementarity-determining region, and each heavy chain or light chain of a known antibody molecule has 3 CDRs. CDR is also referred to as a hypervariable region that exists in a variable region of each heavy chain or light chain of an antibody, and the primary structure of CDR has a site with very high variability. In this description, CDRs of a heavy chain are represented by CDR1, CDR2, and CDR3 derived from the amino-terminus of the amino-terminal sequence of the heavy chain and CDRs of a light chain are represented by CDR1, CDR2, and CDR3 derived from the amino-terminus of the amino-terminal sequence of the light chain. These sites are adjacent to each other in the tertiary structure and determine the specificity of an antigen to which an antibody binds.

As used herein, the term "epitope" refers to any antigenic determinant on an antigen to which a paratope of an antibody binds. An epitope determinant usually contains a chemically active surface type of a molecule, such as an amino acid and a sugar side chain, and usually has specific three-dimensional structural characteristics and specific charge characteristics.

As used herein, the "specific binding" or "immunospecific binding" of an antibody or an antigen-binding fragment thereof are interchangeable herein and refers to the ability of the antibody or the antigen-binding fragment thereof to form one or more non-covalent bonds with the same antigen through non-covalent interaction of the antibody and an antibody-binding site of an antigen. The antigen may be a separated antigen or present in tumor cells. Usually, an antibody immunospecifically binding (or specifically binding) to an antigen binds to the antigen with an affinity constant Ka of about 1 × 10⁷ M⁻¹ or 1 × 10⁸ M⁻¹ or greater (or with a dissociation constant (Kd) of 1 × 10⁷ M or 1 × 10⁸ M or less). An affinity constant can be measured by standard kinetic methods for antibody responses, such as immunoassay, surface plasmon resonance (SPR) (Rich and Myszka (2000) Curr. Opin.Biotechnol 11:54; and Englebienne (1998) Analyst. 123:1599), isothermal titration calorimetry (ITC), or other kinetic interaction assays known in the art. Patent NO. 7,229,619 describing exemplary SPR and ITC methods for calculating the binding affinity of antibodies). Instruments and methods for detecting and monitoring the rate of binding in real-time are known and commercially available (see Malmqvist (2000) Biochem.Soc.Trans. 27:335).

As used herein, the terms "polynucleotide" and "nucleic acid molecule" refer to an oligomer or polymer that contains at least two linked nucleotides or nucleotide derivatives, including deoxyribonucleic acid (DNA) and ribonucleic acid (RNA) that are usually linked together through phosphodiester bonds. As used herein, the term "nucleic acid molecule" is intended to include DNA molecules and RNA molecules. A DNA molecule may be single-stranded or double-stranded and may be cDNA.

As used herein, a separated nucleic acid molecule is a nucleic acid molecule that is separated from other nucleic acid molecules present in a natural source of the nucleic acid molecule. A "separated" nucleic acid molecule, such as a cDNA molecule, may be substantially free of other cellular materials or culture medium when prepared by recombination, or substantially free of chemical precursors or other chemical components when chemically synthesized. Exemplary separated nucleic acid molecules provided herein include a separated nucleic acid molecule encoding the antibody or antigen-binding fragment provided.

As used herein, "operably linking" of nucleic acid sequences, regions, elements or domains means that the nucleic acid regions are functionally related to each other. For example, a promoter can be operably linked to a nucleic acid encoding a polypeptide such that the promoter regulates or mediates transcription of the nucleic acid.

"Conservative sequence modifications" of the sequences in the sequence listings herein are also provided, i.e., nucleotide and amino acid sequence modifications that do not eliminate the binding of an antibody encoded by the nucleotide sequence or containing the amino acid sequence to an antigen. These conservative sequence modifications include conservative nucleotide and amino acid substitutions, nucleotide and amino acid additions, and deletions. For example, a modification can be introduced into the sequence listings herein by the standard technology (e.g., site-directed mutagenesis and PCR-mediated mutagenesis) known in the art. The conservative sequence modifications include amino acid substitutions in which amino acid residues are substituted with amino acid residues with similar side chains. Families of amino acid residues with similar side chains are defined in the art. The families include amino acids with basic side chains (e.g., lysine, arginine, and histidine), amino acids with acidic side chains (e.g., aspartic acid and glutamic acid), amino acids with uncharged polar side chains (e.g., glycine, asparagine, glutamine, serine, threonine, tyrosine, cysteine, and tryptophan), amino acids with non-polar side chains (e.g., alanine, valine, leucine, isoleucine, proline, phenylalanine, and methionine), amino acids with β branched side chains (e.g., threonine, valine, and isoleucine), and amino acids with aromatic side chains (e.g., tyrosine, phenylalanine, tryptophan, and histidine). Therefore, a predicted nonessential amino acid residue in an anti-GPC3 antibody is preferably substituted with another amino acid residue from the same side chain family. Methods for identifying nucleotide and amino acid conservative substitutions that do not eliminate binding to an antigen are well known in the art (for example, referring to Brummell et al., Biochem. 32: 1180-1187 (1993); Kobayashi et al., Protein Eng. 12(10): 879-884 (1999); Burks et al., Proc.Natl.Acad.Sci.USA 94: 412-417 (1997)).

As another option, in another embodiment, mutations can be introduced randomly along all or a portion of the anti-GPC3 antibody coding sequence by, for example, saturation mutagenesis, and the obtained modified anti-GPC3 antibodies can be screened for improved binding activity.

As used herein, "expression" refers to the process of producing a polypeptide by the transcription and translation of a polynucleotide. An expression level of a polypeptide can be assessed by any method known in the art, including, for example, methods for determining the amount of polypeptides produced by a host cell. Such methods may include, but are not limited to, quantification of polypeptides in a cell lysate by ELISA, Coomassie blue staining after gel electrophoresis, Lowry protein assay, and Bradford protein assay.

As used herein, a "host cell" is a cell for receiving, maintaining, replicating, and amplifying a vector. The host cell can also be used for expressing polypeptides encoded by the vector. When the host cell divides, a nucleic acid contained in the vector is replicated such that the nucleic acid is amplified. The host cell may be a eukaryotic cell or prokaryotic cell. Suitable host cells include but are not limited to, CHO cells, various COS cells, HeLa cells, and HEK cells such as HEK 293 cells.

As used herein, the "vector" is a replicable nucleic acid from which one or more heterologous proteins can be expressed when the vector is transformed into an appropriate host cell. The vector includes those vectors into which nucleic acids encoding polypeptides or fragments thereof can be introduced, usually by restriction digestion and ligation. The vector also includes those vectors that contain nucleic acids encoding polypeptides. The vector is used to introduce a nucleic acid encoding a polypeptide into the host cell, so as to amplify the nucleic acid or express/display the polypeptide encoded by the nucleic acid. The vector is usually free but can be designed to allow the integration of a gene or a portion thereof into a chromosome of a genome. Vectors for artificial chromosomes are also contemplated, such as a yeast artificial vector and a mammal artificial chromosome. The selection and use of such intermedia are well-known to those skilled in the art.

As used herein, the vector also includes a "virus vector" or "viral vector". The viral vector is an engineered virus that is operably linked to an exogenous gene to transfer (as an intermedium or shuttle) the exogenous gene into cells.

As used herein, an "expression vector" includes vectors that can express DNA, and the DNA is operably linked to a regulatory sequence that can affect the expression of such DNA fragments, such as a promoter. Such additional fragments may include promoter and terminator sequences and optionally may include one or more origins of replication, one or more selectable markers, enhancers, polyadenylation signals, etc. The expression vector is usually derived from a plasmid or virus DNA or may contain elements of both. Therefore, the expression vector refers to a recombinant DNA or RNA construct, such as a plasmid, a phage, a recombinant virus, and other vectors, which express cloned DNA when introduced into an appropriate host cell. Appropriate expression vectors are well-known to those skilled in the art and include expression vectors that can be replicated in eukaryotic cells and/or prokaryotic cells, free expression vectors, and expression vectors for integration into the genome of a host cell.

As used herein, "treatment" of a disease or disease condition in an individual means that the individual's symptoms are partially or fully alleviated, or remain unchanged after treatment. Therefore, treatment includes prophylaxis, treatment, and/or cure. Prophylaxis refers to the prevention of an underlying disease and/or prevention of symptomatic deterioration or disease development. Treatment also includes any pharmaceutical use of any provided antibody or antigen-binding fragment thereof and composition provided herein.

As used herein, a "therapeutic effect" means an effect resulting from the treatment of an individual that alters, usually improves, or ameliorates symptoms of a disease or disease condition, or cures a disease or disease condition.

As used herein, the "therapeutically effective amount" or "therapeutically effective dose" refers to the amount of a substance, compound, material, or composition containing a compound that is at least sufficient to produce a therapeutic effect when administered to a subject. Therefore, it is the amount necessary to prevent, cure, ameliorate, arrest, or partially arrest symptoms of a disease or disorder.

As used herein, the "prophylactically effective amount" or "prophylactically effective dose" refers to the amount of a substance, compound, material, or composition containing a compound that, when administered to a subject, will have the desired prophylactic effect, such as preventing or delaying the onset or recurrence of a disease or symptom, and reducing the likelihood of the onset or recurrence of a disease or symptom. A full prophylactically effective dose does not have to occur by administering one dose, and may only occur after administering a series of doses. Therefore, the prophylactically effective amount may be administered in one or more administrations.

As used herein, the term "patient" refers to a mammal, such as humans.

### Detailed description

In an aspect, the present disclosure provides a bispecific antibody or an antigen-binding portion thereof, comprising
(a) a first antigen-binding portion of a GPC3 antigen or an antigen-binding fragment thereof that binds to the surface of a target cell, the first antigen-binding portion comprising a first heavy chain and a first light chain, the first antigen-binding portion comprising a first binding domain that binds to a first antigen, wherein the first binding domain comprises a heavy chain CDR selected from amino acid sequences of SEQ ID NO. 11, 12, 13, 21, 32, 33, 64, 65, 66, 72, 73, 79, 80, 81, 101, 106 or any variant thereof, and/or a light chain CDR selected from amino acid sequences of SEQ ID NO. 16, 17, 18, 26, 29, 38, 39, 46, 47, 48, 51, 54, 57, 60, 61, 69, 92, 95, 98 or any variant; and
(b) a second antigen binding portion or an antigen binding fragment thereof that binds to a CD3 antigen on the surface of a T cell, the second antigen binding portion comprising a second heavy chain and a second light chain, the second antigen binding portion comprising a second binding domain that binds to a second antigen.

The antibody or antigen binding portion thereof according to the previous aspect, the first binding domain comprises a heavy chain CDR1 selected from amino acid sequences of SEQ ID NO. 11, 32, 64, 79 or any variant thereof, a heavy chain CDR2 selected from amino acid sequences of SEQ ID NO. 12, 21, 33, 65, 72, 80, 101, 106 or any variant thereof, a heavy chain CDR3 selected from amino acid sequences of SEQ ID NO. 13, 66, 73, 81 or any variant thereof; and/or the first binding domain comprises a light chain CDR1 selected from the amino acid sequences of SEQ ID NO. 16, 38, 46, 60 or any variant thereof, a light chain CDR2 selected from the amino acid sequences of SEQ ID NO. 17, 47 or any variant thereof, a light chain CDR3 selected from the amino acid sequences of SEQ ID NO. 18, 26, 29, 39, 48, 51, 54, 57, 61, 69, 76 or any variant thereof. The antibody or antigen-binding portion thereof according to any one of the previous aspects, the heavy chain CDR1, CDR2, CDR3 sequences of the first binding domain being selected from a first heavy chain CDR1, CDR2, CDR3 sequences of the amino acid sequences of SEQ ID NO. 11, 12, 13, a first heavy chain CDR1, CDR2, CDR3 sequences of amino acid sequences of SEQ ID NO. 11, 21, 13, a first heavy chain CDR1, CDR2, CDR3 sequences of amino acid sequences of SEQ ID NO. 32, 33, 13, a first heavy chain CDR1, CDR2, CDR3 sequences of amino acid sequences of SEQ ID NO. 64, 65, 66, a first heavy chain CDR1, CDR2, CDR3 sequences of amino acid sequences of SEQ ID NO. 11, 72, 73, a first heavy chain CDR1, CDR2, CDR3 sequences of amino acid sequences of SEQ ID NO. 79, 80, 81, a first heavy chain CDR1, CDR2, CDR3 sequences of amino acid sequences of SEQ ID NO. 11, 101, 13, a first heavy chain CDR1, CDR2, CDR3 sequences of amino acid sequences of SEQ ID NO. 11, 106, 13; and the light chain CDR1, CDR2, CDR3 sequences of the first binding domain are selected from a first light chain CDR1, CDR2, CDR3 sequences of the amino acid sequences SEQ ID NO. 16, 17, 18, a first light chain CDR1, CDR2, CDR3 sequences of amino acid sequences of SEQ ID NO. 16, 17, 26, a first light chain CDR1, CDR2, CDR3 sequences of amino acid sequences of SEQ ID NO. 16, 17, 29, a first light chain CDR1, CDR2, CDR3 sequences of amino acid sequences of SEQ ID NO. 38, 17, 39, a first light chain CDR1, CDR2, CDR3 sequences of amino acid sequences SEQ ID NO. 46, 47, 48, a first light chain CDR1, CDR2, CDR3 sequences of amino acid sequences of SEQ ID NO. 16, 17, 51, a first light chain CDR1, CDR2, CDR3 sequences of amino acid sequences of SEQ ID NO. 16, 17, 54, a first light chain CDR1, CDR2, CDR3 sequences of amino acid sequences of SEQ ID NO. 16, 17, 57, a first light chain CDR1, CDR2, CDR3 sequences of amino acid sequences of SEQ ID NO. 60, 17, 61, a first light chain CDR1, CDR2, CDR3 sequences of amino acid sequences of SEQ ID NO. 16, 17, 69, a first light chain CDR1, CDR2, CDR3 sequences of amino acid sequences of SEQ ID NO. 16, 17, 76, a first light chain CDR1, CDR2, CDR3 sequences of amino acid sequences of SEQ ID NO. 92, 17, 61, a first light chain CDR1, CDR2, CDR3 sequences of amino acid sequences of SEQ ID NO. 95, 17, 61, a first light chain CDR1, CDR2, CDR3 sequences of amino acid sequences of SEQ ID NO. 98, 17, 61.

In some embodiments, the first binding domain comprises the first heavy chain CDR1, CDR2, CDR3 sequences of amino acid sequences of SEQ ID NO. 11, 12, 13 and the first light chain CDR1, CDR2, CDR3 sequences of amino acid sequences of SEQ ID NO. 16, 17, 18.

In some embodiments, the first binding domain comprises the first heavy chain CDR1, CDR2, CDR3 sequences of amino acid sequences of SEQ ID NO. 11, 21, 13 and the first light chain CDR1, CDR2, CDR3 sequences of amino acid sequences of SEQ ID NO. 16, 17, 18.

In some embodiments, the first binding domain comprises the first heavy chain CDR1, CDR2, CDR3 sequences of amino acid sequences of SEQ ID NO. 11, 12, 13 and the first light chain CDR1, CDR2, CDR3 sequences of amino acid sequences of SEQ ID NO. 16, 17, 26.

In some embodiments, the first binding domain comprises the first heavy chain CDR1, CDR2, CDR3 sequences of amino acid sequences of SEQ ID NO. 11, 12, 13 and the first light chain CDR1, CDR2, CDR3 sequences of amino acid sequences of SEQ ID NO. 16, 17, 29.

In some embodiments, the first binding domain comprises the first heavy chain CDR1, CDR2, CDR3 sequences of amino acid sequences of SEQ ID NO. 32, 33, 13 and the first light chain CDR1, CDR2, CDR3 sequences of amino acid sequences of SEQ ID NO. 16, 17, 18.

In some embodiments, the first binding domain comprises the first heavy chain CDR1, CDR2, CDR3 sequences of amino acid sequences of SEQ ID NO. 11, 21, 13 and the first light chain CDR1, CDR2, CDR3 sequences of amino acid sequences of SEQ ID NO. 38, 17, 39.

In some embodiments, the first binding domain comprises the first heavy chain CDR1, CDR2, CDR3 sequences of amino acid sequences of SEQ ID NO. 11, 21, 13 and the first light chain CDR1, CDR2, CDR3 sequences of amino acid sequences of SEQ ID NO. 16, 17, 18.

In some embodiments, the first binding domain comprises the first heavy chain CDR1, CDR2, CDR3 sequences of amino acid sequences of SEQ ID NO. 11, 12, 13 and the first light chain CDR1, CDR2, CDR3 sequences of amino acid sequences of SEQ ID NO. 46, 47, 48.

In some embodiments, the first binding domain comprises the first heavy chain CDR1, CDR2, CDR3 sequences of amino acid sequences of SEQ ID NO. 11, 12, 13 and the first light chain CDR1, CDR2, CDR3 sequences of amino acid sequences of SEQ ID NO. 16, 17,51.

In some embodiments, the first binding domain comprises the first heavy chain CDR1, CDR2, CDR3 sequences of amino acid sequences of SEQ ID NO. 11, 12, 13 and the first light chain CDR1, CDR2, CDR3 sequences of amino acid sequences of SEQ ID NO. 16, 17, 54.

In some embodiments, the first binding domain comprises the first heavy chain CDR1, CDR2, CDR3 sequences of amino acid sequences of SEQ ID NO. 11, 12, 13 and the first light chain CDR1, CDR2, CDR3 sequences of amino acid sequences of SEQ ID NO. 16, 17, 57.

In some embodiments, the first binding domain comprises the first heavy chain CDR1, CDR2, CDR3 sequences of amino acid sequences of SEQ ID NO. 11, 12, 13 and the first light chain CDR1, CDR2, CDR3 sequences of amino acid sequences of SEQ ID NO. 60, 17,61.

In some embodiments, the first binding domain comprises the first heavy chain CDR1, CDR2, CDR3 sequences of amino acid sequences of SEQ ID NO. 64, 65, 66 and the first light chain CDR1, CDR2, CDR3 sequences of amino acid sequences of SEQ ID NO. 16, 17, 69.

In some embodiments, the first binding domain comprises the first heavy chain CDR1, CDR2, CDR3 sequences of amino acid sequences of SEQ ID NO. 11, 72, 73 and the first light chain CDR1, CDR2, CDR3 sequences of amino acid sequences of SEQ ID NO. 16, 17,76.

In some embodiments, the first binding domain comprises the first heavy chain CDR1, CDR2, CDR3 sequences of amino acid sequences of SEQ ID NO. 79, 80, 81 and the first light chain CDR1, CDR2, CDR3 sequences of amino acid sequences of SEQ ID NO. 16, 17, 69.

In some embodiments, the first binding domain comprises the first heavy chain CDR1, CDR2, CDR3 sequences of amino acid sequences of SEQ ID NO. 11, 106, 13 and the first light chain CDR1, CDR2, CDR3 sequences of amino acid sequences of SEQ ID NO. 60, 17,61.

In some embodiments, the first binding domain comprises the first heavy chain CDR1, CDR2, CDR3 sequences of amino acid sequences of SEQ ID NO. 11, 106, 13 and the first light chain CDR1, CDR2, CDR3 sequences of amino acid sequences of SEQ ID NO. 92, 17,61.

In some embodiments, the first binding domain comprises the first heavy chain CDR1, CDR2, CDR3 sequences of amino acid sequences of SEQ ID NO. 11, 106, 13 and the first light chain CDR1, CDR2, CDR3 sequences of amino acid sequences of SEQ ID NO. 95, 17,61.

In some embodiments, the first binding domain comprises the first heavy chain CDR1, CDR2, CDR3 sequences of amino acid sequences of SEQ ID NO. 11, 106, 13 and the first light chain CDR1, CDR2, CDR3 sequences of amino acid sequences of SEQ ID NO. 98, 17,61.

The antibody or antigen binding portion thereof according to any of the previous aspects, the first binding domain comprises a first heavy chain variable region selected from amino acid sequences of SEQ ID NO. 9, 19, 30, 40, 62, 70, 77, 99, 102, 104, 107, 109, 111 or any variant thereof and/or comprises a first light chain variable region selected from amino acid sequences of SEQ ID NO. 14, 22, 24, 27, 34, 36, 42, 44, 49, 52, 55, 58, 67, 74, 82, 84, 86, 88, 90, 93, 96 or any variant thereof.

In some embodiments, the first binding domain comprises the first heavy chain variable region of the amino acid sequence of SEQ ID NO. 9 or any variant thereof, and the first light chain variable region of the amino acid sequence of SEQ ID NO. 14 or any variant thereof.

In some embodiments, the first binding domain comprises the first heavy chain variable region of the amino acid sequence of SEQ ID NO. 19 or any variant thereof, and the first light chain variable region of the amino acid sequence of SEQ ID NO. 22 or any variant thereof.

In some embodiments, the first binding domain comprises the first heavy chain variable region of the amino acid sequence of SEQ ID NO. 9 or any variant thereof, and the first light chain variable region of the amino acid sequence of SEQ ID NO. 24 or any variant thereof.

In some embodiments, the first binding domain comprises the first heavy chain variable region of the amino acid sequence of SEQ ID NO. 9 or any variant thereof, and the first light chain variable region of the amino acid sequence of SEQ ID NO. 27 or any variant thereof.

In some embodiments, the first binding domain comprises the first heavy chain variable region of the amino acid sequence of SEQ ID NO. 30 or any variant thereof, and the first light chain variable region of the amino acid sequence of SEQ ID NO. 34 or any variant thereof.

In some embodiments, the first binding domain comprises the first heavy chain variable region of the amino acid sequence of SEQ ID NO. 19 or any variant thereof, and the first light chain variable region of the amino acid sequence of SEQ ID NO. 36 or any variant thereof.

In some embodiments, the first binding domain comprises the first heavy chain variable region of the amino acid sequence of SEQ ID NO. 40 or any variant thereof, and the first light chain variable region of the amino acid sequence of SEQ ID NO. 42 or any variant thereof.

In some embodiments, the first binding domain comprises the first heavy chain variable region of the amino acid sequence of SEQ ID NO. 9 or any variant thereof, and the first light chain variable region of the amino acid sequence of SEQ ID NO. 44 or any variant thereof.

In some embodiments, the first binding domain comprises the first heavy chain variable region of the amino acid sequence of SEQ ID NO. 9 or any variant thereof, and the first light chain variable region of the amino acid sequence of SEQ ID NO. 49 or any variant thereof.

In some embodiments, the first binding domain comprises the first heavy chain variable region of the amino acid sequence of SEQ ID NO. 9 or any variant thereof, and the first light chain variable region of the amino acid sequence of SEQ ID NO. 52 or any variant thereof.

In some embodiments, the first binding domain comprises the first heavy chain variable region of the amino acid sequence of SEQ ID NO. 9 or any variant thereof, and the first light chain variable region of the amino acid sequence of SEQ ID NO. 55 or any variant thereof.

In some embodiments, the first binding domain comprises the first heavy chain variable region of the amino acid sequence of SEQ ID NO. 9 or any variant thereof, and the first light chain variable region of the amino acid sequence of SEQ ID NO. 58 or any variant thereof.

In some embodiments, the first binding domain comprises the first heavy chain variable region of the amino acid sequence of SEQ ID NO. 62 or any variant thereof, and the first light chain variable region of the amino acid sequence of SEQ ID NO. 67 or any variant thereof.

In some embodiments, the first binding domain comprises the first heavy chain variable region of the amino acid sequence of SEQ ID NO. 70 or any variant thereof, and the first light chain variable region of the amino acid sequence of SEQ ID NO. 74 or any variant thereof.

In some embodiments, the first binding domain comprises the first heavy chain variable region of the amino acid sequence of SEQ ID NO. 77 or any variant thereof, and the first light chain variable region of the amino acid sequence of SEQ ID NO. 82 or any variant thereof.

In some embodiments, the first binding domain comprises the first heavy chain variable region of the amino acid sequence of SEQ ID NO. 111 or any variant thereof, and the first light chain variable region of the amino acid sequence of SEQ ID NO. 86 or any variant thereof.

In some embodiments, the first binding domain comprises the first heavy chain variable region of the amino acid sequence of SEQ ID NO. 111 or any variant thereof, and the first light chain variable region of the amino acid sequence of SEQ ID NO. 88 or any variant thereof.

In some embodiments, the first binding domain comprises the first heavy chain variable region of the amino acid sequence of SEQ ID NO. 111 or any variant thereof, and the first light chain variable region of the amino acid sequence of SEQ ID NO. 90 or any variant thereof.

In some embodiments, the first binding domain comprises the first heavy chain variable region of the amino acid sequence of SEQ ID NO. 111 or any variant thereof, and the first light chain variable region of the amino acid sequence of SEQ ID NO. 93 or any variant thereof.

In some embodiments, the first binding domain comprises the first heavy chain variable region of the amino acid sequence of SEQ ID NO. 111 or any variant thereof, and the first light chain variable region of the amino acid sequence of SEQ ID NO. 96 or any variant thereof.

An antibody or antigen binding portion thereof according to any of the previous aspects, the second binding domain is selected from a heavy chain CDR selected from amino acid sequences of SEQ ID NO. 134, 135, 136, 139, 142, 145, 148, 151, 154, 155 or any variant thereof; and/or comprising a light chain CDR selected from amino acid sequences of SEQ ID NO. 115, 116, 117, 122, 123, 128, 131 or any variant thereof.

In some embodiments, the second binding domain comprises a second heavy chain CDR1 selected from the amino acid sequences of SEQ ID NO. 134, 139, 154 or any variant thereof, a second heavy chain CDR2 selected from the amino acid sequences of SEQ ID NO. 135, 155 or any variant thereof, a second heavy chain CDR3 selected from the amino acid sequences of SEQ ID NO. 136, 142, 145, 148, 151 or any variant thereof; and/or the second light chain CDR1 selected from the amino acid sequences of SEQ ID NO. 115, 122 or any variant thereof, the second light chain CDR2 selected from the amino acid sequences of SEQ ID NO. 116, 123, 131 or any variant thereof, the second light chain CDR3 selected from the amino acid sequences of SEQ ID NO. 117, 128 or any variant thereof.

In some embodiments, the heavy chain CDR1, CDR2, CDR3 sequences of the second binding domain are selected from: the second heavy chain CDR1, CDR2, CDR3 sequences of the amino acid sequences of SEQ ID NO. 134, 135, 136, the second heavy chain CDR1, CDR2, CDR3 sequences of the amino acid sequences of SEQ ID NO. 139, 135, 136, the second heavy chain CDR1, CDR2, CDR3 sequences of the amino acid sequences of SEQ ID NO. 139, 135, 142, the second heavy chain CDR1, CDR2, CDR3 sequences of the amino acid sequences SEQ ID NO. 139, 135, 145, the second heavy chain CDR1, CDR2, CDR3 sequences of the amino acid sequences of SEQ ID NO. 139, 135, 148, the second heavy chain CDR1, CDR2, CDR3 sequences of the amino acid sequences SEQ ID NO. 139, 135, 151, the second heavy chain CDR1, CDR2, CDR3 sequences of the amino acid sequences SEQ ID NO. 154, 155, 136; and the light chain CDR1, CDR2, CDR3 sequences of the second binding domain are selected from: the second light chain CDR1, CDR2, CDR3 sequences of the amino acid sequences of SEQ ID NO. 115, 116, 117, the second light chain CDR1, CDR2, CDR3 sequences of the amino acid sequences of SEQ ID NO. 122, 123, 117, the second light chain CDR1, CDR2, CDR3 sequences of the amino acid sequences SEQ ID NO. 115, 116, 128, the second light chain CDR1, CDR2, CDR3 sequences of the amino acid sequences of SEQ ID NO. 115, 131, 128.

In some embodiments, the second binding domain comprises the second heavy chain CDR1, CDR2, CDR3 sequences of the amino acid sequences of SEQ ID NO. 134, 135, 136, and second light chain CDR1, CDR2, CDR3 sequences of the amino acid sequences of SEQ ID NO. 115, 116, 128.

In some embodiments, the second binding domain comprises the second heavy chain CDR1, CDR2, CDR3 sequences of the amino acid sequences of SEQ ID NO. 134, 135, 136, and second light chain CDR1, CDR2, CDR3 sequences of the amino acid sequences of SEQ ID NO. 115, 116, 117.

In some embodiments, the second binding domain comprises the second heavy chain CDR1, CDR2, CDR3 sequences of the amino acid sequences of SEQ ID NO. 134, 135, 136, and second light chain CDR1, CDR2, CDR3 sequences of the amino acid sequences of SEQ ID NO. 122, 123, 117.

In some embodiments, the second binding domain comprises the second heavy chain variable region selected from the amino acid sequences of SEQ ID NO. 132, 137, 140, 143, 146, 149, 152, 156, 158, 160 or any variant thereof, and/or comprises the second light chain variable region selected from the amino acid sequences of SEQ ID NO. 113, 118, 120, 124, 126, 129 or any variant thereof.

In some embodiments, the second binding domain comprises the second heavy chain variable region of the amino acid sequence of SEQ ID NO. 132 or any variant thereof, and the second light chain variable region of the amino acid sequence of SEQ ID NO. 126 or any variant thereof.

In some embodiments, the second binding domain comprises the second heavy chain variable region of the amino acid sequence of SEQ ID NO. 156 or any variant thereof, and the second light chain variable region of the amino acid sequence of SEQ ID NO. 113 or any variant thereof.

In some embodiments, the second binding domain comprises the second heavy chain variable region of the amino acid sequence of SEQ ID NO. 156 or any variant thereof, and the second light chain variable region of the amino acid sequence of SEQ ID NO. 126 or any variant thereof.

In some embodiments, the second binding domain comprises the second heavy chain variable region of the amino acid sequence of SEQ ID NO. 158 or any variant thereof, and the second light chain variable region of the amino acid sequence of SEQ ID NO. 113 or any variant thereof.

In some embodiments, the second binding domain comprises the second heavy chain variable region of the amino acid sequence of SEQ ID NO. 158 or any variant thereof, and the second light chain variable region of the amino acid sequence of SEQ ID NO. 118 or any variant thereof.

In some embodiments, the second binding domain comprises the second heavy chain variable region of the amino acid sequence of SEQ ID NO. 158 or any variant thereof, and the second light chain variable region of the amino acid sequence of SEQ ID NO. 120 or any variant thereof.

In some embodiments, the second binding domain comprises the second heavy chain variable region of the amino acid sequence of SEQ ID NO. 158 or any variant thereof, and the second light chain variable region of the amino acid sequence of SEQ ID NO. 126 or any variant thereof.

The antibody or antigen-binding portion thereof according to any one of the previous aspects, the first light chain of the first antigen binding portion is a kappa-type light chain and the second light chain of the second antigen binding portion is a lambda-type light chain.

In some embodiments, the first light chain variable region of the first antigen binding portion has a Gln₄₃Lys mutation (Vκ_{GPC3}: Gln₄₃Lys). In some embodiments, the first heavy chain variable region of the first antigen-binding portion has a Gln₃₉Glu (VH_{GPC3}: Gln₃₉Glu) mutation.

In some embodiments, the second light chain variable region of the second antigen-binding portion has a Gln₄₀Glu mutation (Vλ_{CD3}: Gln₄₀Glu). In some embodiments, the second heavy chain variable region of the second antigen-binding portion has a Gln₃₉Lys mutation (VH_{CD3}: Gln₃₉Lys).

In some embodiments, the Fc portion of the first antigen-binding portion and the second antigen-binding portion of the bispecific antibody adopts a knob-into-hole structure. In some preferred embodiments, a human IgG4 knob-into-hole structure is adopted.

In some embodiments, the first heavy chain comprises the heavy chain selected from SEQ ID NO. 164, 172 or any variant thereof and the first light chain comprises the light chain selected from SEQ ID NO. 162, 170 or any variant thereof.

In some preferred embodiments, the first heavy chain comprises the heavy chain selected from SEQ ID NO. 164 or any variant thereof and the first light chain comprises the light chain selected from SEQ ID NO. 162 or any variant thereof.

In some preferred embodiments, the first heavy chain comprises the heavy chain selected from SEQ ID NO. 172 or any variant thereof and the first light chain comprises the light chain selected from SEQ ID NO. 170 or any variant thereof.

In some preferred embodiments, the second heavy chain comprises the heavy chain of the amino acid sequence of SEQ ID NO. 168 or any variant thereof and the second light chain comprises the light chain of the amino acid sequence of SEQ ID NO. 166 or any variant thereof.

In a particular embodiment, the first heavy chain of the bispecific antibody comprises the heavy chain of the amino acid sequence of SEQ ID NO. 164 or any variant thereof and the first light chain of the bispecific antibody comprises the light chain of the amino acid sequence of SEQ ID NO. 162 or any variant thereof; the second heavy chain comprises the heavy chain of the amino acid sequence of SEQ ID NO. 168 or any variant thereof and the second light chain comprises the light chain of the amino acid sequence SEQ ID NO. 166 or any variant thereof.

In a particular embodiment, the first heavy chain comprises the heavy chain of the amino acid sequence of SEQ ID NO. 172 or any variant thereof and the first light chain of the bispecific antibody comprises the light chain of the amino acid sequence SEQ ID NO. 170 or any variant thereof; the second heavy chain comprises the heavy chain of the amino acid sequence of SEQ ID NO. 168 or any variant thereof and the second light chain comprises the light chain of the amino acid sequence SEQ ID NO. 166 or any variant thereof.

In one aspect, the present disclosure provides an antibody or antigen-binding portion thereof that binds to GPC3, the antibody comprising the aforementioned first antigen-binding portion.

In some embodiments, the antibody or antigen-binding portion thereof that binds to GPC3 has at least greater than 60%, 65%, 70%, 75%, 80%, 85%, 90%, 95%, 96%, 97%, 98%, 99% or more sequence identity to the antibody or antigen-binding portion thereof of any of the previous aspects.

In one aspect, the present disclosure provides a nucleic acid encoding an antibody or antigen-binding portion thereof according to any one of the previous aspects, or a nucleic acid molecule having at least greater than 60%, 65%, 70%, 75%, 80%, 85%, 90%, 95%, 96%, 97%, 98%, 99% or more sequence identity thereto.

In some embodiments, the nucleic acid encoding the first heavy chain variable region of the first antigen binding portion is selected from nucleotide sequences of SEQ ID NO. 10, 20, 31, 41, 63, 71, 78, 100, 103, 105, 108, 110, 112 or any variant thereof; and/or the nucleic acid encoding the first light chain variable region of the first antigen binding portion is selected from nucleotide sequences of SEQ ID NO. 15, 23, 25, 28, 35, 37, 43, 45, 50, 53, 56, 59, 68, 75, 83, 85, 87, 89, 91, 94, 97 or any variant thereof.

In some preferred embodiments, the nucleic acid encoding the first heavy chain variable region of the first antigen-binding portion is the nucleotide sequence of SEQ ID NO. 10; and the nucleic acid encoding the first light chain variable region is the nucleotide sequence of SEQ ID NO. 15.

In some preferred embodiments, the nucleic acid encoding the first heavy chain variable region of the first antigen-binding portion is the nucleotide sequence of SEQ ID NO. 20; and the nucleic acid encoding the first light chain variable region is the nucleotide sequence of SEQ ID NO. 23.

In some preferred embodiments, the nucleic acid encoding the first heavy chain variable region of the first antigen-binding portion is the nucleotide sequence of SEQ ID NO. 10; and the nucleic acid encoding the first light chain variable region is the nucleotide sequence of SEQ ID NO. 25.

In some preferred embodiments, the nucleic acid encoding the first heavy chain variable region of the first antigen-binding portion is the nucleotide sequence of SEQ ID NO. 10; and the nucleic acid encoding the first light chain variable region is the nucleotide sequence of SEQ ID NO. 28.

In some preferred embodiments, the nucleic acid encoding the first heavy chain variable region of the first antigen-binding portion is the nucleotide sequence of SEQ ID NO. 31; and the nucleic acid encoding the first light chain variable region is the nucleotide sequence of SEQ ID NO. 35.

In some preferred embodiments, the nucleic acid encoding the first heavy chain variable region of the first antigen-binding portion is the nucleotide sequence of SEQ ID NO. 20; and the nucleic acid encoding the first light chain variable region is the nucleotide sequence of SEQ ID NO. 37.

In some preferred embodiments, the nucleic acid encoding the first heavy chain variable region of the first antigen-binding portion is the nucleotide sequence of SEQ ID NO. 41; and the nucleic acid encoding the first light chain variable region is the nucleotide sequence of SEQ ID NO. 43.

In some preferred embodiments, the nucleic acid encoding the first heavy chain variable region of the first antigen-binding portion is the nucleotide sequence of SEQ ID NO. 10; and the nucleic acid encoding the first light chain variable region is the nucleotide sequence of SEQ ID NO. 45.

In some preferred embodiments, the nucleic acid encoding the first heavy chain variable region of the first antigen-binding portion is the nucleotide sequence of SEQ ID NO. 10; and the nucleic acid encoding the first light chain variable region is the nucleotide sequence of SEQ ID NO. 50.

In some preferred embodiments, the nucleic acid encoding the first heavy chain variable region of the first antigen-binding portion is the nucleotide sequence of SEQ ID NO. 10; and the nucleic acid encoding the first light chain variable region is the nucleotide sequence of SEQ ID NO. 53.

In some preferred embodiments, the nucleic acid encoding the first heavy chain variable region of the first antigen-binding portion is the nucleotide sequence of SEQ ID NO. 10; and the nucleic acid encoding the first light chain variable region is the nucleotide sequence of SEQ ID NO. 56.

In some preferred embodiments, the nucleic acid encoding the first heavy chain variable region of the first antigen-binding portion is the nucleotide sequence of SEQ ID NO. 10; and the nucleic acid encoding the first light chain variable region is the nucleotide sequence of SEQ ID NO. 59.

In some preferred embodiments, the nucleic acid encoding the first heavy chain variable region of the first antigen-binding portion is the nucleotide sequence of SEQ ID NO. 63; and the nucleic acid encoding the first light chain variable region is the nucleotide sequence of SEQ ID NO. 68.

In some preferred embodiments, the nucleic acid encoding the first heavy chain variable region of the first antigen-binding portion is the nucleotide sequence of SEQ ID NO. 71; and the nucleic acid encoding the first light chain variable region is the nucleotide sequence of SEQ ID NO. 75.

In some preferred embodiments, the nucleic acid encoding the first heavy chain variable region of the first antigen-binding portion is the nucleotide sequence of SEQ ID NO. 78; and the nucleic acid encoding the first light chain variable region is the nucleotide sequence of SEQ ID NO. 83.

In some preferred embodiments, the nucleic acid encoding the first heavy chain variable region of the first antigen-binding portion is the nucleotide sequence of SEQ ID NO. 112; and the nucleic acid encoding the first light chain variable region is the nucleotide sequence of SEQ ID NO. 87.

In some preferred embodiments, the nucleic acid encoding the first heavy chain variable region of the first antigen-binding portion is the nucleotide sequence of SEQ ID NO. 112; and the nucleic acid encoding the first light chain variable region is the nucleotide sequence of SEQ ID NO. 89.

In some preferred embodiments, the nucleic acid encoding the first heavy chain variable region of the first antigen-binding portion is the nucleotide sequence of SEQ ID NO. 112; and the nucleic acid encoding the first light chain variable region is the nucleotide sequence of SEQ ID NO. 91.

In some preferred embodiments, the nucleic acid encoding the first heavy chain variable region of the first antigen-binding portion is the nucleotide sequence of SEQ ID NO. 112; and the nucleic acid encoding the first light chain variable region is the nucleotide sequence of SEQ ID NO. 94.

In some preferred embodiments, the nucleic acid encoding the first heavy chain variable region of the first antigen-binding portion is the nucleotide sequence of SEQ ID NO. 112; and the nucleic acid encoding the first light chain variable region is the nucleotide sequence of SEQ ID NO. 97.

Preferably, the nucleic acid encoding the first heavy chain of the first antigen-binding portion is selected from the nucleotide sequences of SEQ ID NO. 165, 173 or any variant thereof; and/or the nucleic acid encoding the first light chain of the first antigen binding portion is selected from the nucleotide sequences of SEQ ID NO. 163, 171 or any variant thereof.

In some preferred embodiments, the nucleic acid encoding the first heavy chain of the first antigen-binding portion is the nucleotide sequence of SEQ ID NO. 165 and the nucleic acid encoding the first light chain of the first antigen-binding portion is the nucleotide sequence of SEQ ID NO. 163.

In some preferred embodiments, the nucleic acid encoding the first heavy chain of the first antigen-binding portion is the nucleotide sequence of SEQ ID NO. 173 and the nucleic acid encoding the first light chain of the first antigen-binding portion is the nucleotide sequence of SEQ ID NO. 171.

In some embodiments, the nucleic acid encoding the second heavy chain variable region of the second antigen-binding portion is selected from the nucleotide sequences of SEQ ID NO. 133, 138, 141, 144, 147, 150, 153, 157, 159, 161 or any variant thereof; and/or the nucleic acid encoding the second light chain variable region of the second antigen binding portion is selected from the nucleotide sequences of SEQ ID NO. 114, 119, 121, 125, 127, 130 or any variant thereof.

In some preferred embodiments, the nucleic acid encoding the second heavy chain variable region of the second antigen-binding portion is the nucleotide sequence of SEQ ID NO. 133; and the nucleic acid encoding the second light chain variable region is the nucleotide sequence of SEQ ID NO. 127.

In some preferred embodiments, the nucleic acid encoding the second heavy chain variable region of the second antigen-binding portion is the nucleotide sequence of SEQ ID NO. 157; and the nucleic acid encoding the second light chain variable region is the nucleotide sequence of SEQ ID NO. 114.

In some preferred embodiments, the nucleic acid encoding the second heavy chain variable region of the second antigen-binding portion is the nucleotide sequence of SEQ ID NO. 157; and the nucleic acid encoding the second light chain variable region is the nucleotide sequence of SEQ ID NO. 127.

In some preferred embodiments, the nucleic acid encoding the second heavy chain variable region of the second antigen-binding portion is the nucleotide sequence of SEQ ID NO. 159; and the nucleic acid encoding the second light chain variable region is the nucleotide sequence of SEQ ID NO. 114.

In some preferred embodiments, the nucleic acid encoding the second heavy chain variable region of the second antigen-binding portion is the nucleotide sequence of SEQ ID NO. 159; and the nucleic acid encoding the second light chain variable region is the nucleotide sequence of SEQ ID NO. 119.

In some preferred embodiments, the nucleic acid encoding the second heavy chain variable region of the second antigen-binding portion is the nucleotide sequence of SEQ ID NO. 159; and the nucleic acid encoding the second light chain variable region is the nucleotide sequence of SEQ ID NO. 121.

In some preferred embodiments, the nucleic acid encoding the second heavy chain variable region of the second antigen-binding portion is the nucleotide sequence of SEQ ID NO. 159; and the nucleic acid encoding the second light chain variable region is the nucleotide sequence of SEQ ID NO. 127.

Preferably, the nucleic acid encoding the second heavy chain of the second antigen-binding portion is the nucleotide sequence of SEQ ID NO. 169 or any variant thereof; and/or the nucleic acid encoding the second light chain of the second antigen-binding portion is the nucleotide sequence SEQ ID NO. 167 or any variant thereof.

In some preferred embodiments, the nucleic acid encoding the second heavy chain of the second antigen-binding portion is the nucleotide sequence of SEQ ID NO. 169 and the nucleic acid encoding the second light chain of the second antigen-binding portion is the nucleotide sequence of SEQ ID NO. 167.

In a specific embodiment, the nucleic acid encoding the first heavy chain of the first antigen-binding portion of the bispecific antibody is the nucleotide sequence of SEQ ID NO. 165, and the nucleic acid encoding the first light chain of the first antigen-binding portion is the nucleotide sequence of SEQ ID NO. 163; the nucleic acid encoding the second heavy chain of the second antigen-binding portion is the nucleotide sequence of SEQ ID NO. 169, and the nucleic acid encoding the second light chain of the second antigen-binding portion is the nucleotide sequence of SEQ ID NO. 167.

In a specific embodiment, the nucleic acid encoding the first heavy chain of the first antigen-binding portion of the bispecific antibody is the nucleotide sequence of SEQ ID NO. 173, and the nucleic acid encoding the first light chain of the first antigen-binding portion is the nucleotide sequence of SEQ ID NO. 171; the nucleic acid encoding the second heavy chain of the second antigen-binding portion is the nucleotide sequence of SEQ ID NO. 169, and the nucleic acid encoding the second light chain of the second antigen-binding portion is the nucleotide sequence of SEQ ID NO. 167.

In one aspect, the present disclosure provides a vector comprising the aforementioned nucleic acid.

In one aspect, the present disclosure provides a cell comprising the aforementioned nucleic acid or vector.

In one aspect, the present disclosure provides a composition comprising the aforementioned bispecific antibodies or antigen binding portions thereof, nucleic acid, vector, and/or cells.

In one aspect, the present disclosure provides an antibody-drug conjugate comprising the aforementioned bispecific antibody or antigen-binding portion thereof covalently attached to a therapeutic moiety.

Preferably, the therapeutic moiety is selected from a cytotoxic moiety, a chemotherapeutic agent, a cytokine, an immunosuppressant, an immunostimulant, a lytic peptide, or a radioisotope.

The antibody of the present disclosure is used as a therapeutic or diagnostic tool for various diseases in which GPC3 is unfavorably expressed or found.

In an embodiment of a GPC3-associated disease, the expression of GPC3 is increased in cells of a diseased tissue or organ compared to the state in a healthy tissue or organ. Increase refers to an increase of at least 10%, especially at least 20%, at least 50%, at least 100%, at least 200%, at least 500%, at least 1000%, at least 10000%, or even more. In an embodiment, expression is found in a diseased tissue only, and the expression in a corresponding health tissue is depressed. According to the present disclosure, the GPC3-associated disease includes tumors.

In some embodiments, the tumor is a cancer. In some embodiments, the cancer is a GPC3 positive cancer, e.g., a GPC3 positive liver cancer, a GPC3 positive hepatocellular carcinoma, a GPC positive pancreatic cancer, a GPC positive lung cancer, a GPC positive colon cancer, a GPC positive breast cancer, a GPC positive prostate cancer, a GPC positive leukemia, or a GPC positive lymphoma.

In some embodiments, the therapeutic agent contains an antibody specifically binding to an activated T-cell antigen.

In an embodiment, the therapeutic agent contains an antibody specifically binding to CD3, particularly CD3ε.

A method for treating a disease or disorder with the bispecific antibody of the present disclosure includes the following steps: a therapeutically effective amount of antibody or antigen-binding fragment thereof or nucleic acid molecule or vector or cell or pharmaceutical composition of any one of the aforementioned aspects is administered to a mammal.

In some embodiments, the present disclosure provides a method for treating or preventing cancer, which includes the following steps: an antibody that can bind to GPC3 is administered to a patient, so as to provide a serum level of at least 40 µg/mL. In different embodiments, the antibody is administered to provide at a serum level of at least 50 µg/mL, at least 150 µg/mL, at least 300 µg/mL, at least 400 µg/mL, or at least 500 µg/mL. In different embodiments, the antibody is administered to provide a serum level of no more than 800 µg/mL, 700 µg/mL, 600 µg/mL, 550 µg/mL or 500 µg/mL. In an embodiment, the provided serum level is 40 µg/mL to 700 µg/mL, preferably 40 µg/mL to 600 µg/mL, preferably 50 µg/mL to 500 µg/mL, such as 150 µg/mL to 500 µg/mL and 300 µg/mL to 500 µg/mL. The term "serum level" as used in this description refers to the concentration of the substance in question in the serum. In an embodiment, the serum level is provided for at least 7 days or at least 14 days. In an embodiment, the method includes administering the antibody in a dose of at least 300 mg/m², such as at least 600 mg/m², preferably up to 1500 mg/m², up to 1200 mg/m², or up to 1000 mg/m².

In some embodiments, the present disclosure provides a method for treating or preventing cancer, which includes administering to a patient an antibody that can bind to GPC3 in a dose of at least 300 mg/m², such as at least 600 mg/m², and preferably up to 1500 mg/m², up to 1200 mg/m² or up to 1000 mg/m².

In some embodiments, the disclosure provides a method of treating or preventing a cancer disease comprising administering an antibody that can bind to GPC3 to a patient, wherein at least 50%, preferably 60%, 70%, 80%, or 90% of the cancer cells of the patient are positive for GPC3 and/or at least 40%, preferably 50% or 60% of the cancer cells of the patient are positive for surface expression of GPC3. In this aspect, the present disclosure also provides a method for treating or preventing cancer, which includes the following steps: a. identifying a patient showing at least 50%, preferably 60%, 70%, 80%, or 90%, of GPC3-positive cancer cells and/or at least 40%, preferably 50% or 60%, of cancer cells, the cancer cells are positive for the surface expression of GPC3; and b. administering an antibody that can bind to GPC3 to the patient. In an embodiment, at least 95% or at least 98% of cancer cells in the patient are positive for GPC3. In an embodiment, at least 70%, at least 80%, or at least 90% of cancer cells in the patient are positive for the surface expression of GPC3.

In an embodiment of the method of any aspect described herein, the outcome of cancer treatment is to achieve disease stabilization. In an embodiment, the disease stabilization is achieved for at least 2 months, at least 3 months, or at least 6 months.

In some embodiments, the present disclosure provides a method for achieving the disease stabilization of a cancer patient, which includes administering an antibody that can bind to GPC3 to a patient. In an embodiment, the disease stabilization is achieved for at least 2 months, at least 3 months, or at least 6 months.

In an embodiment of the method of any aspect described herein, the antibody is administered in a single dose or multiple doses.

In some embodiments, the present disclosure provides a method for treating or preventing cancer, which includes administering an antibody that can bind to GPC3 to a patient in multiple doses.

In a case that the antibody is administered in multiple doses according to the present disclosure, the antibody is administered in preferably at least 3 doses, at least 4 doses, at least 5 doses, at least 6 doses, at least 7 doses, at least 8 doses, at least 9 doses or at least 10 doses, and preferably up to 30 doses, 25 doses, 20 doses, 15 doses or 10 doses. Doses of the antibody are preferably administered at intervals of at least 7 days, at least 10 days, at least 14 days, or at least 20 days. Doses of the antibody are preferably administered at intervals of 7 to 30 days or 10 to 20 days, preferably about 14 days.

In an embodiment, the antibody is administered to provide a serum level of at least 40 µg/mL. In different embodiments, the antibody is administered to provide at a serum level of at least 50 µg/mL, at least 150 µg/mL, at least 300 µg/mL, at least 400 µg/mL, or at least 500 µg/mL. In different embodiments, the antibody is administered to provide a serum level of no more than 800 µg/mL, 700 µg/mL, 600 µg/mL, 550 µg/mL, or 500 µg/mL. In one embodiment, the serum level provided is from 40 µg/ml to 700 µg/ml, preferably from 40 µg/ml to 600 µg/ml, preferably from 50 µg/ml to 500 µg/ml, such as from 150 µg/ml to 500 µg/ml or from 300 µg/ml to 500 µg/ml. In an embodiment, the serum level is provided for at least 7 days or at least 14 days. In one embodiment, the method comprises administering the antibody in a dose of at least 300 mg/m², such as at least 600 mg/m² and preferably at most 1500 mg/m², at most 1200 mg/m² or at most 1000 mg/m².

Use of the antibody or the antigen-binding fragment thereof or the nucleic acid or the vector or the cell or the pharmaceutical composition of any one of the aforementioned aspects in the preparation of a drug for treating a GPC3-associated disorder in a mammal is provided.

According to any one of the aforementioned aspects, optionally, the antibody is conjugated to other drugs such as a labeled or cytotoxic conjugate.

In an aspect, the present disclosure also includes a kit. For example, the kit includes the antibody or a fragment or homolog or derivative thereof of the present disclosure, such as a labeled or cytotoxic conjugate, antibody instructions, a conjugate that kills specific types of cells, etc. The instructions may include directions for using the antibody, conjugate, etc. in vitro, in vivo, or ex vivo. The antibody may be in the form of a liquid or solid and is usually lyophilized. This kit may contain other appropriate reagents, such as a buffer, a reconstitution solution, and other components necessary for the intended use. A reagent combination packaged in predetermined doses and instructions for its use are contemplated, and the use is, for example, therapeutic use or diagnostic assay. In the case that the antibody is labeled with, for example, an enzyme, the kit may include a substrate and a cofactor required for the enzyme (e.g., a substrate precursor that provides a detectable chromophore or fluorophore). In addition, other additives, such as a stabilizer and a buffer (e.g., a blocking buffer and a lysis buffer), may be included. The relative amounts of various reagents can be varied such that a concentrate of a reagent solution is provided, which achieves user flexibility, space savings, reagent savings, etc. These reagents may also be provided in the form of dry powder, usually in a lyophilized form, including an excipient, which can provide a reagent solution at an appropriate concentration when dissolved.

Use of the antibody or the functional fragment or the nucleic acid or the vector or the cell or the pharmaceutical composition or the kit of any one of the aforementioned aspects in the preparation of a reagent for inhibiting the binding to GPC3 is provided.

In addition, the antibody of the present disclosure can also be used for immunoassay, a purification method, and other methods using immunoglobulins or fragments thereof. Such use is well-known to those skilled in the art.

Correspondingly, the present disclosure also provides a composition containing the anti-GPC3 antibody or the fragment thereof of the present disclosure, and the antibody is conveniently combined with a pharmaceutically acceptable carrier, a diluent, or an excipient, as is common practice in the art.

The term "pharmaceutical composition" refers to the preparation of various preparations. A preparation containing a therapeutically effective amount of polyvalent antibody is a sterile liquid solution, liquid suspension, or lyophilized form, optionally containing a stabilizer or excipient.

The antibody of the present disclosure may be used as a composition that is administered alone, or used in combination with other active agents.

In some embodiments, the humanized antibody of the present disclosure is conjugated to a therapeutic moiety (i.e., a drug). The therapeutic moiety may be, for example, a cytotoxin, a chemotherapeutic agent, a cytokine, an immunosuppressive drug, an immunostimulant, a lytic peptide, or a radioisotope. Such a conjugate is referred to as an "antibody-drug conjugate" or "ADC" herein.

In some embodiments, the antibody is conjugated to a cytotoxic moiety. The cytotoxic moiety may for example be selected from: paclitaxel; cytochalasin B; gramicidin D; ethidium bromide; emetine; mitomycin; etoposide; teniposide; vincristine; vinblastine; colchicine; doxorubicin; daunorubicin; dihydroxy anthrenedione; a tubulin inhibitor such as maytansine and an analog or derivative thereof; an antimitotic agent such as monomethyl auristatins E and F and analogs or derivatives thereof; aplysiatoxins 10 and 15 and analogs thereof; irinotecan and an analog thereof; mitoxantrone; mithramycin; actinomycin D; 1-dehydrotestosterone; a glucocorticoid; procaine; tetracaine; lidocaine; propranolol; puromycin; calicheamicin and an analog or derivative thereof; an anti-metabolite such as methotrexate, 6-mercaptopurine, 6-thioguanine, cytarabine, fludarabine, 5-fluorouracil, decanedizine, hydroxycarbamide, asparaginase, gemcitabine, and cladribine; an alkylating agent such as dichloromethyldiethylamine, a thiopurine, chlorambucil, melphalan, carmustine (BSNU), lomustine (CCNU), cyclophosphamide, busulfan, mitobronitol, streptozotocin, dacarbazine (DTIC), procarbazine, and mitomycin C; a platinum derivative such as cisplatin or carboplatin; duocarmycin A, duocarmycin SA, rapamycin (CC-1065), and analogs or derivatives thereof; an antibiotic such as actinomycin, bleomycin, daunorubicin, doxorubicin, idarubicin, photomycin, mitomycin, mitoxantrone, perimycin, and diazepam (AMC); pyrrolo[2,1-c][1,4]-benzodiazepine (PDB); diphtheria toxin and related molecules such as diphtheria A chain and an active fragment and a hybrid molecule thereof, ricin such as ricin A and deglycosylated ricin A chain toxin, cholera toxin, a Shiga-like toxin such as SLT I, SLT II and SLT IIV, LT toxin, C3 toxin, a Shiga toxin, pertussis toxin, tetanus toxin, a soybean Bowman-Birk protease inhibitor, Pseudomonas exotoxin, aroline, saporin, Adenia heterophylla root toxin, gelsolin, abrin A chain, Adenia heterophylla root A chain, α-sarcin, Aleurites fordii protein, caryophyllin protein, a Phytolacca americana protein such as PAPI, PAPII and PAP-S, a Momordica charantia inhibitor, curcin, crotin, a Sapaonaria officinalis inhibitor, gelonin, mitomycin, restrictocin, phenomycin, and enomycin toxin; ribonuclease (RNase); DNase I and Staphylococcus aureus endotoxin A; Phytolacca acinosa antiviral protein; and diphtheria toxin and Pseudomonas endotoxin.

In some embodiments, the antibody is conjugated to an auristatin or a peptide analog, derivative or prodrug thereof. It has been shown that auristatins interfere with microtubule dynamics, GTP hydrolysis, and nuclear and cell division, and have anticancer and antifungal activity. For example, auristatin E can react with p-acetylbenzoic acid or benzoylvaleric acid to respectively produce AEB and AEVB. Other typical auristatin derivatives include AFP, monomethyl auristatin F (MMAF), and monomethyl auristatin E (MMAE). Appropriate auristatins and analogs, derivatives, and prodrugs thereof, as well as appropriate adaptor for conjugating auristatins to Ab are described in, for example, U.S. patent NO. 5,635,483, 5,780,588 and 6,214,345, as well as international patent application publications WO02088172, WO2004010957, WO2005081711, WO2005084390, WO2006132670, WO03026577, WO200700860, WO207011968 and WO205082023.

In some embodiments, the antibody is conjugated to pyrrolo[2,1-c][1,4]-benzodiazepine (PDB) or a peptide analog, derivative, or prodrug thereof. Appropriate PDB and PDB derivatives, as well as related technologies, are described in, for example, Hartley J. A. et al., Cancer Res 2010; 70(17):6849-6858; Antonow D.et al, Cancer J 2008; 14(3): 154-169; Howard P.W.et al, Bioorg Med Chem Lett 2009; 19:6463-6466 and Sagnou et al, Bioorg Med Chem Lett 2000; 10(18):2083-2086.

In some embodiments, the antibody is conjugated to a cytotoxic moiety of an anthracycline, maytansine, a calicheamicin, duocarmycin, rapamycin (CC-1065), aplysiatoxin 10, aplysiatoxin 15, irinotecan, monomethyl auristatin E, monomethyl auristatin F, PDB, or any analog, derivative or prodrug thereof.

In some embodiments, the antibody is conjugated to an anthracycline, or an analog, derivative or prodrug thereof. In some embodiments, the antibody is conjugated to maytansine, or an analog, derivative or prodrug thereof. In some embodiments, the antibody is conjugated to a calicheamicin, or an analog, derivative or prodrug thereof. In some embodiments, the antibody is conjugated to duocarmycin, or an analog, derivative or prodrug thereof. In some embodiments, the antibody is conjugated to rapamycin (CC-1065), or an analog, derivative or prodrug thereof. In some embodiments, the antibody is conjugated to aplysiatoxin 10, or an analog, derivative or prodrug thereof. In some embodiments, the antibody is conjugated to aplysiatoxin 15, or an analog, derivative, or prodrug thereof. In some embodiments, the antibody is conjugated to monomethyl auristatin E, or an analog, derivative, or prodrug thereof. In some embodiments, the antibody is conjugated to monomethyl auristatin F, or an analog, derivative, or prodrug thereof. In some embodiments, the antibody is conjugated to pyrrolo[2,1-c][1,4]-benzodiazepine, or an analog, derivative, or prodrug thereof. In some embodiments, the antibody is conjugated to irinotecan or an analog, derivative, or prodrug thereof.

In some embodiments, the antibody is conjugated to a cytokine (e.g. IL-2, IL-4, IL-6, IL-7, IL-10, IL-12, IL-13, IL-15, IL-18, IL-23, IL-24, IL-27, IL-28a, IL-28b, IL-29, KGF, IFNα, IFNβ, IFNγ, GM-CSF, CD40L, Flt3 ligand, stem cell factor, ancestine, and TNFα).

In some embodiments, the antibody is conjugated to a radioisotope or a chelate containing a radioisotope. For example, the antibody may be conjugated to a chelate adaptor (e.g., DOTA, DTPA, and thiacetam) that allows the antibody to be complexed with the radioisotope. The antibody may also or optionally contain one or more radiolabeled amino acids or other radiolabeled molecules or is conjugated to the radiolabeled amino acids or radiolabeled molecules. Non-limiting examples of radioisotopes include ³H, ¹⁴C, ¹⁵N, ³⁵S, ⁹⁰Y, ⁹⁹Tc, ¹²⁵I, ¹³¹I, ¹⁸⁶Re, ²¹³Bi, ²²⁵Ac and ²²⁷Th. For therapeutic purposes, radioisotopes emitting beta or alpha particle radiation may be used, such as ¹³¹I, ⁹⁰Y, ²¹¹At, ²¹²Bi, ⁶⁷Cu, ¹⁸⁶Re, ¹⁸⁸Re and ²¹²Pb.

The technologies for conjugating molecules to antibodies are well-known in the art. Usually, a nucleic acid molecule is covalently linked to lysine or cysteine on an antibody through an N-hydroxysuccinimide eater or maleimide functional group. It is reported that the homogeneity of a conjugate can be improved by conjugation methods using engineered cysteine or integrating unnatural amino acids. Particularly, those skilled in the art can also expect to use a tag (e.g., a tag containing Gin peptide and a Q-tag) containing glutamine serving as an acyl donor or polypeptide engineering (e.g., amino acid deletions, insertions, substitutions and mutations on a polypeptide) to generate a reactive endogenous glutamine engineered Fc-containing polypeptide. Then, a transglutaminase can be covalently cross-linked with an amine donor agent (e.g., a small molecule containing or linked to a reactive amine) to form a population of stable and homogeneous engineered Fc-containing polypeptide conjugates in which the amine donor agent is specifically conjugated to the Fc-containing polypeptide through the tag containing glutamine serving as an acyl donor tag or accessible/exposed/reactive endogenous glutamine sites (WO2012059882).

It should be understood that therapeutic agents according to the described embodiments will be administered with suitable pharmaceutically acceptable carriers, excipients, and other reagents incorporated into the preparation to provide improved transfer, delivery, tolerability, etc. A large number of appropriate preparations can be found in the pharmacopoeia known to all medicinal chemists: Remington's Pharmaceutical Sciences (the 15th edition, Mack Publishing Company, Easton, Pa. (1975)), especially Chapter 87 of Blaug and Seymour therein. These preparations include, for example, powders, pastes, ointments, gels, waxes, oils, lipids, lipid-containing (cation or anion) carriers (such as Lipofectin^{™}), DNA conjugates, anhydrous slurries, oil-in-water and water-in-oil emulsions, emulsion polyethylene glycols (polyethylene glycols of various molecular weights), semisolid gels, and semisolid mixtures containing polyethylene glycol. Any of the above mixtures is applicable to the treatment or therapy according to the present disclosure under the conditions that an active ingredient of the preparation is not inactivated by the preparation, and the preparation is physiologically compatible and tolerates the route of administration.

In an embodiment, the antibody can be used as a therapeutic agent. Such a therapeutic agent is usually used for treating, alleviating, and/or preventing a disease or pathology associated with aberrant GPC3 expression, activity, and/or signaling in a subject. A treatment regimen can be implemented using standard methods by identifying a subject, such as a human patient suffering from (or at risk of developing) GPC3-associated symptoms such as a disease or disorder associated with aberrant GPC3 expression, activity, and/or signaling. An antibody preparation, preferably an antibody preparation with high specificity and high affinity for its target antigen, is administered to a subject and will generally produce an effect due to its binding to the target. The administered antibody can eliminate or inhibit or hinder the expression, activity, and/or signal transduction function of the target (e.g., GPC3). The administered antibody can eliminate or inhibit or hinder the binding of the target (e.g., GPC3) to an endogenous ligand to which it naturally binds. For example, the antibody binds to the target and regulates, blocks, inhibits, reduces, antagonizes, neutralizes, and/or hinders the expression, activity, and/or signal transduction of GPC3 in other ways. In some embodiments, in order to treat an abnormal GPC3 expression-associated disease or disorder, an antibody having the heavy chain and light chain CDRs can be administered to a subject.

In another embodiment, antibodies against GPC3 can be used in methods known in the art related to GPC3 localization and/or quantification (e.g., measurement of GPC3 and/or a GPC3 level in an appropriate physiological sample, diagnostic methods, and protein imaging). In a given embodiment, an antibody having the specificity for GPC3 or a derivative, fragment, analog or homolog thereof and containing an antigen-binding domain derived from the antibody is used as a pharmaceutically active compound (referred to as a "therapeutic agent" hereinafter).

In another embodiment, an antibody having the specificity for GPC3 is used for separating GPC3 polypeptide through a standard technology such as immunoaffinity, chromatography, and immunoprecipitation. An antibody (or a fragment thereof) against GPC3 protein can be used for detecting proteins in a biological sample. In some embodiments, the detection of GPC3 in a biological sample can be used as part of a clinical testing procedure, for example, used for determining the efficacy of a given treatment regimen. Detection can be facilitated by conjugating (i.e., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, and acetylcholinesterase; examples of suitable prosthetic groups include streptavidin/biotin and avidin/biotin; examples of suitable fluorescent materials include umbelliferone, fluorescein, fluorescein isothiocyanate, rhodamine, dichlorotriazine fluorescein, dansyl chloride, and phycoerythrin; an example of the luminescent material includes luminol; examples of the bioluminescent materials include luciferase, fluorescein and aequorin, and examples of suitable radioactive materials include ¹²⁵I, ¹³¹I, ³⁵S or ³H.

In another embodiment, the antibody of the present disclosure can be used as a reagent for detecting the presence of GPC3 or a protein fragment thereof in a sample. In some embodiments, the antibody contains a detectable label. The antibody is a polyclonal antibody, or more preferably a monoclonal antibody. The whole antibody or a fragment (e.g., Fab, scFv, and F(ab')₂) is used. The term "label" with respect to an antibody is intended to include direct labeling of the antibody by conjugating (i.e., physically linking) a detectable substance to the antibody, and indirect labeling of the antibody by reaction with another directly labeled reagent. Examples of indirect labeling include the detection of a primary antibody with a fluorescently-labeled secondary antibody, and end-labeling of the antibody with biotin to enable detection with fluorescently-labeled streptavidin. The term "biological sample" is intended to include tissues, cells, and biological fluids separated from a subject, as well as tissues, cells, and fluids present in the subject. Therefore, the term "biological sample" used includes blood and fractions or components of blood, including serum, plasma, and lymph. In other words, the detection method of the embodiment can be used to detect an analyte mRNA, protein, or genomic DNA in a biological sample both in vitro and in vivo. For example, the analyte mRNA in-vitro detection technologies include Norhtern hybridization and in situ hybridization. The analyte protein in-vitro detection technologies include enzyme-linked immunosorbent assay (ELISA), Western blotting, immunoprecipitation, and immunofluorescence. The analyte genomic DNA in-vitro detection technologies include Southern hybridization. Immunoassay procedures are described, for example, in "ELISA: Theory and Practice: Methods in Molecular Biology ", vol. 42, J. R. Crowther (ed.) Human Press, Totowa, N. J. 1995; "immunoassay", E. Diamandis and T. Christopoulus, Academic Press, Inc. San Diego, Calif., 1996; and "Practice and Theory of Enzyme Immunoassays", P. Tijssen, Elsevier Science Publishers, Amsterdam, 1985. In addition, the analyte protein in-vivo detection technologies include the introduction of a labeled anti-analyte protein antibody into a subject. For example, the antibody may be labeled with a radioactive label, and the presence and position of the radioactive label in the subject are detected by standard imaging technology.

The antibody or the derivative, fragment, analog, and homolog thereof described herein can be incorporated into a pharmaceutical composition suitable for administration. The principles and considerations involved in the preparation of such a composition and guidance for selecting components are well-known in the art.

Such a composition usually contains an antibody and a pharmaceutically acceptable carrier. In the case that an antibody fragment is used, the minimum inhibitory fragment that specifically binds to a binding domain of a target protein may be preferred. For example, based on the variable region of the antibody, a peptide molecule that retains the ability to bind to the sequence of the target protein can be designed. Such a peptide can by chemically synthesized and/or by DNA recombination (referring to, for example, Marasco et al., Proc. Natl. Acad. Sci. USA, 90:7889-7893(1993)).

As used herein, the term "pharmaceutically acceptable carrier" is intended to include any and all solvents, dispersion media, coatings, antibacterial agents and antifungal agents, isotonic agents and absorption delaying agents, etc., compatible with pharmaceutical administration. Suitable pharmaceutically acceptable carriers are described in the latest edition of Remington's Pharmaceutical Sciences, which is a standard reference work in the art and incorporated herein by reference. Preferred examples of such carriers or diluents include but are not limited to, water, saline, Ringer's solution, dextrose solution, and 5% human serum albumin. Liposomes and non-aqueous carriers, such as immobilized oils, can also be used. The use of such media and reagents for pharmaceutically active substances is well-known in the art. Except for their incompatibility with the antibody, the use of any conventional medium or reagent in the composition is envisioned.

The pharmaceutical composition of the aforementioned embodiment is prepared to be compatible with its intended route of administration. Examples of routes of administration include parenteral administration, such as intravenous, intradermal, subcutaneous, oral (e.g., inhalation), transdermal (i.e., topical), transmucosal and rectal administration. A solution or suspension for parenteral, intradermal, or subcutaneous administration may include the following components: a sterile diluent for injection such as water, a saline solution, a fixed oil, polyethylene glycol, glycerin, propylene glycol, and other synthesized solvent; an antibacterial agent such as benzyl alcohol and methylparaben; an antioxidant such as ascorbic acid and sodium bisulfite; a chelating agent such as ethylenediaminetetraacetic acid (EDTA); a buffer such as an acetate, citrate, and phosphate; and a reagent that regulates osmotic pressure such as sodium chloride and dextrose. The pH can be regulated with an acid or base, such as hydrochloric acid and sodium hydroxide. A parenteral administration may be packaged into an ampoule, a disposable syringe, or a glass or plastic multi-dose vial.

A pharmaceutical composition suitable for injection includes sterile aqueous solutions (water-soluble herein) or dispersions and sterile powders for the extemporaneous preparation of sterile injectable solutions or dispersions. For intravenous administration, suitable pharmaceutically acceptable carriers include normal saline, bacteriostatic water, Cremophor EL^{™} (BASF, Parsippany, N.J.) or phosphate buffered saline (PBS). In all cases, the composition is required to be sterile and should be fluid to the extent of ease of injection. The composition is required to be stable under the preparation and storage conditions and is required to prevent the contamination of microorganisms such as bacteria and fungi. The carrier may be a solvent or dispersion medium containing, for example, water, ethanol, and polyol (e.g., glycerin, propylene glycol, and liquid polyethylene glycol), or an appropriate mixture thereof. For example, for dispersion, the required particle size is maintained through a coating such as a lecithin, and appropriate liquidity can be kept through a surfactant. The action of microorganisms can be prevented through various antibacterial and antifungal agents, such as paraben, chlorobutanol, phenol, ascorbic acid, and thimerosal. In many cases, the composition preferably contains an isotonic agent, such as a saccharide, a polyol (such as mannitol and sorbitol), and sodium chloride. Absorption of the composition for injection can be prolonged by adding a reagent for delaying absorption, such as aluminum monostearate and gelatin, in the composition.

As required, a sterile injectable solution can be prepared by incorporating a required amount of antibody into a suitable solvent with one or a combination (as required) of the ingredients enumerated above, filtering, and sterilizing. In general, a dispersion is prepared by incorporating an antibody into a sterile carrier containing an alkaline dispersion medium and required other ingredients among those enumerated above. For sterile powder for preparation of a sterile injectable solution, the preparation methods are vacuum-drying and freeze-drying to obtain a powder that contains an active ingredient and any additional desired ingredient from a sterile-filtered solution of these ingredients described above.

For inhalation administration, a compound is delivered as an aerosol spray from a pressurized vessel or dispenser, or nebulizer containing a suitable propellant, such as a gas such as carbon dioxide.

Systemic administration can also be performed via transmucosal or transdermal routes. For transmucosal or transdermal administration, a penetrant suitable for permeating the barrier is used in preparation. Such a penetrant is generally known in the art and includes, for example, a detergent, a bile salt, and a fusidic acid derivative for transmucosal administration. Transmucosal administration can be performed by using a nasal spray or suppository. For transdermal administration, one or more antibodies can be prepared into a paste, ointment, gel or cream as generally known in the art.

The compound can also be prepared in the form of a suppository (e.g., with a conventional suppository base such as cocoa butter and other glycerides) or a retention enema for delivery via the rectum.

In an embodiment, the antibody can be prepared with a carrier that will protect the antibody from rapid elimination from the body, such as a sustained release/controlled release preparation, including an implant and a microencapsulated delivery system. Biodegradable or biocompatible polymers such as ethylene vinyl acetate, a polyanhydride, polyglycolic acid, collagen, a polyorthoester, and polylactic acid, can be used. Preparation methods of such preparations are apparent to those skilled in the art.

It is especially advantageous to prepare parenteral compositions in the dose unit form for ease of administration and uniformity of dose. As used herein, the dose unit form refers to a physically separable unit suitable as a unit dose for a subject to be treated; and each unit contains predetermined amounts of one or more antibodies that are calculated to achieve a desired therapeutic effect when used in combination with a required drug carrier. The specification for the dose unit form of the embodiment is dictated by and directly dependent on the unique characteristics of the antibody and a particular therapeutic effect to be achieved, and the limitations inherent in the art of formulation of such antibodies for the treatment of individuals.

The pharmaceutical composition may be placed in a vessel, pack, or dispenser together with instructions for administration.

The preparation described herein may also contain more than one antibody, preferably those with complementary activities that do not adversely affect each other, depending on the particular condition to be treated. Alternatively, or in addition, a composition may contain, for example, an agent that enhances its function, such as a cytotoxic agent, a cytokine, a chemotherapeutic agent, and a growth inhibitor. Such molecules are appropriately present together in amounts effective for the intended purpose. For example, the molecules may be present together in a kit or used together.

In an embodiment, one or more antibodies can be administered in combination treatment, that is, administered in combination with other agents such as a therapeutic agent (which can be used for treating a pathological condition or disorder, such as various forms of cancer, autoimmune disorders, and inflammatory diseases). The term "combination" herein refers to the substantially synchronous, simultaneous, or sequential administration of reagents. In the case of sequential administration, when a second compound is started to be administered, the first compound of the two compounds is still preferably detected at a treatment site at an effective concentration. In one case, "combination" may also be that the kit contains both the antibody of the present disclosure and other therapeutic agents. For example, combination treatment may include co-preparation and/or co-administration of one or more antibodies described herein and one or more additional therapeutic agents (e.g., one or more cytokine and growth factor inhibitors, immunosuppressants, anti-inflammatory agents, metabolic inhibitors, enzyme inhibitors, and/or cytotoxins or cytostatic agents, as described in more detail below). Such combination treatment can advantageously utilize lower doses of the therapeutic agents administered, thus avoiding possible toxicity or complications associated with various monotherapies.

In an embodiment, compared with a corresponding therapeutic regimen without administration of the anti-GPC3 antibody, the therapeutic regimen effectively reduces the cytokine release in the subject that is associated with the administration of the T cell activation therapeutic agent.

For purposes of clarity and conciseness of description, features are described herein as part of the same or separate embodiments. However, it should be understood that the scope of the present disclosure may include some embodiments with combinations of all or some of the described features.

### Example

### Example 1. Antigen expression and construction of stably-transfected cells

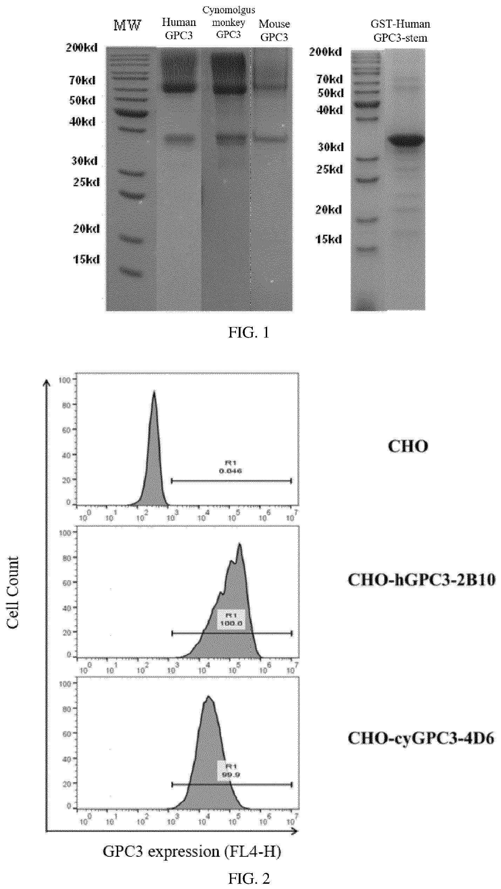

The extracellular domain of human GPC3 (25-563) was amplified by PCR using human GPC3 (Sino biological, HG10088) as a template, and then transfected into HEK 293E cells to express recombinant human GPC3 protein (SEQ ID NO. 1). Or the membrane-proximal sequence (aa 524-563) was inserted into the EcoRI site of the pGEX3X vector by the Gibson assembly method. The plasmid was transfected into E. coli BL21 (DE3). The recombinant GST-human GPC3 stem protein (SEQ ID NO. 2) was obtained by IPTG-induced expression. The recombinant GST-cynomolgus monkey GPC3 stem protein (SEQ ID NO. 3) and recombinant GST-mouse GPC3 stem protein (SEQ ID NO. 4) were expressed in the same way. FIG. 1 shows non-reduced SDS-PAGE results for recombinant GPC3 proteins.

The Lenti vector containing the full-length sequence of human and rhesus GPC3 and the packaging plasmid (Genecoepia) were co-transfected into HEK293T cells. After 48 hours of transfection, the supernatant of the culture medium containing pseudovirus was collected. 10 µL of supernatant of the culture medium was used to infect 1 × 10⁶ CHO cells, and puromycin was added for resistance selection. Finally, stably-transfected cells CHO-hGPC3 (clone 2B10) and CHO-cynoGPC3 (clone 4D6) with high expression of human or rhesus GPC3 were isolated. FIG. 2 shows flow cytometry results for GPC3 stably-transfected cells.

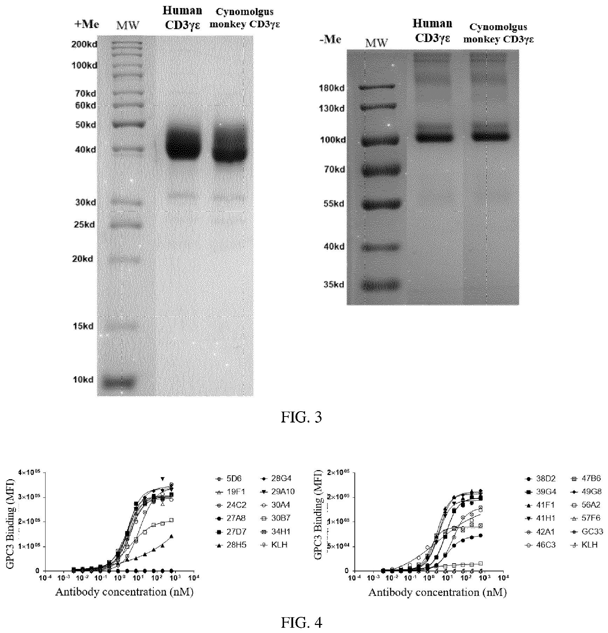

Construction of human or monkey CD3 εγ heterodimers: Nucleotide sequences of extracellular regions of human CD3γ (UniProt P09693, Gln23-Asn116) and CD3ε (UniProt P07766, Gln23-Asp126) were synthesized. The C-terminals were respectively fused with a human IgG Fc hole or Fc knob. A human CD3εγ-Fc heterodimer was formed by expression (an amino acid sequence of human CD3γ IgG Fc (hole) is shown as SEQ ID NO: 5, and an amino acid sequence of human CD3ε IgG Fc (knob) is shown as SEQ ID NO: 6). Cynomolgus monkey CD3 γ (UniProt Q95LI7, Gln23-Asn110) and CD3ε (UniProt Q95LI5, Gln22-Asp117) were also synthesized. The C-terminal was fused to cynomolgus monkey IgG Fc hole or Fc knob. The Cynomolgus monkey CD3εγ-Fc heterodimer was formed by expression. The amino acid sequence of Cynomolgus monkey CD3γ IgG Fc (hole) is shown in SEQ ID NO. 7, and the amino acid sequence of Cynomolgus monkey CD3γ IgG Fc (knob) is shown in SEQ ID NO. 8. Recombinant plasmids expressing CD3y-Fc and CD3ε-Fc were mixed with 3 mg/mL of PEI (Polysciences, # 24765-2) and co-transfected into HEK 293E cells (culture medium OPM-293 CD03 DPM). After 7 days of culturing at 37°C, 120 rpm, and 5% CO₂, the supernatant of the culture medium was collected and purified by the Protein A affinity chromatography to obtain recombinant human or cynomolgus monkey CD3 εγ-Fc protein with an SDS-PAGE purity higher than 95% (FIG. 3).

### Example 2. Animal Immunization and Preparation of GPC3 Antibody

### 1. GPC3 Antibody Screening

Female Balb/C mice aged 4-6 weeks were selected. Transient expression plasmids were constructed using the full-length or membrane-proximal sequence of the human GPC3 extracellular domain (aa 524-563). Mice were immunized with DNA by a gene gun, with 20 µg plasmid/mouse, once a week. Serum titer was determined after 8 immunizations. CHO-hGPC3 stably-transfected cells (5 × 10⁶ cells/mouse) were used for boost immunization. Three days later, the mice were killed by cervical dislocation, and the spleens and peripheral lymph nodes of the mice were collected. Total RNA was extracted after grinding and centrifugation. After reverse transcription, PCR amplification was performed using light and heavy chain specific primers, respectively, to obtain light and heavy chain variable region cDNA libraries of the antibody. Using the phage antibody library display technology, the GPC3 immune library based on filamentous phage M13 was constructed for subsequent phage panning.

Using a classical panning mode, human recombinant GPC3 protein was coated on an Immuno tube, and 1 mL of GPC3 phage immune library was added for incubation and panning. The phages displaying high-affinity Fab were eluted and infected with TG1 bacteria. The phages were prepared again for the next round of panning. After two rounds of panning and screening, the single clone was inoculated into a 96-well U-shaped plate for IPTG induction expression. The supernatant of the culture medium was taken for ELISA. A total of 323 positive clones recognizing the membrane-proximal end of the human GPC3 extracellular region were obtained.

Partial clones were selected by sequencing. The light and heavy chain genes were cloned into eukaryotic expression vectors containing antibody light chain constant region or human IgG1 heavy chain constant region CH1-CH3, respectively, and co-transfected into HEK293E cells. After 5-6 days of culture at 37°C, 120 rpm, and 5% CO₂, the supernatant of the culture medium was collected and purified by the Protein A chromatography column to obtain an IgG anti-GPC3 chimeric antibody. The control antibody GC33 was synthesized according to Nakano K et al. (2009) and expressed as a human IgG1 chimeric antibody (abbreviated as GC33).

The GPC3 antibody variable region sequences are shown below:

**Table 1. GPC3 murine antibody variable region sequences**

| GPC 3 muri ne antib ody | HCVR | | HCD R1 | HCD R2 | HCD R3 | LCVR | | LCD R1 | LCD R2 | LCD R3 |
|---|---|---|---|---|---|---|---|---|---|---|
| | Amin o Acid Seque nce | Nucle otide Seque nce | Amin o Acid Seque nce | Amin o acid Seque nce | Amin o Acid Sequ ence | Amin o acid Sequ ence | Nucle otide Seque nce | Amin o acid Sequ ence | Amin o Acid Sequ ence | Amin o acid Sequ ence |
| 5D6 | 9 | 10 | 11 | 12 | 13 | 14 | 15 | 16 | 17 | 18 |
| 29A1 0 | 19 | 20 | 11 | 21 | 13 | 22 | 23 | 16 | 17 | 18 |
| 34H1 | 9 | 10 | 11 | 12 | 13 | 24 | 25 | 16 | 17 | 26 |
| 19F1 | 9 | 10 | 11 | 12 | 13 | 27 | 28 | 16 | 17 | 29 |
| 30B7 | 30 | 31 | 32 | 33 | 13 | 34 | 35 | 16 | 17 | 18 |
| 28G4 | 19 | 20 | 11 | 21 | 13 | 36 | 37 | 38 | 17 | 39 |
| 30A4 | 40 | 41 | 11 | 21 | 13 | 42 | 43 | 16 | 17 | 18 |
| 38D2 | 9 | 10 | 11 | 12 | 13 | 44 | 45 | 46 | 47 | 48 |
| 39G4 | 9 | 10 | 11 | 12 | 13 | 49 | 50 | 16 | 17 | 51 |
| 41F1 | 9 | 10 | 11 | 12 | 13 | 52 | 53 | 16 | 17 | 54 |
| 41H1 | 9 | 10 | 11 | 12 | 13 | 55 | 56 | 16 | 17 | 57 |
| 49G8 | 9 | 10 | 11 | 12 | 13 | 58 | 59 | 60 | 17 | 61 |
| 46C3 | 62 | 63 | 64 | 65 | 66 | 67 | 68 | 16 | 17 | 69 |
| 42A1 | 70 | 71 | 11 | 72 | 73 | 74 | 75 | 16 | 17 | 76 |
| 47B6 | 77 | 78 | 79 | 80 | 81 | 82 | 83 | 16 | 17 | 69 |

### 5D6

The amino acid sequence of the heavy chain VH is shown in SEQ ID NO. 9; its encoding nucleic acid is shown in SEQ ID NO. 10; and its HCDR1, HCDR2, HCDR3 are shown in SEQ ID NO. 11, 12, 13, respectively.

<------------FR1------------> CDR1 <-----FR2----> CDR2 <------------
QVQLQQSGAELVRPGASVKLSCKALGYTFADYEIHWVKQTPVHGLEWIGAIHPG
SGGTAYNQTFEGKATLTADKSSTTA
-------FR3--------> CDR3 <---FR4--->
YMELSSLTSEDSAVYYCTRYYSFAYWGQGTLVTVSA

### Nucleic acid sequence

CAGGTCCAGCTGCAGCAGTCTGGGGCTGAGCTGGTGAGGCCTGGGGCTTCAGT
GAAGCTGTCCTGCAAGGCTTTGGGCTACACATTTGCTGACTATGAAATACACTG
GGTGAAGCAGACACCTGTGCATGGCCTGGAGTGGATTGGAGCTATTCATCCAG
GAAGTGGTGGTACTGCCTACAATCAGACGTTCGAGGGCAAGGCCACACTGACT
GCAGACAAATCCTCCACCACAGCCTACATGGAGCTCAGCAGCCTGACATCTGA
GGACTCTGCTGTCTATTACTGTACAAGATACTATTCCTTTGCTTACTGGGGCCAA
GGGACTCTGGTCACTGTCTCTGCA

The amino acid sequence of light chain VK is shown in SEQ ID NO. 14; its encoding nucleic acid is shown in SEQ ID NO. 15; and its LCDR1, LCDR2, LCDR3 are shown in SEQ ID NO. 16, 17, 18, respectively.

<---------FR1--------->          CDR1          <-----FR2----->   CDR2   <----------------
DVLMTQTPLSLPVSLGDQASISCRSSQSLVHSNGNTYLHWYLQKPGQSPKLLIYK
VSNRFSGVPDRFSGSGSGTDFTLK
--FR3-------->     CDR3   <---FR4--->
ISRVEAEDLGVYFCSQSTHVPLTFGAGTKLELK

### Nucleic acid sequence

GATGTTTTGATGACCCAAACTCCACTCTCCCTGCCTGTCAGTCTTGGAGATCAA
GCCTCCATCTCTTGCAGATCTAGTCAGAGCCTTGTACACAGTAATGGAAACACC
TATTTACATTGGTACCTGCAGAAGCCAGGCCAGTCTCCAAAGCTCCTGATCTAC
AAAGTTTCCAACCGATTTTCTGGGGTCCCAGACAGGTTCAGTGGCAGTGGATC

AGGGACAGATTTCACACTCAAGATCAGCAGAGTGGAGGCTGAGGATCTGGGA
GTTTATTTCTGCTCTCAAAGTACACATGTTCCGCTCACGTTCGGTGCTGGGACC
AAGCTGGAGCTGAAA

### 29A10

The amino acid sequence of the heavy chain VH is shown in SEQ ID NO. 19; its encoding nucleic acid is shown in SEQ ID NO. 20; and its HCDR1, HCDR2, HCDR3 are shown in SEQ ID NO. 11, 21, 13, respectively.

<-----------FR1-----------> CDR1 <-----FR2----> CDR2 <-----------
EVQLQQSGAELVRPGASVKLSCKALGYTFADYEIHWVKQTPVHGLEWIGAIHPG
SGGTAYNQTFKGKATLTADKSSTTA
-------FR3--------> CDR3 <---FR4--->
YMELSSLTSEDSAVYYCTRYYSFAYWGQGTLVTVSA

### Nucleic acid sequence

GAGGTTCAACTGCAGCAGTCTGGGGCTGAGCTGGTGAGGCCTGGGGCTTCAGT
GAAGCTGTCCTGCAAGGCTTTGGGCTACACATTTGCTGACTATGAAATACACTG
GGTGAAGCAGACACCTGTGCATGGCCTGGAGTGGATTGGAGCTATTCATCCAG
GAAGTGGTGGTACTGCCTACAATCAGACGTTCAAGGGCAAGGCCACACTGACT
GCAGACAAATCCTCCACCACAGCCTACATGGAGCTCAGCAGCCTGACATCTGA
GGACTCTGCTGTCTATTACTGTACAAGATACTATTCCTTTGCTTACTGGGGCCAA
GGGACTCTGGTCACTGTCTCTGCA

The amino acid sequence of light chain VK is shown in SEQ ID NO. 22; its encoding nucleic acid is shown in SEQ ID NO. 23; and its LCDR1, LCDR2, LCDR3 are shown in SEQ ID NO. 16, 17, 18, respectively.

<---------FR1--------->          CDR1          <-----FR2----->   CDR2   <----------------
DVVMTQTPLSLPVSLGDQASISCRSSQSLVHSNGNTYLHWYLQKPGQSPKLLIYK
VSNRFSGVPDRFSGSGSGTDFTLK
--FR3-------->     CDR3    <---FR4--->
ISRVEAEDLGVYFCSQSTHVPLTFGAGTKLEIK

### Nucleic acid sequence

GATGTTGTGATGACCCAAACTCCACTCTCCCTGCCTGTCAGTCTTGGAGATCAA

GCCTCCATCTCTTGCAGATCTAGTCAGAGCCTTGTACACAGTAATGGAAACACC
TATTTACATTGGTACCTGCAGAAGCCAGGCCAGTCTCCAAAGCTCCTGATCTAC
AAAGTTTCCAACCGATTTTCTGGGGTCCCAGACAGGTTCAGTGGCAGTGGATC
AGGGACAGATTTCACACTCAAGATCAGCAGAGTGGAGGCTGAGGATCTGGGA
GTTTATTTCTGCTCTCAAAGTACACATGTTCCGCTCACGTTCGGTGCTGGGACC
AAGCTGGAAATAAAA

### 34H1

The amino acid sequence of the heavy chain VH is shown in SEQ ID NO. 9; its encoding nucleic acid is shown in SEQ ID NO. 10; and its HCDR1, HCDR2, HCDR3 are shown in SEQ ID NO. 11, 12, 13, respectively.

<------------FR1------------> CDR1 <-----FR2----> CDR2 <------------
QVQLQQSGAELVRPGASVKLSCKALGYTFADYEIHWVKQTPVHGLEWIGAIHPG
SGGTAYNQTFEGKATLTADKSSTTA
-------FR3--------> CDR3 <---FR4--->
YMELSSLTSEDSAVYYCTRYYSFAYWGQGTLVTVSA

### Nucleic acid sequence

CAGGTCCAGCTGCAGCAGTCTGGGGCTGAGCTGGTGAGGCCTGGGGCTTCAGT
GAAGCTGTCCTGCAAGGCTTTGGGCTACACATTTGCTGACTATGAAATACACTG
GGTGAAGCAGACACCTGTGCATGGCCTGGAGTGGATTGGAGCTATTCATCCAG
GAAGTGGTGGTACTGCCTACAATCAGACGTTCGAGGGCAAGGCCACACTGACT
GCAGACAAATCCTCCACCACAGCCTACATGGAGCTCAGCAGCCTGACATCTGA
GGACTCTGCTGTCTATTACTGTACAAGATACTATTCCTTTGCTTACTGGGGCCAA
GGGACTCTGGTCACTGTCTCTGCA

The amino acid sequence of light chain VK is shown in SEQ ID NO. 24; its encoding nucleic acid is shown in SEQ ID NO. 25; and its LCDR1, LCDR2, LCDR3 are shown in SEQ ID NO. 16, 17, 26, respectively.

<-----------FR1----------->          CDR1          <-----FR2----->   CDR2   <-----------------
DVVMTQTPLSLPVSLGDQASISCRSSQSLVHSNGNTYLHWYLQKPGQSPKLLIYK
VSNRFSGVPDRFSGSGSGTDFTLK
--FR3--------->     CDR3    <---FR4--->

ISRVEAEDLGVYFCSQSIHVPLTFGAGTKLELK

### Nucleic acid sequence

GATGTTGTGATGACCCAAACTCCACTCTCCCTGCCTGTCAGTCTTGGAGATCAA
GCCTCCATCTCTTGCAGATCTAGTCAGAGCCTTGTACACAGTAATGGAAACACC
TATTTACATTGGTACCTGCAGAAGCCAGGCCAGTCTCCAAAGCTCCTGATCTAC
AAAGTTTCCAACCGATTTTCTGGGGTCCCAGACAGGTTCAGTGGCAGTGGATC
AGGGACAGATTTCACACTCAAGATCAGCAGAGTGGAGGCTGAGGATCTGGGA
GTTTATTTCTGCTCTCAAAGTATACATGTTCCGCTCACGTTCGGTGCTGGGACCA
AGCTGGAGCTGAAA

### 19F1

The amino acid sequence of the heavy chain VH is shown in SEQ ID NO. 9; its encoding nucleic acid is shown in SEQ ID NO. 10; and its HCDR1, HCDR2, HCDR3 are shown in SEQ ID NO. 11, 12, 13, respectively.

<------------FR1------------> CDR1 <-----FR2----> CDR2 <------------
QVQLQQSGAELVRPGASVKLSCKALGYTFADYEIHWVKQTPVHGLEWIGAIHPG
SGGTAYNQTFEGKATLTADKSSTTA
-------FR3--------> CDR3 <---FR4--->
YMELSSLTSEDSAVYYCTRYYSFAYWGQGTLVTVSA

### Nucleic acid sequence

CAGGTCCAGCTGCAGCAGTCTGGGGCTGAGCTGGTGAGGCCTGGGGCTTCAGT
GAAGCTGTCCTGCAAGGCTTTGGGCTACACATTTGCTGACTATGAAATACACTG
GGTGAAGCAGACACCTGTGCATGGCCTGGAGTGGATTGGAGCTATTCATCCAG
GAAGTGGTGGTACTGCCTACAATCAGACGTTCGAGGGCAAGGCCACACTGACT
GCAGACAAATCCTCCACCACAGCCTACATGGAGCTCAGCAGCCTGACATCTGA
GGACTCTGCTGTCTATTACTGTACAAGATACTATTCCTTTGCTTACTGGGGCCAA
GGGACTCTGGTCACTGTCTCTGCA

The amino acid sequence of light chain VK is shown in SEQ ID NO. 27; its encoding nucleic acid is shown in SEQ ID NO. 28; and its LCDR1, LCDR2, LCDR3 are shown in SEQ ID NO. 16, 17, 29, respectively.

<-----------FR1----------> CDR1 <-----FR2-----> CDR2 <-----------

DVLMTQTPLSLPVSLGDQASISCRSSQSLVHSNGNTYLHWYLQKPGQSPKLLIYK
VSNRFSGVPDRFSGSGSGTDFTLK
--FR3--------> CDR3 <---FR4--->
ISRVEAEDLGVYFCSQSTHIPTFGGGTKLELK

### Nucleic acid sequence

GATGTTTTGATGACCCAAACTCCACTCTCCCTGCCTGTCAGTCTTGGAGATCAA
GCCTCCATCTCTTGCAGATCTAGTCAGAGCCTTGTACACAGTAATGGAAACACC
TATTTACATTGGTACCTGCAGAAGCCAGGCCAGTCTCCAAAGCTCCTGATCTAC
AAAGTTTCCAACCGATTTTCTGGGGTCCCAGACAGGTTCAGTGGCAGTGGATC
AGGGACAGATTTCACACTCAAGATCAGCAGAGTGGAGGCTGAGGATCTGGGA
GTTTATTTCTGCTCTCAAAGTACACATATTCCCACGTTCGGTGGAGGCACCAAG
CTGGAGCTGAAA

### 30B7

The amino acid sequence of the heavy chain VH is shown in SEQ ID NO. 30; its encoding nucleic acid is shown in SEQ ID NO. 31; and its HCDR1, HCDR2, HCDR3 are shown in SEQ ID NO. 32, 33 and 13, respectively.

<-----------FR1-----------> CDR1 <-----FR2----> CDR2 <-----------

QVQLQQSGAELVRPGASVRLSCKALGYTFTDYEMHWVKQTPVHGLEWIGAIHP

GSGGTAYNQKFKGKATLTADKSSSTA

-------FR3--------> CDR3 <---FR4--->

YMELSSLTSEDSAVYYCTRYYSFAYWGQGTLVTVSA

### Nucleic acid sequence

CAGGTTCAGCTGCAGCAGTCTGGGGCTGAGCTGGTGAGGCCTGGGGCTTCAGT
GAGGCTGTCCTGCAAGGCTTTGGGCTACACATTTACTGACTATGAAATGCACTG
GGTGAAGCAGACACCTGTGCATGGCCTGGAATGGATTGGAGCTATTCATCCAG
GAAGTGGTGGTACTGCCTACAATCAGAAGTTCAAGGGCAAGGCCACACTGACT
GCAGACAAATCCTCCAGCACAGCCTACATGGAGCTCAGCAGCCTGACATCTGA
GGACTCTGCTGTCTATTACTGTACAAGATACTATTCCTTTGCTTACTGGGGCCAA
GGGACTCTGGTCACTGTCTCTGCA

The amino acid sequence of light chain VK is shown in SEQ ID NO. 34; its encoding nucleic acid is shown in SEQ ID NO. 35; and its LCDR1, LCDR2, LCDR3 are shown in SEQ ID NO. 16, 17, 18, respectively.

<-----------FR1----------->          CDR1          <-----FR2----->   CDR2   <-----------------
DVLMTQTPLSLPVSLGDQASISCRSSQSLVHSNGNTYLHWYLQKPGQSPKLLIYK
VSNRFSGVPDRFSGSGSGTDFTLK
--FR3--------->     CDR3     <---FR4-->
ISRVEAEDLGVYFCSQSTHVPLTFGAGTKLEIK

### Nucleic acid sequence

GATGTTTTGATGACCCAAACTCCACTCTCCCTGCCTGTCAGTCTTGGAGATCAA
GCCTCCATCTCTTGCAGATCTAGTCAGAGCCTTGTACACAGTAATGGAAACACC
TATTTACATTGGTACCTGCAGAAGCCAGGCCAGTCTCCAAAGCTCCTGATCTAC
AAAGTTTCCAACCGATTTTCTGGGGTCCCAGACAGGTTCAGTGGCAGTGGATC
AGGGACAGATTTCACACTCAAGATCAGCAGAGTGGAGGCTGAGGATCTGGGA
GTTTATTTCTGCTCTCAAAGTACACATGTTCCGCTCACGTTCGGTGCTGGGACC
AAGCTGGAAATCAAA

### 28G4

The amino acid sequence of the heavy chain VH is shown in SEQ ID NO. 19; its encoding nucleic acid is shown in SEQ ID NO. 20; and its HCDR1, HCDR2, HCDR3 are shown in SEQ ID NO. 11, 21, 13, respectively.

<-----------FR1-----------> CDR1 <-----FR2----> CDR2 <-----------

EVQLQQSGAELVRPGASVKLSCKALGYTFADYEIHWVKQTPVHGLEWIGAIHPG

SGGTAYNQTFKGKATLTADKSSTTA

-------FR3--------> CDR3 <---FR4--->

YMELSSLTSEDSAVYYCTRYYSFAYWGQGTLVTVSA

### Nucleic acid sequence

GAGGTTCAACTGCAGCAGTCTGGGGCTGAGCTGGTGAGGCCTGGGGCTTCAGT
GAAGCTGTCCTGCAAGGCTTTGGGCTACACATTTGCTGACTATGAAATACACTG
GGTGAAGCAGACACCTGTGCATGGCCTGGAGTGGATTGGAGCTATTCATCCAG
GAAGTGGTGGTACTGCCTACAATCAGACGTTCAAGGGCAAGGCCACACTGACT
GCAGACAAATCCTCCACCACAGCCTACATGGAGCTCAGCAGCCTGACATCTGA

GGACTCTGCTGTCTATTACTGTACAAGATACTATTCCTTTGCTTACTGGGGCCAA
GGGACTCTGGTCACTGTCTCTGCA

The amino acid sequence of light chain VK is shown in SEQ ID NO. 36; its encoding nucleic acid is shown in SEQ ID NO. 37; and its LCDR1, LCDR2, LCDR3 are shown in SEQ ID NO. 38, 17, 39, respectively.

<---------FR1--------->          CDR1          <-----FR2----->    CDR2    <----------------

DVLMTQTPLSLPVSLGDQASISCRSSQSIVHSNGNTYLQWYLQKPGQSPKLLIYK

VSNRFSGVPDRFSGSGSGTDFTLK

--FR3-------->     CDR3    <---FR4--->

ISRVEAEDLGVYYCFQGSHVPYTFGGGTKLEIK

### Nucleic acid sequence

GATGTTTTGATGACCCAAACTCCACTCTCCCTGCCTGTCAGTCTTGGAGATCAA
GCCTCCATCTCTTGCAGATCTAGTCAGAGCATTGTACATAGTAATGGAAACACCT
ATTTACAATGGTACCTGCAGAAACCAGGCCAGTCTCCAAAGCTCCTGATCTACA
AAGTTTCCAACCGATTTTCTGGGGTCCCAGACAGGTTCAGTGGCAGTGGATCA
GGGACAGATTTCACACTCAAGATCAGCAGAGTGGAGGCTGAGGATCTGGGAGT
TTATTACTGCTTTCAAGGTTCACATGTTCCGTACACGTTCGGAGGGGGGACCAA
GCTGGAAATAAAA

### 30A4

The amino acid sequence of the heavy chain VH is shown in SEQ ID NO. 40; its encoding nucleic acid is shown in SEQ ID NO. 41; and its HCDR1, HCDR2, HCDR3 are shown in SEQ ID NO. 11, 21, 13, respectively.

<-----------FR1-----------> CDR1 <-----FR2----> CDR2 <-----------

QVQLQQSGAELVRPGASVKLSCKALGYAFADYEIHWVKQTPVHGLEWIGAIHPG

SGGTAYNQTFKGKATLTADKSSTTA

-------FR3--------> CDR3 <---FR4--->

YMELSSLTSEDSAVYYCTRYYSFAYWGQGTLVTVSA

### Nucleic acid sequence

CAGGTCCAGCTGCAGCAGTCTGGGGCTGAGCTGGTGAGGCCTGGGGCTTCAGT
GAAGCTGTCCTGCAAGGCTTTGGGCTACGCATTTGCTGACTATGAAATACACTG

GGTGAAGCAGACACCTGTGCATGGCCTGGAGTGGATTGGAGCTATTCATCCAG
GAAGTGGTGGTACTGCCTACAATCAGACGTTCAAGGGCAAGGCCACACTGACT
GCAGACAAATCCTCCACCACAGCCTACATGGAGCTCAGCAGCCTGACATCTGA
GGACTCTGCTGTCTATTACTGTACAAGATACTATTCCTTTGCTTACTGGGGCCAA
GGGACTCTGGTCACTGTCTCTGCA

The amino acid sequence of light chain VK is shown in SEQ ID NO. 42; its encoding nucleic acid is shown in SEQ ID NO. 43; and its LCDR1, LCDR2, LCDR3 are shown in SEQ ID NO. 16, 17, 18, respectively.

<---------FR1---------> CDR1 <-----FR2-----> CDR2 <-----------------
DVVMTQTPLSLPVSLGDQASISCRSSQSLVHSNGNTYLHWYLQKPGQSPKLLIYK
VSNRFSGVPDRFSGSGSGTDFTLK
--FR3--------> CDR3 <---FR4-->
ISRVEAEDLGVYFCSQSTHVPLTFGAGTKLELK

### Nucleic acid sequence

GATGTTGTGATGACCCAAACTCCACTCTCCCTGCCTGTCAGTCTTGGAGATCAA
GCCTCCATCTCTTGCAGATCTAGTCAGAGCCTTGTACACAGTAATGGAAACACC
TATTTACATTGGTACCTGCAGAAGCCAGGCCAGTCTCCAAAGCTCCTGATCTAC
AAAGTTTCCAACCGATTTTCTGGGGTCCCAGACAGGTTCAGTGGCAGTGGATC
AGGGACAGATTTCACACTCAAGATCAGCAGAGTGGAGGCTGAGGATCTGGGA
GTTTATTTCTGCTCTCAAAGTACACATGTTCCGCTCACGTTCGGTGCTGGGACC
AAGCTGGAGCTGAAA

### 38D2

The amino acid sequence of the heavy chain VH is shown in SEQ ID NO. 9; its encoding nucleic acid is shown in SEQ ID NO. 10; and its HCDR1, HCDR2, HCDR3 are shown in SEQ ID NO. 11, 12, 13, respectively.

<------------FR1------------> CDR1 <-----FR2----> CDR2 <------------
QVQLQQSGAELVRPGASVKLSCKALGYTFADYEIHWVKQTPVHGLEWIGAIHPG
SGGTAYNQTFEGKATLTADKSSTTA
-------FR3--------> CDR3 <---FR4--->
YMELSSLTSEDSAVYYCTRYYSFAYWGQGTLVTVSA

### Nucleic acid sequence

CAGGTCCAGCTGCAGCAGTCTGGGGCTGAGCTGGTGAGGCCTGGGGCTTCAGT
GAAGCTGTCCTGCAAGGCTTTGGGCTACACATTTGCTGACTATGAAATACACTG
GGTGAAGCAGACACCTGTGCATGGCCTGGAGTGGATTGGAGCTATTCATCCAG
GAAGTGGTGGTACTGCCTACAATCAGACGTTCGAGGGCAAGGCCACACTGACT
GCAGACAAATCCTCCACCACAGCCTACATGGAGCTCAGCAGCCTGACATCTGA
GGACTCTGCTGTCTATTACTGTACAAGATACTATTCCTTTGCTTACTGGGGCCAA
GGGACTCTGGTCACTGTCTCTGCA

The amino acid sequence of light chain VK is shown in SEQ ID NO. 44; its encoding nucleic acid is shown in SEQ ID NO. 45; and its LCDR1, LCDR2, LCDR3 are shown in SEQ ID NO. 46, 47, 48, respectively.

<---------FR1--------->          CDR1          <-----FR2----->   CDR2   <-----------------
DVVMTQTPLSLPVSLGDQASISCRSSQSLENSNGNTYLNWYLQKPGQSPQLLIYR
VSNRFSGVLDRFSGSGSGTDFTLK
--FR3-------->     CDR3     <---FR4-->
ISRVEAEDLGVYFCLQVTHVPYTFGGGTKLEIK

### Nucleic acid sequence

GATGTTGTGATGACCCAAACTCCACTCTCCCTGCCTGTCAGTCTTGGAGATCAA
GCCTCCATCTCTTGCAGGTCTAGTCAGAGCCTTGAAAACAGTAATGGAAACAC
CTATTTGAACTGGTACCTCCAGAAACCAGGCCAGTCTCCACAGCTCCTGATCTA
CAGGGTTTCCAACCGATTTTCTGGGGTCCTAGACAGGTTCAGTGGTAGTGGATC
AGGGACAGATTTCACACTGAAAATCAGCAGAGTGGAGGCTGAGGATTTGGGA
GTTTATTTCTGCCTCCAAGTTACACATGTCCCGTACACGTTCGGAGGGGGGACC
AAGCTGGAAATAAAA

### 39G4

The amino acid sequence of the heavy chain VH is shown in SEQ ID NO. 9; its encoding nucleic acid is shown in SEQ ID NO. 10; and its HCDR1, HCDR2, HCDR3 are shown in SEQ ID NO. 11, 12, 13, respectively.

<-----------FR1-----------> CDR1 <-----FR2----> CDR2 <-----------
QVQLQQSGAELVRPGASVKLSCKALGYTFADYEIHWVKQTPVHGLEWIGAIHPG

SGGTAYNQTFEGKATLTADKSSTTA

-------FR3--------> CDR3 <---FR4--->

YMELSSLTSEDSAVYYCTRYYSFAYWGQGTLVTVSA

### Nucleic acid sequence

CAGGTCCAGCTGCAGCAGTCTGGGGCTGAGCTGGTGAGGCCTGGGGCTTCAGT
GAAGCTGTCCTGCAAGGCTTTGGGCTACACATTTGCTGACTATGAAATACACTG
GGTGAAGCAGACACCTGTGCATGGCCTGGAGTGGATTGGAGCTATTCATCCAG
GAAGTGGTGGTACTGCCTACAATCAGACGTTCGAGGGCAAGGCCACACTGACT
GCAGACAAATCCTCCACCACAGCCTACATGGAGCTCAGCAGCCTGACATCTGA
GGACTCTGCTGTCTATTACTGTACAAGATACTATTCCTTTGCTTACTGGGGCCAA
GGGACTCTGGTCACTGTCTCTGCA

The amino acid sequence of light chain VK is shown in SEQ ID NO. 49; its encoding nucleic acid is shown in SEQ ID NO. 50; and its LCDR1, LCDR2, LCDR3 are shown in SEQ ID NO. 16, 17, 51, respectively.

<---------FR1---------> CDR1 <-----FR2-----> CDR2 <-----------------
DVVVTQTPLSLPVSLGDQASISCRSSQSLVHSNGNTYLHWYLQKPGQSPKLLIYK
VSNRFSGVPDRFSGSGSGTDFTLK
--FR3--------> CDR3 <---FR4-->
ISRVEAEDLGVYFCSQSTHVPTFGGGTKLEIK

### Nucleic acid sequence

GATGTTGTGGTGACTCAAACTCCACTCTCCCTGCCTGTCAGTCTTGGAGATCAG
GCCTCCATCTCTTGCAGATCTAGTCAGAGCCTTGTACACAGTAATGGAAACACC
TATTTACATTGGTACCTGCAGAAGCCAGGCCAGTCTCCAAAGCTCCTGATCTAC
AAAGTTTCCAACCGATTTTCTGGGGTCCCAGACAGGTTCAGTGGCAGTGGATC
AGGGACAGATTTCACACTCAAGATCAGCAGAGTGGAGGCTGAGGATCTGGGA
GTTTATTTCTGCTCTCAAAGTACACATGTTCCGACGTTCGGTGGAGGCACCAAG
CTGGAAATCAAA

### 41F1

The amino acid sequence of the heavy chain VH is shown in SEQ ID NO. 9; its encoding nucleic acid is shown in SEQ ID NO. 10; and its HCDR1, HCDR2, HCDR3 are shown in SEQ ID NO. 11, 12, 13, respectively.

<-----------FR1-----------> CDR1 <-----FR2----> CDR2 <-----------

QVQLQQSGAELVRPGASVKLSCKALGYTFADYEIHWVKQTPVHGLEWIGAIHPG

SGGTAYNQTFEGKATLTADKSSTTA

-------FR3-------> CDR3 <---FR4--->

YMELSSLTSEDSAVYYCTRYYSFAYWGQGTLVTVSA

### Nucleic acid sequence

CAGGTCCAGCTGCAGCAGTCTGGGGCTGAGCTGGTGAGGCCTGGGGCTTCAGT
GAAGCTGTCCTGCAAGGCTTTGGGCTACACATTTGCTGACTATGAAATACACTG
GGTGAAGCAGACACCTGTGCATGGCCTGGAGTGGATTGGAGCTATTCATCCAG
GAAGTGGTGGTACTGCCTACAATCAGACGTTCGAGGGCAAGGCCACACTGACT
GCAGACAAATCCTCCACCACAGCCTACATGGAGCTCAGCAGCCTGACATCTGA
GGACTCTGCTGTCTATTACTGTACAAGATACTATTCCTTTGCTTACTGGGGCCAA
GGGACTCTGGTCACTGTCTCTGCA

The amino acid sequence of light chain VK is shown in SEQ ID NO. 52; its encoding nucleic acid is shown in SEQ ID NO. 53; and its LCDR1, LCDR2, LCDR3 are shown in SEQ ID NO. 16, 17, 54, respectively.

<---------FR1--------->          CDR1          <-----FR2----->   CDR2   <-----------------
DVLMTQTPLSLPVSLGDQASISCRSSQSLVHSNGNTYLHWYLQKPGQSPKLLIYK
VSNRFSGVPDRFSGSGSGTDFTLK
--FR3-------->     CDR3     <---FR4-->
ISRVEAEDLGVYFCSQNTHVPLTFGAGTKLELK

### Nucleic acid sequence

GATGTTTTGATGACCCAAACTCCACTCTCCCTGCCTGTCAGTCTTGGAGATCAA
GCCTCCATCTCTTGCAGATCTAGTCAGAGCCTTGTACACAGTAATGGAAACACC
TATTTACATTGGTACCTGCAGAAGCCAGGCCAGTCTCCAAAGCTCCTGATCTAC
AAAGTTTCCAACCGATTTTCTGGGGTCCCAGACAGGTTCAGTGGCAGTGGATC
AGGGACAGATTTCACACTCAAGATCAGCAGAGTGGAGGCTGAGGATCTGGGA
GTTTATTTCTGCTCTCAAAATACACATGTTCCGCTCACGTTCGGTGCTGGGACC
AAGCTGGAGCTGAAA

### 41H1

The amino acid sequence of the heavy chain VH is shown in SEQ ID NO. 9; its encoding nucleic acid is shown in SEQ ID NO. 10; and its HCDR1, HCDR2, HCDR3 are shown in SEQ ID NO. 11, 12, 13, respectively.

<------------FR1------------> CDR1 <-----FR2----> CDR2 <------------
QVQLQQSGAELVRPGASVKLSCKALGYTFADYEIHWVKQTPVHGLEWIGAIHPG
SGGTAYNQTFEGKATLTADKSSTTA
-------FR3--------> CDR3 <---FR4--->
YMELSSLTSEDSAVYYCTRYYSFAYWGQGTLVTVSA

### Nucleic acid sequence

CAGGTCCAGCTGCAGCAGTCTGGGGCTGAGCTGGTGAGGCCTGGGGCTTCAGT
GAAGCTGTCCTGCAAGGCTTTGGGCTACACATTTGCTGACTATGAAATACACTG
GGTGAAGCAGACACCTGTGCATGGCCTGGAGTGGATTGGAGCTATTCATCCAG
GAAGTGGTGGTACTGCCTACAATCAGACGTTCGAGGGCAAGGCCACACTGACT
GCAGACAAATCCTCCACCACAGCCTACATGGAGCTCAGCAGCCTGACATCTGA
GGACTCTGCTGTCTATTACTGTACAAGATACTATTCCTTTGCTTACTGGGGCCAA
GGGACTCTGGTCACTGTCTCTGCA

The amino acid sequence of light chain VK is shown in SEQ ID NO. 55; its encoding nucleic acid is shown in SEQ ID NO. 56; and its LCDR1, LCDR2, LCDR3 are shown in SEQ ID NO. 16, 17, 57, respectively.

<---------FR1---------> CDR1 <-----FR2-----> CDR2 <-----------------
DVVMTQTPLSLPVSLGDQASISCRSSQSLVHSNGNTYLHWYLQKPGQSPKLLIYK
VSNRFSGVPDRFSGSGSGTDFTLR
--FR3--------> CDR3 <---FR4-->
ISRVEAEDLGVYFCSQSTRVPLTFGAGTKLELK

### Nucleic acid sequence

GATGTTGTGATGACCCAGACTCCACTCTCCCTGCCTGTCAGTCTTGGAGATCAA
GCCTCCATCTCTTGCAGATCTAGTCAGAGCCTTGTACACAGTAATGGAAACACC
TATTTACATTGGTACCTGCAGAAGCCAGGCCAGTCTCCAAAGCTCCTGATCTAC
AAAGTTTCCAACCGATTTTCTGGGGTCCCAGACAGGTTCAGTGGCAGTGGATC

AGGGACAGATTTCACACTCAGGATCAGCAGAGTGGAGGCTGAGGATCTGGGA
GTTTATTTCTGCTCTCAAAGTACACGTGTTCCGCTCACGTTCGGTGCTGGGACC
AAGCTGGAGCTGAAA

### 49G8

The amino acid sequence of the heavy chain VH is shown in SEQ ID NO. 9; its encoding nucleic acid is shown in SEQ ID NO. 10; and its HCDR1, HCDR2, HCDR3 are shown in SEQ ID NO. 11, 12, 13, respectively.

<-----------FR1-----------> CDR1 <-----FR2----> CDR2 <-----------

QVQLQQSGAELVRPGASVKLSCKALGYTFADYEIHWVKQTPVHGLEWIGAIHPG

SGGTAYNQTFEGKATLTADKSSTTA

-------FR3-------> CDR3 <---FR4--->

YMELSSLTSEDSAVYYCTRYYSFAYWGQGTLVTVSA

### Nucleic acid sequence

CAGGTCCAGCTGCAGCAGTCTGGGGCTGAGCTGGTGAGGCCTGGGGCTTCAGT
GAAGCTGTCCTGCAAGGCTTTGGGCTACACATTTGCTGACTATGAAATACACTG
GGTGAAGCAGACACCTGTGCATGGCCTGGAGTGGATTGGAGCTATTCATCCAG
GAAGTGGTGGTACTGCCTACAATCAGACGTTCGAGGGCAAGGCCACACTGACT
GCAGACAAATCCTCCACCACAGCCTACATGGAGCTCAGCAGCCTGACATCTGA
GGACTCTGCTGTCTATTACTGTACAAGATACTATTCCTTTGCTTACTGGGGCCAA
GGGACTCTGGTCACTGTCTCTGCA

The amino acid sequence of light chain VK is shown in SEQ ID NO. 58; its encoding nucleic acid is shown in SEQ ID NO. 59; and its LCDR1, LCDR2, LCDR3 are shown in SEQ ID NO. 60, 17, 61, respectively.

<---------FR1---------> CDR1 <-----FR2-----> CDR2 <----------------
DVVMTQTPLSLPVSLGDQASISCRSSQSIVHSNGNTYLEWYLQKPGQSPKLLIYK
VSNRFSGVPDRFSGSGSGTDFTLK
--FR3--------> CDR3 <---FR4-->
ISRVEAEDLGVYFCLQVTHVPLTFGAGTKLELK

### Nucleic acid sequence

GATGTTGTGATGACCCAAACTCCACTCTCCCTGCCTGTCAGTCTTGGAGATCAA

GCCTCCATCTCTTGCAGATCTAGTCAGAGCATTGTACATAGTAATGGAAACACCT
ATTTAGAATGGTACCTGCAGAAACCAGGCCAGTCTCCAAAGCTCCTGATCTACA
AAGTTTCCAACCGATTTTCTGGGGTCCCAGACAGGTTCAGTGGCAGTGGATCA
GGGACAGATTTCACACTCAAGATCAGCAGAGTGGAGGCTGAGGATCTGGGAGT
TTATTTCTGCCTCCAAGTTACACATGTCCCGCTCACGTTCGGTGCTGGGACCAA
GCTGGAGCTGAAA

### 46C3

The amino acid sequence of the heavy chain VH is shown in SEQ ID NO. 62; its encoding nucleic acid is shown in SEQ ID NO. 63; and its HCDR1, HCDR2, HCDR3 are shown in SEQ ID NO. 64, 65, 66, respectively.

<-----------FR1-----------> CDR1 <-----FR2----> CDR2 <-----------
QVQLQQSGPELVKPGASVKISCKASGYTFTDYYINWVKQKPGQGLEWIGWIYPG
SGNTKYNEKFKGKATLTVDTSSSTA
-------FR3-------->CDR3<--FR4-->
YMQLSSLTSEDTAVYFCARGRWGQGTLVTVSA

### Nucleic acid sequence

CAGGTCCAGTTGCAACAGTCTGGACCTGAGCTGGTGAAGCCTGGGGCTTCAGT
GAAGATATCCTGCAAGGCTTCTGGCTACACCTTCACTGACTACTATATAAACTGG
GTGAAGCAGAAGCCTGGACAGGGACTTGAGTGGATTGGATGGATTTATCCTGG
AAGCGGTAATACTAAGTACAATGAGAAGTTCAAGGGCAAGGCCACATTGACTG
TAGACACATCCTCCAGCACAGCCTACATGCAGCTCAGCAGCCTGACATCCGAG
GACACTGCTGTCTATTTCTGTGCAAGGGGGCGCTGGGGCCAAGGGACTCTGGT
CACTGTCTCTGCA

The amino acid sequence of light chain VK is shown in SEQ ID NO. 67; its encoding nucleic acid is shown in SEQ ID NO. 68; and its LCDR1, LCDR2, LCDR3 are shown in SEQ ID NO. 16, 17, 69, respectively.

<---------FR1---------> CDR1 <-----FR2-----> CDR2 <-----------------
DVVMTQTPLSLPVSLRDQASISCRSSQSLVHSNGNTYLHWYLQKPGQSPKLLIYK
VSNRFSGVPDRFSGSGSGTDFTLK
--FR3--------> CDR3 <---FR4-->

ISRVEAEDLGVYFCSQSTHVPRTFGGGTKLELK

### Nucleic acid sequence

GATGTTGTGATGACCCAGACTCCACTCTCCCTGCCTGTCAGTCTTCGAGATCAA
GCCTCCATCTCTTGCAGATCTAGTCAGAGCCTTGTACACAGTAATGGAAACACC
TATTTACATTGGTACCTGCAGAAGCCAGGCCAGTCTCCAAAGCTCCTGATCTAC
AAAGTTTCCAACCGATTTTCTGGGGTCCCAGACAGGTTCAGTGGCAGTGGATC
AGGGACAGATTTCACACTCAAGATCAGCAGAGTGGAGGCTGAGGATCTGGGA
GTTTATTTCTGCTCTCAAAGTACACATGTTCCTCGGACGTTCGGTGGAGGCACC
AAGCTGGAGCTGAAA

### 42A1

The amino acid sequence of the heavy chain VH is shown in SEQ ID NO. 70; its encoding nucleic acid is shown in SEQ ID NO. 71; and its HCDR1, HCDR2, HCDR3 are shown in SEQ ID NO. 11, 72, 73, respectively.

<-----------FR1-----------> CDR1 <-----FR2----> CDR2 <-----------

QVQLQQSGAELVRPGASVKLSCKALGYTFADYEIHWVKQTPVHGLEWIGAIHPG

SGGTAYSQTFKGKATLTADKSSTTA

-------FR3--------> CDR3 <---FR4--->

YMELSSLTSEDSAVYYCTRYFSFAYWGQGTLVTVSA

### Nucleic acid sequence

CAGGTTCAGCTGCAGCAGTCTGGGGCTGAGCTGGTGAGGCCTGGGGCTTCAGT
GAAGCTGTCCTGCAAGGCTTTGGGCTACACATTTGCTGACTATGAAATACACTG
GGTGAAGCAGACACCTGTGCATGGCCTGGAATGGATTGGAGCTATTCATCCAG
GAAGTGGTGGTACTGCCTACAGTCAGACGTTCAAGGGCAAGGCCACACTGACT
GCAGACAAATCCTCCACCACAGCCTACATGGAGCTCAGCAGCCTGACATCTGA
GGACTCTGCTGTCTATTACTGTACAAGATACTTTTCCTTTGCTTACTGGGGCCAA
GGGACTCTGGTCACTGTCTCTGCA

The amino acid sequence of light chain VK is shown in SEQ ID NO. 74; its encoding nucleic acid is shown in SEQ ID NO. 75; and its LCDR1, LCDR2, LCDR3 are shown in SEQ ID NO. 16, 17, 76, respectively.

<-----------FR1----------> CDR1 <-----FR2-----> CDR2 <-----------

DVVVTQTPLSLPVSLGDQASISCRSSQSLVHSNGNTYLHWYLQKPGQSPKLLIYK
VSNRFSGVPDRFSGSGSGTDFTLK
--FR3--------> CDR3 <---FR4-->
ISRVEAEDLGVYFCSQSTHAPYTFGGGTRLEIK

### Nucleic acid sequence

GATGTTGTGGTGACTCAAACTCCACTCTCCCTGCCTGTCAGTCTTGGAGATCAA
GCCTCCATCTCTTGCAGATCTAGTCAGAGCCTTGTACACAGTAATGGAAACACC
TATTTACATTGGTACCTGCAGAAGCCAGGCCAGTCTCCAAAGCTCCTGATCTAC
AAAGTTTCCAACCGATTTTCTGGGGTCCCAGACAGGTTCAGTGGCAGTGGATC
AGGGACAGATTTCACACTCAAGATCAGCAGAGTGGAGGCTGAGGATCTGGGA
GTTTATTTCTGCTCTCAAAGTACACATGCTCCGTACACGTTCGGAGGGGGGACC
AGACTGGAAATAAAA

### 47B6

The amino acid sequence of the heavy chain VH is shown in SEQ ID NO. 77; its encoding nucleic acid is shown in SEQ ID NO. 78; and its HCDR1, HCDR2, HCDR3 are shown in SEQ ID NO. 79, 80, 81, respectively.

<-----------FR1-----------> CDR1 <-----FR2----> CDR2 <-----------
QVQLQQSGAELMKPGASVKISCKATGYTFSSYWIEWVKQRPGHGLEWIGEILPG
SGSTNYNEKFKGKATFTADTSSNTA
-------FR3-------->CDR3<--FR4-->
YMQLSSLTSEDSAVYYCARGYWGQGTLVTVSA

### Nucleic acid sequence

CAGGTTCAGCTGCAACAGTCTGGAGCTGAGCTGATGAAGCCTGGGGCCTCAGT
GAAGATATCCTGCAAGGCTACTGGCTACACATTCAGTAGCTACTGGATAGAGTG
GGTAAAGCAGAGGCCTGGACATGGCCTTGAGTGGATTGGAGAGATTTTACCTG
GAAGTGGTAGTACTAACTACAATGAGAAGTTCAAGGGCAAGGCCACATTCACT
GCAGATACATCCTCCAACACAGCCTACATGCAACTCAGCAGCCTGACATCTGAG
GACTCTGCCGTCTATTACTGTGCGAGGGGTTACTGGGGCCAAGGGACTCTGGTC
ACTGTCTCTGCA

The amino acid sequence of light chain VK is shown in SEQ ID NO. 82; its encoding nucleic acid is shown in SEQ ID NO. 83; and its LCDR1, LCDR2, LCDR3 are shown in SEQ ID NO. 16, 17, 69, respectively.

<-----------FR1----------->          CDR1          <-----FR2----->    CDR2    <-----------------
DIVMTQAPLSLPVSLGDQASISCRSSQSLVHSNGNTYLHWYLQKPGQSPKLLIYK
VSNRFSGVPDRFSGSGSGTDFTLK
--FR3--------->      CDR3      <---FR4-->
ISRVEAEDLGVYFCSQSTHVPRTFGGGTKLEIK

### Nucleic acid sequence

GATATTGTGATGACGCAGGCTCCACTCTCCCTGCCTGTCAGTCTTGGAGATCAA
GCCTCCATCTCTTGCAGATCTAGTCAGAGCCTTGTACACAGTAATGGAAACACC
TATTTACATTGGTACCTGCAGAAGCCAGGCCAGTCTCCAAAGCTCCTGATCTAC
AAAGTTTCCAACCGATTTTCTGGGGTCCCAGACAGGTTCAGTGGCAGTGGATC
AGGGACAGATTTCACACTCAAGATCAGCAGAGTGGAGGCTGAGGATCTGGGA
GTTTATTTCTGCTCTCAAAGTACACATGTTCCTCGGACGTTCGGTGGAGGCACC
AAGCTGGAAATCAAA

The binding of the antibodies to human GPC3 stably-transfected cells was detected by FACS. CHO-human GPC3 stably-transfected cells prepared in Example 1 in the logarithmic growth phase were adjusted to 5 × 10⁵ cells/ml with 4% calf serum (Hyclone, SH30626.06). 100 µl/well of cell suspension was added to a 96-well U-plate, and centrifuged at 300 g for 5 min. The supernatant was discarded. 100 µL of gradient diluted antibodies (initial concentration 200nM, 3-fold dilution, 12 gradients) was added to each well and incubated at 4°C for 45 min. The secondary antibody Alexa Fluro647 labeled goat anti-human IgG Fc (1: 300 dilution) was added at 50 µL/well, incubated on ice for 45 minutes, and detected by flow cytometry. FIG. 4 shows the flow cytometry detection results of chimeric antibodies and human GPC3-CHO stably-transfected cells.

Using BLI technology (Octet), the antibody to be tested was captured on the Protein A probe. The binding capacity with human GPC3 protein (10 µg/ml as the initial concentration, 2-fold gradient, a total of 5 concentration points) was detected. The affinity constant KD was obtained according to the association and dissociation rates.

Based on the results of FACS and Octet, a total of 15 positive monoclonal antibodies were obtained, which could recognize the GPC3 receptor on the cell membrane with high affinity and bind to recombinant GPC3 protein. Table 2 shows the affinity constants of the monoclonal antibodies to human GPC3.

**Table 2. Binding activity of monoclonal antibodies to human GPC3**

| Antibody | FACS | Octet | | |
|---|---|---|---|---|
| | EC₅₀(nM) | KD (nM) | kon(1/Ms) | kdis(1/s) |
| 5D6 | 3.73 | 27.7 | 1.05E+05 | 2.92E-03 |
| 29A10 | 3.39 | 19.8 | 1.42E+05 | 2.81E-03 |
| 34H1 | 2.1 | 12.9 | 1.71E+05 | 2.21E-03 |
| 19F1 | 3.59 | 18.3 | 1.16E+05 | 2.13E-03 |
| 30B7 | 2.78 | 10.9 | 7.17E+04 | 7.83E-04 |
| 28G4 | 2.87 | 11.3 | 9.89E+04 | 1.12E-03 |
| 30A4 | 3.68 | 19.9 | 1.30E+05 | 2.58E-03 |
| 38D2 | 3.35 | 11.3 | 7.34E+04 | 8.32E-04 |
| 39G4 | 2.79 | 26.2 | 7.95E+04 | 2.08E-03 |
| 41F1 | 0.91 | 18.5 | 9.79E+04 | 1.81E-03 |
| 41H1 | 1.07 | 25 | 1.06E+05 | 2.64E-03 |
| 49G8 | 1.18 | 3.32 | 6.94E+04 | 2.31E-04 |
| 46C3 | 0.46 | 24.3 | 6.36E+04 | 1.54E-03 |
| 42A1 | 6.69 | 40.5 | 6.00E+04 | 2.43E-03 |
| 47B6 | 0.38 | 48.2 | 3.08E+04 | 1.49E-03 |
| GC33 | 2.74 | 4.17 | 5.49E+04 | 2.29E-04 |

### 2. Humanization of GPC3 candidate antibodies

### 2.1 Species cross-reactivity of candidate antibodies

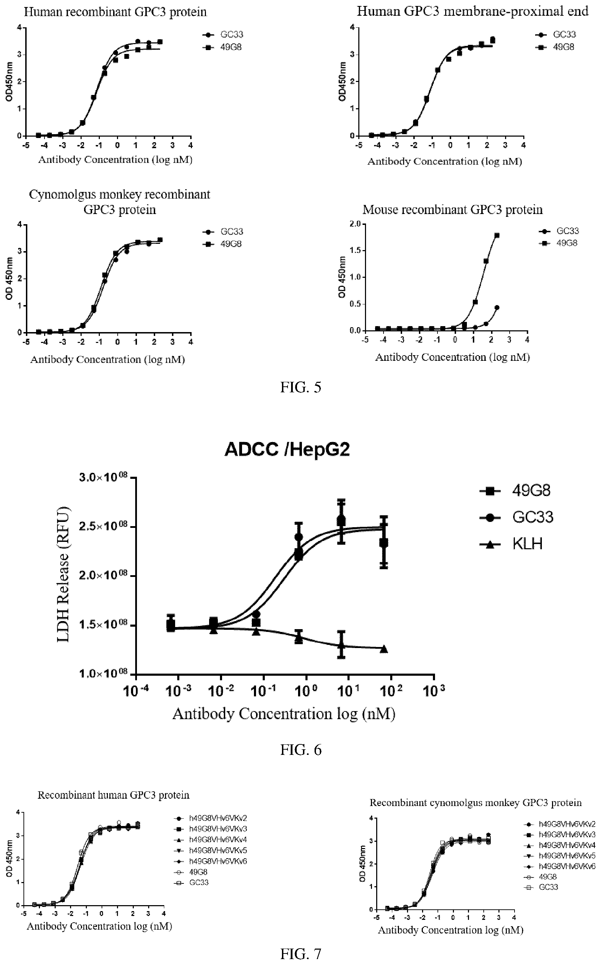

The high-affinity positive clone 49G8 was selected to detect binding activity to recombinant human, cynomolgus monkey, mouse GPC3 full-length proteins, and recombinant human GPC3 extracellular domain membrane-proximal proteins. 1 µg/ml of the above-mentioned protein was coated overnight at 4°C. After blocking, 49G8 chimeric antibody (initial concentration 200 nM, 3-fold gradient dilution, a total of 12 concentrations) was added and reacted for 1 hour at room temperature. The secondary antibody HPR-labeled goat anti-human IgG Fc was added. After being developed by the TMB solution, the reaction was stopped with concentrated sulfuric acid, and the absorbance was read at 450 nm. The ELISA results are shown in FIG. 5. The 49G8 antibody can bind to the full-length human and cynomolgus monkey GPC3 proteins and the membrane-proximal end of the extracellular region of human GPC3 with a slightly higher affinity than the control antibody and a weak affinity to mouse GPC3 protein (Table 3).

**Table 3 ELISA results of 49G8 and different species of GPC3 full-length protein and human GPC3 membrane-proximal protein**

| | **Human GPC3** | **Cynomolgus Monkey GPC3** | **Mouse GPC3** | **GST-Human GPC3-stem** |
|---|---|---|---|---|
| **49G8** | 0.072nM | 0.120nM | Slightly Weak | 0.074nM |
| **GC33** | 0.074nM | 0.160nM | Slightly Weak | 0.060nM |

### 2.2 Antibody-dependent cell-mediated cytotoxicity (ADCC)

Human hepatoma cells expressing GPC3 (HepG2) were used as target cells. The density of target cells was adjusted to 5 × 10⁴ cells/well, and effector cells NK92MI-CD16a were adjusted to 1 × 10⁵ cells/well. Gradient-diluted antibody (66.7nM as the initial concentration, 10-fold gradient, 6 dilutions in total) was added and incubated at a 37°C, 5% CO₂ incubator for 4 hours. The supernatant was taken to detect the release of LDH. The results are shown in FIG. 6. The 49G8 chimeric antibody can mediate an ADCC effect on the target cell HepG2.

### 2.3 Humanization of 49G8 Antibody

The murine antibody 49G8 variable region sequence was humanized by CDR grafting and point mutation. Mab 49G8 murine antibody light chain is mouse IMGT_mVK1-110. Human IMGT_hVK2-28 with the highest homology with its framework region was selected for CDR transplantation, and human IGKJ4*01 with the highest homology was selected for FM4; The heavy chain of murine antibody was IGHV1-15. Human germline gene IMGT_hVH1-69 was selected for CDR transplantation, and human IGHJ4*01 with the highest homology was selected for FM4. Reverse mutations were designed to synthesize a total of 6 heavy chain variable region and 6 light chain variable region humanized variants. After combination and pairing, they were transiently expressed in HEK 293E cells. After 5-6 days of culture at 37°C, 120 rpm, and 5% CO₂, the supernatant of the culture medium was collected and purified by the Protein A chromatographic column to obtain the humanized anti-GPC3 antibody.

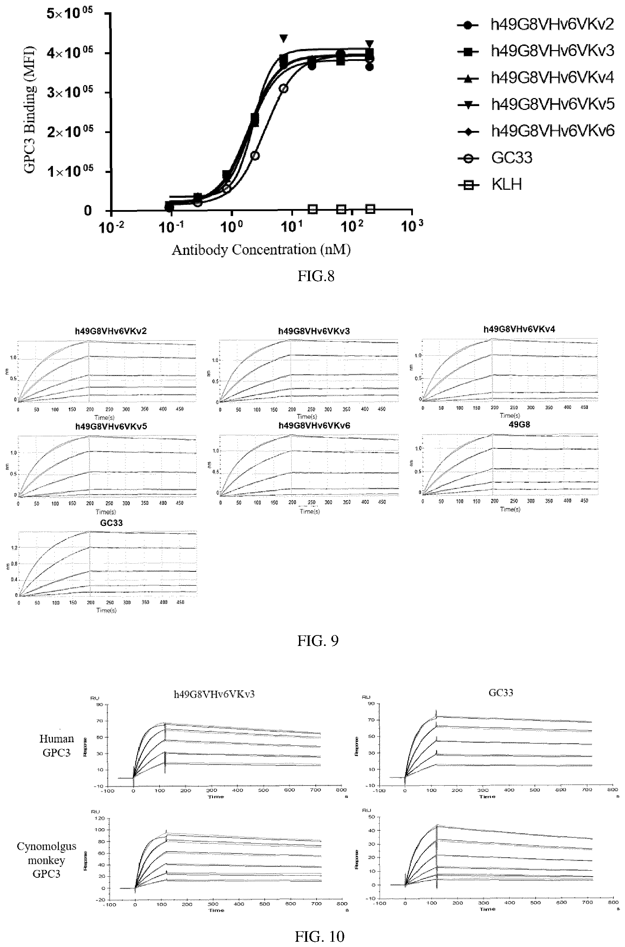

The binding activity of 49G8 humanized antibody to human and Cynomolgus monkey GPC3 full-length protein was determined by the ELISA method. The binding of the 49G8 humanized antibody to human GPC3-CHO stably-transfected cells was detected by flow cytometry. The ELISA results are shown in FIG. 7. The 49G8 humanized antibody can bind to both recombinant human and cynomolgus monkey GCP3 proteins with high affinity. The FACS results are shown in FIG. 8. the affinity of the 49G8 humanized antibody for human GPC3-CHO stably-transfected cells was comparable to that of the pre-humanized mouse antibody and slightly higher than that of the control antibody. The humanized antibody heavy/light chain variable region sequences are as follows:

**Table 4. Light chain variable region sequences of humanized GPC3 antibody**

| GPC3 humanized antibody | LCVR | | LCDR1 | LCDR2 | LCDR3 |
|---|---|---|---|---|---|
| | Amino Acid Sequence | Nucleotide Sequence | Amino Acid Sequence | Amino Acid Sequence | Amino Acid Sequence |
| h49G8VKv1 | 84 | 85 | 60 | 17 | 61 |
| h49G8VKv2 | 86 | 87 | 60 | 17 | 61 |
| h49G8VKv3 | 88 | 89 | 60 | 17 | 61 |
| h49G8VKv4 | 90 | 91 | 92 | 17 | 61 |
| h49G8VKv5 | 93 | 94 | 95 | 17 | 61 |
| h49G8VKv6 | 96 | 97 | 98 | 17 | 61 |

### h49G8VKv1

The amino acid sequence of light chain VK is shown in SEQ ID NO. 84; its encoding nucleic acid is shown in SEQ ID NO. 85; and its LCDR1, LCDR2, LCDR3 are shown in SEQ ID NO. 60, 17, 61, respectively.

DVVMTQSPLSLPVTLGQPASISCRSSQSIVHSNYNTYLEWYQQRPGQSPRLLIYKV
SNRFSGVPDRFSGSGSGTDFTLKISRVEAEDVGVYFCLQVTHVPLTFGQGTKLEIK

### Nucleic acid sequence

GACGTGGTCATGACACAGAGCCCTCTGAGCCTGCCTGTGACACTGGGACAGCC
TGCCAGCATCAGCTGTAGAAGCAGCCAGAGCATCGTGCACAGCAACTACAACA
CATACCTGGAATGGTATCAGCAGAGGCCTGGCCAGTCTCCTAGACTGCTGATCT
ACAAGGTGTCCAACAGATTCAGCGGCGTGCCCGACAGATTCTCTGGCTCTGGA
TCTGGCACCGACTTCACCCTGAAGATCTCCAGAGTGGAAGCCGAGGACGTGGG
CGTGTACTTCTGTCTCCAGGTCACACACGTGCCCCTGACATTTGGCCAGGGCAC
CAAGCTGGAAATCAAG

### h49G8VKv2

The amino acid sequence of light chain VK is shown in SEQ ID NO. 86; its encoding nucleic acid is shown in SEQ ID NO. 87; and its LCDR1, LCDR2, LCDR3 are shown in SEQ ID NO. 60, 17, 61, respectively.

DVVMTQSPLSLPVTPGEPASISCRSSQSIVHSNYNTYLEWYLQKPGQSPQLLIYKV
SNRFSGVPDRFSGSGSGTDFTLKISRVEAEDVGVYFCLQVTHVPLTFGQGTKLEIK

### Nucleic acid sequence

GACGTGGTCATGACACAGAGCCCTCTGAGCCTGCCTGTGACACCTGGCGAACC
TGCCAGCATCAGCTGTAGAAGCAGCCAGAGCATCGTGCACAGCAACTACAACA
CATACCTGGAGTGGTATCTGCAGAAGCCCGGCCAGTCTCCTCAGCTGCTGATCT
ACAAGGTGTCCAACAGATTCAGCGGCGTGCCCGACAGATTCTCTGGCTCTGGA
TCTGGCACCGACTTCACCCTGAAGATCTCCAGAGTGGAAGCCGAGGACGTGGG
CGTGTACTTCTGTCTCCAGGTCACACACGTGCCCCTGACATTTGGCCAGGGCAC
CAAGCTGGAAATCAAG

### h49G8VKv3

The amino acid sequence of light chain VK is shown in SEQ ID NO. 88; its encoding nucleic acid is shown in SEQ ID NO. 89; and its LCDR1, LCDR2, LCDR3 are shown in SEQ ID NO. 60, 17, 61, respectively.

DVVMTQSPLSLPVTPGEPASISCRSSQSIVHSNGNTYLEWYLQKPGQSPQLLIYKV
SNRFSGVPDRFSGSGSGTDFTLKISRVEAEDVGVYFCLQVTHVPLTFGQGTKLEIK

### Nucleic acid sequence

GACGTGGTCATGACACAGAGCCCTCTGAGCCTGCCTGTGACACCTGGCGAACC
TGCCAGCATCAGCTGTAGAAGCAGCCAGAGCATCGTGCACAGCAACGGCAAC
ACATACCTGGAGTGGTATCTGCAGAAGCCCGGCCAGTCTCCTCAGCTGCTGATC
TACAAGGTGTCCAACAGATTCAGCGGCGTGCCCGACAGATTCTCTGGCTCTGG
ATCTGGCACCGACTTCACCCTGAAGATCTCCAGAGTGGAAGCCGAGGACGTGG
GCGTGTACTTCTGTCTCCAGGTCACACACGTGCCCCTGACATTTGGCCAGGGCA
CCAAGCTGGAAATCAAG

### h49G8VKv4

The amino acid sequence of light chain VK is shown in SEQ ID NO. 90; its encoding nucleic acid is shown in SEQ ID NO. 91; and its LCDR1, LCDR2, LCDR3 are shown in SEQ ID NO. 92, 17, 61, respectively.

DVVMTQSPLSLPVTPGEPASISCRSSQSIVHSNSNTYLEWYLQKPGQSPQLLIYKV
SNRFSGVPDRFSGSGSGTDFTLKISRVEAEDVGVYFCLQVTHVPLTFGQGTKLEIK

### Nucleic acid sequence

GACGTGGTCATGACACAGAGCCCTCTGAGCCTGCCTGTGACACCTGGCGAACC
TGCCAGCATCAGCTGTAGAAGCAGCCAGAGCATCGTGCACAGCAACAGCAAC
ACATACCTGGAGTGGTATCTGCAGAAGCCCGGCCAGTCTCCTCAGCTGCTGATC
TACAAGGTGTCCAACAGATTCAGCGGCGTGCCCGACAGATTCTCTGGCTCTGG
ATCTGGCACCGACTTCACCCTGAAGATCTCCAGAGTGGAAGCCGAGGACGTGG
GCGTGTACTTCTGTCTCCAGGTCACACACGTGCCCCTGACATTTGGCCAGGGCA
CCAAGCTGGAAATCAAG

### h49G8VKv5

The amino acid sequence of light chain VK is shown in SEQ ID NO. 93; its encoding nucleic acid is shown in SEQ ID NO. 94; and its LCDR1, LCDR2, LCDR3 are shown in SEQ ID NO. 95, 17, 61, respectively.

DVVMTQSPLSLPVTPGEPASISCRSSQSIVHSNTNTYLEWYLQKPGQSPQLLIYKV
SNRFSGVPDRFSGSGSGTDFTLKISRVEAEDVGVYFCLQVTHVPLTFGQGTKLEIK

### Nucleic acid sequence

GACGTGGTCATGACACAGAGCCCTCTGAGCCTGCCTGTGACACCTGGCGAACC
TGCCAGCATCAGCTGTAGAAGCAGCCAGAGCATCGTGCACAGCAACACAAAC
ACATACCTGGAGTGGTATCTGCAGAAGCCCGGCCAGTCTCCTCAGCTGCTGATC
TACAAGGTGTCCAACAGATTCAGCGGCGTGCCCGACAGATTCTCTGGCTCTGG
ATCTGGCACCGACTTCACCCTGAAGATCTCCAGAGTGGAAGCCGAGGACGTGG
GCGTGTACTTCTGTCTCCAGGTCACACACGTGCCCCTGACATTTGGCCAGGGCA
CCAAGCTGGAAATCAAG

### h49G8VKv6

The amino acid sequence of light chain VK is shown in SEQ ID NO. 96; its encoding nucleic acid is shown in SEQ ID NO. 97; and its LCDR1, LCDR2, LCDR3 are shown in SEQ ID NO. 98, 17, 61, respectively.

DVVMTQSPLSLPVTPGEPASISCRSSQSIVHSNANTYLEWYLQKPGQSPQLLIYKV
SNRFSGVPDRFSGSGSGTDFTLKISRVEAEDVGVYFCLQVTHVPLTFGQGTKLEIK

### Nucleic acid sequence

GACGTGGTCATGACACAGAGCCCTCTGAGCCTGCCTGTGACACCTGGCGAACC
TGCCAGCATCAGCTGTAGAAGCAGCCAGAGCATCGTGCACAGCAACGCCAACA
CATACCTGGAGTGGTATCTGCAGAAGCCCGGCCAGTCTCCTCAGCTGCTGATCT
ACAAGGTGTCCAACAGATTCAGCGGCGTGCCCGACAGATTCTCTGGCTCTGGA
TCTGGCACCGACTTCACCCTGAAGATCTCCAGAGTGGAAGCCGAGGACGTGGG
CGTGTACTTCTGTCTCCAGGTCACACACGTGCCCCTGACATTTGGCCAGGGCAC
CAAGCTGGAAATCAAG

**Table 5. Heavy chain variable region sequences of humanized GPC3 antibodies**

| GPC3 humanized | HCVR | | HCDR1 | HCDR2 | HCDR3 |
|---|---|---|---|---|---|
| | Amino Acid | Nucleotide | Amino Acid | Amino Acid | Amino Acid |
| antibody | Sequence | Sequence | Sequence | Sequence | Sequence |
| h49G8 VHv1 | 99 | 100 | 11 | 101 | 13 |
| h49G8VHv2 | 102 | 103 | 11 | 101 | 13 |
| h49G8VHv3 | 104 | 105 | 11 | 106 | 13 |
| h49G8VHv4 | 107 | 108 | 11 | 101 | 13 |
| h49G8VHv5 | 109 | 110 | 11 | 101 | 13 |
| h49G8VHv6 | 111 | 112 | 11 | 106 | 13 |

### h49G8VHv1

The amino acid sequence of the heavy chain VH is shown in SEQ ID NO. 99; its encoding nucleic acid is shown in SEQ ID NO. 100; and its HCDR1, HCDR2, HCDR3 are shown in SEQ ID NO. 11, 101, 13, respectively.

QVQLVQSGAEVKKPGSSVKVSCKASGYTFADYEIHWVRQAPGQGLEWMGAIHP
GSGGTAYNQKFQGRVTLTADKSSTTAYMELSSLRSEDTAVYYCTRYYSFAYWGQ
GTLVTVSS

### Nucleic acid sequence

CAGGTTCAGCTGGTTCAGTCTGGCGCCGAAGTGAAGAAACCTGGCAGCAGCG
TGAAGGTGTCCTGCAAGGCTAGCGGCTACACCTTCGCCGACTACGAGATCCAC
TGGGTCCGACAGGCTCCAGGACAGGGACTTGAATGGATGGGCGCTATCCATCC
TGGCTCTGGCGGCACAGCTTACAACCAGAAATTCCAGGGCAGAGTGACCCTGA
CCGCCGACAAGTCTAGCACCACCGCCTACATGGAACTGAGCAGCCTGAGAAGC
GAGGACACCGCCGTGTACTACTGCACCCGGTACTACAGCTTCGCCTACTGGGG
ACAGGGAACCCTGGTCACAGTCAGCTCT

### h49G8VHv2

The amino acid sequence of the heavy chain VH is shown in SEQ ID NO. 102; its encoding nucleic acid is shown in SEQ ID NO. 103; and its HCDR1, HCDR2, HCDR3 are shown in SEQ ID NO. 11, 101, 13, respectively.

QVQLVQSGAELKKPGSSVKVSCKASGYTFADYEIHWVRQAPGQGLEWMGAIHP
GSGGTAYNQKFQGRVTLTADKSSTTAYMELSSLRSEDTAVYYCTRYYSFAYWGQ

GTLVTVSS

### Nucleic acid sequence

CAGGTTCAGCTGGTTCAGTCTGGCGCCGAACTGAAGAAACCTGGCAGCAGCG
TGAAGGTGTCCTGCAAGGCTAGCGGCTACACCTTCGCCGACTACGAGATCCAC
TGGGTCCGACAGGCTCCAGGACAGGGACTTGAATGGATGGGCGCTATCCATCC
TGGCTCTGGCGGCACAGCTTACAACCAGAAATTCCAGGGCAGAGTGACCCTGA
CCGCCGACAAGTCTAGCACCACCGCCTACATGGAACTGAGCAGCCTGAGAAGC
GAGGACACCGCCGTGTACTACTGCACCCGGTACTACAGCTTCGCCTACTGGGG
ACAGGGAACCCTGGTCACAGTCAGCTCT

### h49G8VHv3

The amino acid sequence of the heavy chain VH is shown in SEQ ID NO. 104; its encoding nucleic acid is shown in SEQ ID NO. 105; and its HCDR1, HCDR2, HCDR3 are shown in SEQ ID NO. 11, 106, 13, respectively.

QVQLVQSGAEVKKPGSSVKVSCKASGYTFADYEIHWVRQAPGQGLEWMGAIHP
GSGGTAYAQKFQGRVTLTADKSSTTAYMELSSLRSEDTAVYYCTRYYSFAYWGQ
GTLVTVSS

### Nucleic acid sequence

CAGGTTCAGCTGGTTCAGTCTGGCGCCGAAGTGAAGAAACCTGGCAGCAGCG
TGAAGGTGTCCTGCAAGGCTAGCGGCTACACCTTCGCCGACTACGAGATCCAC
TGGGTCCGACAGGCTCCAGGACAGGGACTTGAATGGATGGGCGCTATCCATCC
TGGCTCTGGCGGCACAGCTTACGCCCAGAAATTCCAGGGCAGAGTGACCCTGA
CCGCCGACAAGTCTAGCACCACCGCCTACATGGAACTGAGCAGCCTGAGAAGC
GAGGACACCGCCGTGTACTACTGCACCCGGTACTACAGCTTCGCCTACTGGGG
ACAGGGAACCCTGGTCACAGTCAGCTCT

### h49G8VHv4

The amino acid sequence of the heavy chain VH is shown in SEQ ID NO. 107; its encoding nucleic acid is shown in SEQ ID NO. 108; and its HCDR1, HCDR2, HCDR3 are shown in SEQ ID NO. 11, 101, 13, respectively.

QVQLVQSGAEVKKPGSSVKVSCKASGYTFADYEIHWVRQAPGQGLEWMGAIHP
GSGGTAYNQKFQGRVTLTADESSTTAYMELSSLRSEDTAVYYCTRYYSFAYWGQ

GTLVTVSS

### Nucleic acid sequence

CAGGTTCAGCTGGTTCAGTCTGGCGCCGAAGTGAAGAAACCTGGCAGCAGCG
TGAAGGTGTCCTGCAAGGCTAGCGGCTACACCTTCGCCGACTACGAGATCCAC
TGGGTCCGACAGGCTCCAGGACAGGGACTTGAATGGATGGGCGCTATCCATCC
TGGCTCTGGCGGCACAGCTTACAACCAGAAATTCCAGGGCAGAGTGACCCTGA
CCGCCGACGAGTCTAGCACCACCGCCTACATGGAACTGAGCAGCCTGAGAAGC
GAGGACACCGCCGTGTACTACTGCACCCGGTACTACAGCTTCGCCTACTGGGG
ACAGGGAACCCTGGTCACAGTCAGCTCT

### h49G8VHv5

The amino acid sequence of the heavy chain VH is shown in SEQ ID NO. 109; its encoding nucleic acid is shown in SEQ ID NO. 110; and its HCDR1, HCDR2, HCDR3 are shown in SEQ ID NO. 11, 101, 13, respectively.

QVQLVQSGAELKKPGSSVKVSCKASGYTFADYEIHWVRQAPGQGLEWMGAIHP
GSGGTAYNQKFQGRVTLTADESSTTAYMELSSLRSEDTAVYYCTRYYSFAYWGQ
GTLVTVSS

### Nucleic acid sequence

CAGGTTCAGCTGGTTCAGTCTGGCGCCGAACTGAAGAAACCTGGCAGCAGCG
TGAAGGTGTCCTGCAAGGCTAGCGGCTACACCTTCGCCGACTACGAGATCCAC
TGGGTCCGACAGGCTCCAGGACAGGGACTTGAATGGATGGGCGCTATCCATCC
TGGCTCTGGCGGCACAGCTTACAACCAGAAATTCCAGGGCAGAGTGACCCTGA
CCGCCGACGAGTCTAGCACCACCGCCTACATGGAACTGAGCAGCCTGAGAAGC
GAGGACACCGCCGTGTACTACTGCACCCGGTACTACAGCTTCGCCTACTGGGG
ACAGGGAACCCTGGTCACAGTCAGCTCT

### h49G8VHv6

The amino acid sequence of the heavy chain VH is shown in SEQ ID NO. 111; its encoding nucleic acid is shown in SEQ ID NO. 112; and its HCDR1, HCDR2, HCDR3 are shown in SEQ ID NO. 11, 106, 13, respectively.

QVQLVQSGAEVKKPGSSVKVSCKASGYTFADYEIHWVRQAPGQGLEWMGAIHP
GSGGTAYAQKFQGRVTLTADESSTTAYMELSSLRSEDTAVYYCTRYYSFAYWGQ

GTLVTVSS

### Nucleic acid sequence

CAGGTTCAGCTGGTTCAGTCTGGCGCCGAAGTGAAGAAACCTGGCAGCAGCG
TGAAGGTGTCCTGCAAGGCTAGCGGCTACACCTTCGCCGACTACGAGATCCAC
TGGGTCCGACAGGCTCCAGGACAGGGACTTGAATGGATGGGCGCTATCCATCC
TGGCTCTGGCGGCACAGCTTACGCCCAGAAATTCCAGGGCAGAGTGACCCTGA
CCGCCGACGAGTCTAGCACCACCGCCTACATGGAACTGAGCAGCCTGAGAAGC
GAGGACACCGCCGTGTACTACTGCACCCGGTACTACAGCTTCGCCTACTGGGG
ACAGGGAACCCTGGTCACAGTCAGCTCT

### 2.3 Affinity of humanized antibody

### 1) Biolayer Interferometry (BLI)

Using BLI technology (Octet), the antibody to be tested was captured on the Protein A probe. The binding capacity with human GPC3 protein (10 µg/ml as the initial concentration, 2-fold gradient, a total of 5 concentration points) was detected. The affinity constant KD was obtained according to the association and dissociation rates. The results are shown in FIG. 9. The 49G8 humanized antibody binds to the recombinant GPC3 protein with high affinity. The affinity data is seen in Table 6.

**Table 6. Affinity results of 49G8 humanized antibody with human GPC3 full-length protein by BLI analysis**

| | KD (nM) | kon(1/Ms) | kd (1/s) |
|---|---|---|---|
| h49G8VHv6VKv2 | 2.00 | 9.36E+04 | 1.87E-04 |
| h49G8VHv6VKv3 | 1.42 | 9.48E+04 | 1.34E-04 |
| h49G8VHv6VKv4 | 2.28 | 8.99E+04 | 2.05E-04 |
| h49G8VHv6VKv5 | 2.15 | 9.00E+04 | 1.93E-04 |
| h49G8VHv6VKv6 | 2.38 | 9.24E+04 | 2.20E-04 |
| 49G8 | 1.17 | 7.97E+04 | 9.35E-05 |
| GC33 | 1.35 | 8.26E+04 | 1.11E-04 |

### 2) Surface Plasmon Resonance (SPR)

Humanized antibodies with higher affinity were selected and compared for binding to human and cynomolgus monkey GPC3. The humanized antibody h49G8VHv6VKv3 was captured by the Protein A chip (GE healthcare). The recombinant proteins of human and cynomolgus monkey GPC3 diluted in gradient (5 µg/ml as the initial concentration, 2-fold gradient, 5 concentration points in total) were flowed through the chip at a flow rate of 30 µL/min, with a binding time of 150 seconds and dissociation time of 600 seconds. The kinetic constant was fitted using Biacore T200 evaluation software with a Langmuir 1: 1 kinetics model. The result is shown in FIG. 10. The humanized antibody h49G8VHv6VKv3 binds to human and cynomolgus monkey GPC3 with high affinity. The affinity for binding to human GPC3 is 1.39 nM, and the affinity for binding to cynomolgus monkey GPC3 is 2.10 nM, which is comparable. The affinity data is seen in Table 7.

**Table 7. Affinity results of candidate antibody that binds to human and cynomolgus monkey GPC3 full-length protein by SPR analysis**

| | Human GPC3 | | | Cynomolgus Monkey GPC3 | | |
|---|---|---|---|---|---|---|
| | ka (1/Ms) | kd (1/s) | KD (nM) | ka (1/Ms) | kd (1/s) | KD (nM) |
| GC33 | 1.81E+05 | 1.85E-04 | 1.03 | 6.72E+04 | 4.29E-04 | 6.38 |
| h49G8VHv6VKv3 | 2.53E+05 | 3.51E-04 | 1.39 | 1.19E+05 | 2.50E-04 | 2.10 |

### 2.4 Endocytosis of humanized antibody

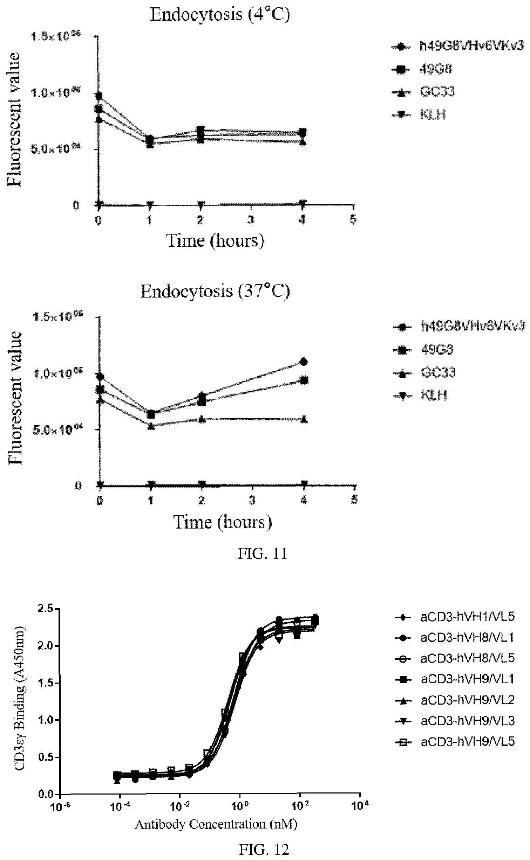

HepG2 cells in the logarithmic growth phase were taken and washed once with a pre-warmed culture medium. The cells were subjected to centrifugation at 1000 rpm for 3 min. The supernatant was discarded. A serum-free culture medium was added to resuspend the cells to 1 × 10⁶ cells/mL. The cells were pipetted into an EP tube at 200 µL/tube and added with 500 µL of pre-cooled 0.5% BSA/PBS. The centrifugation was performed at 1000 rpm for 3 min. The supernatant was discarded. 10 µg/mL GPC3 test antibody was added and incubated at 4°C or 37°C for 60 min, 120 min, 240 min, 120 min, 180 min, and 240 min, respectively. After the incubation of the primary antibody at each time point ends, 500 µL of pre-cooled 0.5% BSA/PBS was added for washing 2 times. Then, 1: 300 diluted secondary antibody AF647-labeled goat anti-human IgG Fc was directly added for incubation for 45 min. At the end of the secondary antibody incubation, 500 µL of 0.5% BSA/PBS was added for washing 2 times. The cells were resuspended to 200 µL for FACS detection. As shown in FIG. 11, the GPC3 humanized antibody showed no significant endocytosis at 37°C and 4°C.

### Example 3. Construction of GPC3 x CD3 bispecific antibody

### 1. Construction of CD3 humanized antibody

Murine hybridoma CD3 antibody (EMBO J.1985.4(2): 337-344; J.Immuno1.1986,137(4): 1097-100; J.Exp.Med.1991,174: 319-326; J.Immunol.1991,147(9): 3047-52) recognizes the human and monkey CD3 receptor, the sequence of which is as follows:
The anti-CD3 murine monoclonal antibody light chain amino acid sequence is shown in SEQ ID NO. 174: 
QAVVTQESALTTSPGETVTLTCRSSTGAVTTSNYANWVQQKPDHLFTGLIGGTNK
RAPGVPARFSGSLIGDKAALTITGAQTEDEAIYFCALWYSNLWVFGGGTKLTVL
The anti-CD3 murine monoclonal antibody heavy chain amino acid sequence is shown in SEQ ID NO. 175:

EVQLVESGGGLVQPKGSLKLSCAASGFTFNTYAMNWVRQAPGKGLEWVARIRSK
YNNYATYYADSVKDRFTISRDDSQSILYLQMNNLKTEDTAMYYCVRHGNFGNSY
VSWFAYWGQGTLVTVSS

The anti-CD3 mouse monoclonal antibody was humanized. Human germline gene IMGT_hVL7-43 with the highest homology was selected for light chain CDR transplantation, and human IGLJ3*02 was selected as FM4. Human IMGT_hVH3-73 was selected for heavy chain CDR transplantation, and human IGHJ4*01 was selected as FM4. Different heavy and light chain variants were designed and obtained (see Tables 8 and 9).

**Table 8. Light chain variable region sequences of CD3 humanized antibodies**

| | | | | | |
|---|---|---|---|---|---|
| | | | | | |

| CD3 humanized antibody | SEQ ID NOs: 113 -131 | | | | |
|---|---|---|---|---|---|
| | LCVR | | LCDR1 | LCDR2 | LCDR3 |
| | Amino Acid Sequence | Nucleotide Sequence | Amino Acid Sequence | Amino Acid Sequence | Amino Acid Sequence |
| hVL1 | 113 | 114 | 115 | 116 | 117 |
| hVL2 | 118 | 119 | 115 | 116 | 117 |
| hVL3 | 120 | 121 | 122 | 123 | 117 |
| hVL4 | 124 | 125 | 122 | 123 | 117 |
| hVL5 | 126 | 127 | 115 | 116 | 128 |
| hVL6 | 129 | 130 | 115 | 131 | 128 |

The amino acid sequence of hVL1 is shown in SEQ ID NO. 113; its encoding nucleic acid is shown in SEQ ID NO. 114; and its LCDR1, LCDR2, LCDR3 are shown in SEQ ID NO. 115, 116, 117, respectively.

QAVVTQEPSLTVSPGGTVTLTCRSSTGAVTTSNYANWVQQKPGQAPRGLIGGTN
KRAPWTPARFSGSLLGGKAA
LTLSGAQPEDEAEYYCALWYSNLWVFGGGTKLTVL

### Nucleic acid sequence

CAGGCTGTGGTCACACAAGAGCCTAGCCTGACAGTGTCTCCTGGCGGCACAGT
GACCCTGACCTGTAGATCTTCTACAG
GCGCCGTGACCACCAGCAACTACGCTAATTGGGTGCAGCAGAAGCCCGGCCAG
GCTCCTAGAGGACTGATCGGCGGAAC
AAACAAGAGAGCCCCTTGGACACCCGCCAGATTCTCTGGATCTCTGCTCGGCG
GAAAGGCCGCTCTGACACTTTCTGGT
GCTCAGCCTGAGGACGAGGCCGAGTACTATTGTGCCCTGTGGTACAGCAACCT
GTGGGTGTTCGGCGGAGGCACCAAAC
TGACAGTTCTG

The amino acid sequence of hVL2 is shown in SEQ ID NO. 118; its encoding nucleic acid is shown in SEQ ID NO. 119; and its LCDR1, LCDR2, LCDR3 are shown in SEQ ID NO. 115, 116, 117, respectively.

QAVVTQEPSLTVSPGGTVTLTCRSSTGAVTTSNYANWVQEKPGQAPRGLIGGTNK
RAPWTPARFSGSLLGGKAA
LTLSGAQPEDEAEYYCALWYSNLWVFGGGTKLTVL

### Nucleic acid sequence

CAGGCTGTGGTCACACAAGAGCCTAGCCTGACAGTGTCTCCTGGCGGCACAGT
GACCCTGACCTGTAGATCTTCTACAG
GCGCCGTGACCACCAGCAACTACGCTAATTGGGTGCAGGAGAAGCCCGGCCA
GGCTCCTAGAGGACTGATCGGCGGAAC
AAACAAGAGAGCCCCTTGGACACCCGCCAGATTCTCTGGATCTCTGCTCGGCG
GAAAGGCCGCTCTGACACTTTCTGGT
GCTCAGCCTGAGGACGAGGCCGAGTACTATTGTGCCCTGTGGTACAGCAACCT
GTGGGTGTTCGGCGGAGGCACCAAAC
TGACAGTTCTG

The amino acid sequence of hVL3 is shown in SEQ ID NO. 120; its encoding nucleic acid is shown in SEQ ID NO. 121; and its LCDR1, LCDR2, LCDR3 are shown in SEQ ID NO. 122, 123, 117, respectively.

EAVVTQEPSLTVSPGGTVTLTCESSDGAVTTSNYANWVQEKPGQAPRGLIGGTNK
EAPWTPARFSGSLLGGKAA
LTLSGAQPEDEAEYYCALWYSNLWVFGGGTKLTVL

### Nucleic acid sequence

GAGGCTGTGGTCACACAAGAGCCTAGCCTGACAGTGTCTCCTGGCGGCACAGT
GACCCTGACCTGTGAGTCTTCTGACG
GCGCCGTGACCACCAGCAACTACGCTAATTGGGTGCAGGAGAAGCCCGGCCA
GGCTCCTAGAGGACTGATCGGCGGAAC
AAACAAGGAGGCCCCTTGGACACCCGCCAGATTCTCTGGATCTCTGCTCGGCG
GAAAGGCCGCTCTGACACTTTCTGGT
GCTCAGCCTGAGGACGAGGCCGAGTACTATTGTGCCCTGTGGTACAGCAACCT
GTGGGTGTTCGGCGGAGGCACCAAAC
TGACAGTTCTG

The amino acid sequence of hVL4 is shown in SEQ ID NO. 124; its encoding nucleic acid is shown in SEQ ID NO. 125; and its LCDR1, LCDR2, LCDR3 are shown in SEQ ID NO. 122, 123, 117, respectively.

QAVVTQEPSLTVSPGGTVTLTCESSDGAVTTSNYANWVQEKPGQAPRGLIGGTNK

EAPWTPARFSGSLLGGKAA

LTLSGAQPEDEAEYYCALWYSNLWVFGGGTKLTVL

### Nucleic acid sequence

CAGGCTGTGGTCACACAAGAGCCTAGCCTGACAGTGTCTCCTGGCGGCACAGT
GACCCTGACCTGTGAGTCTTCTGACG
GCGCCGTGACCACCAGCAACTACGCTAATTGGGTGCAGGAGAAGCCCGGCCA
GGCTCCTAGAGGACTGATCGGCGGAAC
AAACAAGGAGGCCCCTTGGACACCCGCCAGATTCTCTGGATCTCTGCTCGGCG
GAAAGGCCGCTCTGACACTTTCTGGT
GCTCAGCCTGAGGACGAGGCCGAGTACTATTGTGCCCTGTGGTACAGCAACCT
GTGGGTGTTCGGCGGAGGCACCAAAC
TGACAGTTCTG

The amino acid sequence of hVL5 is shown in SEQ ID NO. 126; its encoding nucleic acid is shown in SEQ ID NO. 127; and its LCDR1, LCDR2, LCDR3 are shown in SEQ ID NO. 115, 116, 128, respectively.

QAVVTQEPSLTVSPGGTVTLTCRSSTGAVTTSNYANWVQEKPGQAPRGLIGGTNK
RAPWTPARFSGSLLGGKAA
LTITGAQAEDEAEYYCVLWYSNLWVFGGGTKLTVL

### Nucleic acid sequence

CAGGCTGTGGTCACACAAGAGCCTAGCCTGACAGTGTCTCCTGGCGGCACAGT
GACCCTGACCTGTAGATCTTCTACAG
GCGCCGTGACCACCAGCAACTACGCTAATTGGGTGCAGGAGAAGCCCGGCCA
GGCTCCTAGAGGACTGATCGGCGGAAC
AAACAAGAGAGCCCCTTGGACACCCGCCAGATTCTCTGGATCTCTGCTCGGCG
GAAAGGCCGCTCTGACAATCACTGGT
GCTCAGGCTGAGGACGAGGCCGAGTACTATTGTGTGCTGTGGTACAGCAACCT
GTGGGTGTTCGGCGGAGGCACCAAAC
TGACAGTTCTG

The amino acid sequence of hVL6 is shown in SEQ ID NO. 129; its encoding nucleic acid is shown in SEQ ID NO. 130; and its LCDR1, LCDR2, LCDR3 are shown in SEQ ID NO. 115, 131, 128, respectively.

QAVVTQEPSLTVSPGGTVTLTCRSSTGAVTTSNYANWFQEKPGQAPRGLIYGTNK
RAPWTPARFSGSLLGGKAA
LTLSGAQAEDEAEYYCVLWYSNLWVFGGGTKLTVL

### Nucleic acid sequence

CAGGCTGTGGTCACACAAGAGCCTAGCCTGACAGTGTCTCCTGGCGGCACAGT
GACCCTGACCTGTAGATCTTCTACAG
GCGCCGTGACCACCAGCAACTACGCTAATTGGTTCCAGGAGAAGCCCGGCCAG
GCTCCTAGAGGACTGATCTACGGAAC
AAACAAGAGAGCCCCTTGGACACCCGCCAGATTCTCTGGATCTCTGCTCGGCG
GAAAGGCCGCTCTGACACTTTCTGGT
GCTCAGGCTGAGGACGAGGCCGAGTACTATTGTGTCCTGTGGTACAGCAACCT
GTGGGTGTTCGGCGGAGGCACCAAAC
TGACAGTTCTG

**Table 9. Heavy chain variable region sequences of CD3 humanized antibodies**

| CD3 humanized antibody | SEQ ID NOs: 132 -161 | | | | |
|---|---|---|---|---|---|
| | HCVR | | HCDR1 | HCDR2 | HCDR3 |
| | Amino Acid Sequence | Nucleotide Sequence | Amino Acid Sequence | Amino Acid Sequence | Amino Acid Sequence |
| hVH1 | 132 | 133 | 134 | 135 | 136 |
| hVH2 | 137 | 138 | 139 | 135 | 136 |
| hVH3 | 140 | 141 | 139 | 135 | 142 |
| hVH4 | 143 | 144 | 139 | 135 | 145 |
| hVH5 | 146 | 147 | 139 | 135 | 148 |
| hVH6 | 149 | 150 | 139 | 135 | 151 |
| hVH7 | 152 | 153 | 154 | 155 | 136 |
| hVH8 | 156 | 157 | 134 | 135 | 136 |
| hVH9 | 158 | 159 | 134 | 135 | 136 |
| hVH10 | 160 | 161 | 134 | 135 | 136 |

The amino acid sequence of hVH1 is shown in SEQ ID NO. 132; its encoding nucleic acid is shown in SEQ ID NO. 133; and its HCDR1, HCDR2, HCDR3 are shown in SEQ ID NO. 134, 135, 136, respectively.

EVQLVESGGGLVQPGGSLRLSCAASGFTFNTYAMNWVRQAPGKGLEWVSRIRSK
YNNYATYYADSVKDRFTISRDDSKNTLYLQMNSLRAEDTAVYYCVRHGNFGNSY
VSWFAYWGQGTLVTVSS

### Nucleic acid sequence

GAGGTGCAGCTGGTTGAATCTGGCGGAGGACTGGTTCAGCCTGGCGGATCTCT
GAGACTGTCTTGTGCCGCCAGCGGCT
TCACCTTCAACACCTACGCTATGAACTGGGTCCGACAGGCCCCTGGCAAAGGA
CTGGAATGGGTGTCCAGAATCAGGTC
CAAGTACAACAACTACGCCACCTACTACGCCGACAGCGTGAAGGACAGATTCA
CCATCAGCAGGGACGACAGCAAGAAC
ACCCTGTACCTGCAGATGAACAGCCTGAGAGCCGAGGACACCGCCGTGTACTA
CTGTGTCAGACACGGCAACTTCGGCA
ACAGCTATGTGTCTTGGTTTGCCTACTGGGGCCAGGGCACACTGGTCACAGTTA
GCTCT

The amino acid sequence of hVH2 is shown in SEQ ID NO. 137; its encoding nucleic acid is shown in SEQ ID NO. 138; and its HCDR1, HCDR2, HCDR3 are shown in SEQ ID NO. 139, 135, 136, respectively.

EVQLVESGGGLVQPGGSLRLSCAASGFTFSTYAMNWVRKAPGKGLEWVSRIRSK
YNNYATYYADSVKDRFTISRDDSKNTLYLQMNSLRAEDTAVYYCVRHGNFGNSY
VSWFAYWGQGTLVTVSS

### Nucleic acid sequence

GAGGTGCAGCTGGTTGAATCTGGCGGAGGACTGGTTCAGCCTGGCGGATCTCT
GAGACTGTCTTGTGCCGCCAGCGGCT
TCACCTTCTCCACCTACGCTATGAACTGGGTCCGAAAGGCCCCTGGCAAAGGA
CTGGAATGGGTGTCCAGAATCAGGTC
CAAGTACAACAACTACGCCACCTACTACGCCGACAGCGTGAAGGACAGATTCA

CCATCAGCAGGGACGACAGCAAGAAC
ACCCTGTACCTGCAGATGAACAGCCTGAGAGCCGAGGACACCGCCGTGTACTA
CTGTGTCAGACACGGCAACTTCGGCA
ACAGCTATGTGTCTTGGTTTGCCTACTGGGGCCAGGGCACACTGGTCACAGTTA
GCTCT

The amino acid sequence of hVH3 is shown in SEQ ID NO. 140; its encoding nucleic acid is shown in SEQ ID NO. 141; and its HCDR1, HCDR2, HCDR3 are shown in SEQ ID NO. 139, 135, 142, respectively.

EVQLVESGGGLVQPGGSLRLSCAASGFTFSTYAMNWVRKAPGKGLEWVSRIRSK
YNNYATYYADSVKDRFTISRDDSKNTLYLQMNSLRAEDTAVYYCVRHGNFGESY
VSWFAYWGQGTLVTVSS

### Nucleic acid sequence

GAGGTGCAGCTGGTTGAATCTGGCGGAGGACTGGTTCAGCCTGGCGGATCTCT
GAGACTGTCTTGTGCCGCCAGCGGCT
TCACCTTCTCCACCTACGCTATGAACTGGGTCCGAAAGGCCCCTGGCAAAGGA
CTGGAATGGGTGTCCAGAATCAGGTC
CAAGTACAACAACTACGCCACCTACTACGCCGACAGCGTGAAGGACAGATTCA
CCATCAGCAGGGACGACAGCAAGAAC
ACCCTGTACCTGCAGATGAACAGCCTGAGAGCCGAGGACACCGCCGTGTACTA
CTGTGTCAGACACGGCAACTTCGGCG
AGAGCTATGTGTCTTGGTTTGCCTACTGGGGCCAGGGCACACTGGTCACAGTTA
GCTCT

The amino acid sequence of hVH4 is shown in SEQ ID NO. 143; its encoding nucleic acid is shown in SEQ ID NO. 144; and its HCDR1, HCDR2, HCDR3 are shown in SEQ ID NO. 139, 135, 145, respectively.

EVQLVESGGGLVQPGGSLRLSCAASGFTFSTYAMNWVRKAPGKGLEWVSRIRSK
YNNYATYYADSVKDRFTISRDDSKNTLYLQMNSLRAEDTAVYYCVRHGNFGQSY
VSWFAYWGQGTLVTVSS

### Nucleic acid sequence

GAGGTGCAGCTGGTTGAATCTGGCGGAGGACTGGTTCAGCCTGGCGGATCTCT

GAGACTGTCTTGTGCCGCCAGCGGCT
TCACCTTCTCCACCTACGCTATGAACTGGGTCCGAAAGGCCCCTGGCAAAGGA
CTGGAATGGGTGTCCAGAATCAGGTC
CAAGTACAACAACTACGCCACCTACTACGCCGACAGCGTGAAGGACAGATTCA
CCATCAGCAGGGACGACAGCAAGAAC
ACCCTGTACCTGCAGATGAACAGCCTGAGAGCCGAGGACACCGCCGTGTACTA
CTGTGTCAGACACGGCAACTTCGGCC
AGAGCTATGTGTCTTGGTTTGCCTACTGGGGCCAGGGCACACTGGTCACAGTTA
GCTCT

The amino acid sequence of hVH5 is shown in SEQ ID NO. 146; its encoding nucleic acid is shown in SEQ ID NO. 147; and its HCDR1, HCDR2, HCDR3 are shown in SEQ ID NO. 139, 135, 148, respectively.

EVQLVESGGGLVQPGGSLRLSCAASGFTFSTYAMNWVRKAPGKGLEWVSRIRSK
YNNYATYYADSVKDRFTISRDDSKNTLYLQMNSLRAEDTAVYYCVRHGNFGDSY
VSWFAYWGQGTLVTVSS

### Nucleic acid sequence

GAGGTGCAGCTGGTTGAATCTGGCGGAGGACTGGTTCAGCCTGGCGGATCTCT
GAGACTGTCTTGTGCCGCCAGCGGCT
TCACCTTCTCCACCTACGCTATGAACTGGGTCCGAAAGGCCCCTGGCAAAGGA
CTGGAATGGGTGTCCAGAATCAGGTC
CAAGTACAACAACTACGCCACCTACTACGCCGACAGCGTGAAGGACAGATTCA
CCATCAGCAGGGACGACAGCAAGAAC
ACCCTGTACCTGCAGATGAACAGCCTGAGAGCCGAGGACACCGCCGTGTACTA
CTGTGTCAGACACGGCAACTTCGGCG
ACAGCTATGTGTCTTGGTTTGCCTACTGGGGCCAGGGCACACTGGTCACAGTTA
GCTCT

The amino acid sequence of hVH6 is shown in SEQ ID NO. 149; its encoding nucleic acid is shown in SEQ ID NO. 150; and its HCDR1, HCDR2, HCDR3 are shown in SEQ ID NO. 139, 135, 151, respectively.

EVQLVESGGGLVQPGGSLRLSCAASGFTFSTYAMNWVRKAPGKGLEWVSRIRSK

YNNYATYYADSVKDRFTISRDDSKNTLYLQMNSLRAEDTAVYYCVRHGNFGTSY
VSWFAYWGQGTLVTVSS

### Nucleic acid sequence

GAGGTGCAGCTGGTTGAATCTGGCGGAGGACTGGTTCAGCCTGGCGGATCTCT
GAGACTGTCTTGTGCCGCCAGCGGCT
TCACCTTCTCCACCTACGCTATGAACTGGGTCCGAAAGGCCCCTGGCAAAGGA
CTGGAATGGGTGTCCAGAATCAGGTC
CAAGTACAACAACTACGCCACCTACTACGCCGACAGCGTGAAGGACAGATTCA
CCATCAGCAGGGACGACAGCAAGAAC
ACCCTGTACCTGCAGATGAACAGCCTGAGAGCCGAGGACACCGCCGTGTACTA
CTGTGTCAGACACGGCAACTTCGGCA
CCAGCTATGTGTCTTGGTTTGCCTACTGGGGCCAGGGCACACTGGTCACAGTTA
GCTCT

The amino acid sequence of hVH7 is shown in SEQ ID NO. 152; its encoding nucleic acid is shown in SEQ ID NO. 153; and its HCDR1, HCDR2, HCDR3 are shown in SEQ ID NO. 154, 155, 136, respectively.

EVQLVESGGGLVQPGGSLRLSCAASGFTFSDYAMNWVRKAPGKGLEWVSRIRSK
YNNYATYYADSVEDRFTISRDDSKNTLYLQMNSLRAEDTAVYYCVRHGNFGNSY
VSWFAYWGQGTLVTVSS

### Nucleic acid sequence

GAGGTGCAGCTGGTTGAATCTGGCGGAGGACTGGTTCAGCCTGGCGGATCTCT
GAGACTGTCTTGTGCCGCCAGCGGCT
TCACCTTCTCCGACTACGCTATGAACTGGGTCCGAAAGGCCCCTGGCAAAGGA
CTGGAATGGGTGTCCAGAATCAGGTC
CAAGTACAACAACTACGCCACCTACTACGCCGACAGCGTGGAGGACAGATTCA
CCATCAGCAGGGACGACAGCAAGAAC
ACCCTGTACCTGCAGATGAACAGCCTGAGAGCCGAGGACACCGCCGTGTACTA
CTGTGTCAGACACGGCAACTTCGGCA
ACAGCTATGTGTCTTGGTTTGCCTACTGGGGCCAGGGCACACTGGTCACAGTTA
GCTCT

The amino acid sequence of hVH8 is shown in SEQ ID NO. 156; its encoding nucleic acid is shown in SEQ ID NO. 157; and its HCDR1, HCDR2, HCDR3 are shown in SEQ ID NO. 134, 135, 136, respectively.

EVQLVESGGGLVQPGGSLRLSCAASGFTFNTYAMNWVRKAPGKGLEWVGRIRSK
YNNYATYYADSVKDRFTISRDDSKNSLYLQMNSLKTEDTAVYYCARHGNFGNSY
VSWFAYWGQGTLVTVSS

### Nucleic acid sequence

GAGGTGCAGCTGGTTGAATCTGGCGGAGGACTGGTTCAGCCTGGCGGATCTCT
GAGACTGTCTTGTGCCGCCAGCGGCT
TCACCTTCAACACCTACGCTATGAACTGGGTCCGAAAGGCCCCTGGCAAAGGA
CTGGAATGGGTGGGAAGAATCAGGTC
CAAGTACAACAACTACGCCACCTACTACGCCGACAGCGTGAAGGACAGATTCA
CCATCAGCAGGGACGACAGCAAGAAC
AGCCTGTACCTGCAGATGAACAGCCTGAAAACCGAGGACACCGCCGTGTACTA
CTGTGCCAGACACGGCAACTTCGGCA
ACAGCTATGTGTCTTGGTTTGCCTACTGGGGCCAGGGCACACTGGTCACAGTTA
GCTCT

The amino acid sequence of hVH9 is shown in SEQ ID NO. 158; its encoding nucleic acid is shown in SEQ ID NO. 159; and its HCDR1, HCDR2, HCDR3 are shown in SEQ ID NO. 134, 135, 136, respectively.

EVQLVESGGGLVQPGGSLRLSCAASGFTFNTYAMNWVRKAPGKGLEWVGRIRSK
YNNYATYYADSVKDRFTISRDDSKNSLYLQMNSLKTEDTAVYYCVRHGNFGNSY
VSWFAYWGQGTLVTVSS

### Nucleic acid sequence

GAGGTGCAGCTGGTTGAATCTGGCGGAGGACTGGTTCAGCCTGGCGGATCTCT
GAGACTGTCTTGTGCCGCCAGCGGCT
TCACCTTCAACACCTACGCTATGAACTGGGTCCGAAAGGCCCCTGGCAAAGGA
CTGGAATGGGTGGGAAGAATCAGGTC
CAAGTACAACAACTACGCCACCTACTACGCCGACAGCGTGAAGGACAGATTCA
CCATCAGCAGGGACGACAGCAAGAAC

AGCCTGTACCTGCAGATGAACAGCCTGAAAACCGAGGACACCGCCGTGTACTA
CTGTGTCAGACACGGCAACTTCGGCA
ACAGCTATGTGTCTTGGTTTGCCTACTGGGGCCAGGGCACACTGGTCACAGTTA
GCTCT

The amino acid sequence of hVH10 is shown in SEQ ID NO. 160; its encoding nucleic acid is shown in SEQ ID NO. 161; and its HCDR1, HCDR2, HCDR3 are shown in SEQ ID NO. 134, 135, 136, respectively.

EVQLVESGGGLVQPGGSLRLSCAASGFTFNTYAMNWVRKAPGKGLEWVARIRSK
YNNYATYYADSVKDRFTISRDDSKNSLYLQMNSLKTEDTAVYYCVRHGNFGNSY
VSWFAYWGQGTLVTVSS

### Nucleic acid sequence

GAGGTGCAGCTGGTTGAATCTGGCGGAGGACTGGTTCAGCCTGGCGGATCTCT
GAGACTGTCTTGTGCCGCCAGCGGCT
TCACCTTCAACACCTACGCTATGAACTGGGTCCGAAAGGCCCCTGGCAAAGGA
CTGGAATGGGTGGCCAGAATCAGGTC
CAAGTACAACAACTACGCCACCTACTACGCCGACAGCGTGAAGGACAGATTCA
CCATCAGCAGGGACGACAGCAAGAAC
AGCCTGTACCTGCAGATGAACAGCCTGAAAACCGAGGACACCGCCGTGTACTA
CTGTGTCAGACACGGCAACTTCGGCA
ACAGCTATGTGTCTTGGTTTGCCTACTGGGGCCAGGGCACACTGGTCACAGTTA
GCTCT

The light and heavy chain humanized variants were subjected to full sequence synthesis respectively, and cloned into an eukaryotic expression vector containing antibody lambda light chain constant region or human IgG4 heavy chain constant region CH1-CH3. They were co-transfected into HEK293E cells and cultured at 37 °C, 120 rpm, and 5% CO₂ for 5-6 days. The supernatant of the culture medium was collected and purified using the Protein A chromatography column.

### 2. Affinity of humanized CD3 antibody

Human CD3 εγ protein was coated overnight at 4 °C. After blocking with 2% skim milk, different dilutions of CD3 antibodies was added to each well and incubated for 1 hour. The secondary antibody HPR-labeled goat anti-human IgG Fc was added. After being developed by the TMB solution, the reaction was stopped with the concentrated sulfuric acid, and the absorbance was read at 450 nm (the results are shown in FIG. 12 and Table 8). FIG. 12 shows a combination of anti-CD3 humanized antibodies (including aCD3-hVH8/VL1, aCD3-hVH9/VL1, aCD3-hVH9/VL2, aCD3-hVH9/VL3, aCD3-hVH1/VL5, aCD3-hVH8/VL5, aCD3-hVH9/VL5) that bind to human CD3 εγ protein, with the CD3 humanized antibody binding with high affinity to the recombinant CD3 εγ protein.

### 3. Recognition of Jurkat T cells by CD3 humanized antibody

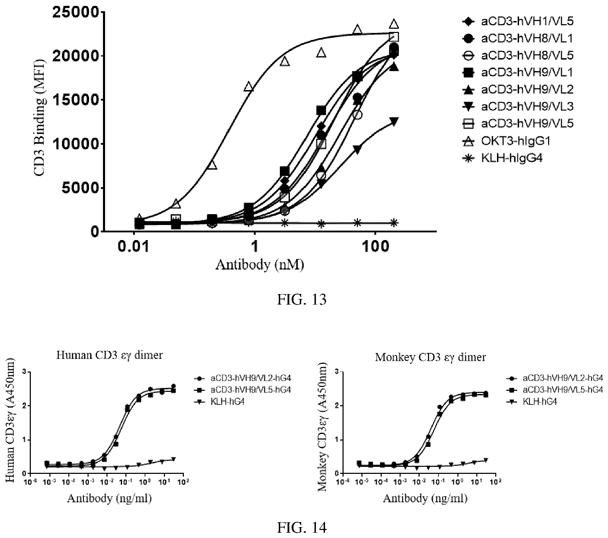

Jurkat cells in the logarithmic growth phase were taken, blocked with 3% BSA for 30 min, and added to 96-well U-plate at 5 × 10⁴ cells per well. The supernatant was discarded after centrifugation. 50 µL of gradient diluted antibody was added (antibody concentration: from 30 µg/mL, 3-fold dilution for 5 gradients) per well, and incubated at 4°C for 1 hour. After washing off the primary antibodies, the secondary antibody Alexa Fluro647-labeled goat anti-human IgG Fc was added with 1: 300 dilution (Jackson ImmunoResearch, 109-606-170) and incubated for 45 min at 4°C. After washing, 50 µL PBS was added per well to perform resuspending for FACS (iQue, intellicyt) assay (the results are seen in FIG. 13, and Table 10).

**Table 10. Affinities of CD3 humanized antibodies**

| | ELISA (CD3εγ) | FACS Jurkat |
|---|---|---|
| aCD3-hVH1/VL5 | 0.60 nM | 10 nM |
| aCD3-hVH8/VL1 | 0.65 nM | 15 nM |
| aCD3-hVH8/VL5 | 0.74 nM | Slightly weak |
| aCD3-hVH9/VL1 | 0.49 nM | 7 nM |
| aCD3-hVH9/VL2 | 0.44 nM | 25 nM |
| aCD3-hVH9/VL3 | 0.70 nM | Weak |
| aCD3-hVH9/VL5 | 0.42 nM | 20 nM |
| OKT3-hIgG1 | ND | 0.27 nM |
| KLH-hIgG4 | - | - |

| | | |
|---|---|---|
| ND: not detected -: No binding | | |

FIG. 13 shows a combination of anti-CD3 humanized antibodies that bind to Jurkat cells, where the CD3 humanized antibodies hVH9/VL5 (aCD3-hVH9/VL5) and hVH9/VL2 (aCD3-hVH9/VL2) are both significantly weaker than the control antibody OKT3, binding to Jurkat cells with moderate affinity.

### 4. Cross-recognition of CD3 humanized antibodies with human and monkey CD3 εγ antigens

Human CD3 εγ protein and monkey CD3 εγ protein were coated separately overnight at 4°C. After blocking with 2% skim milk, different dilutions of CD3 antibody were added to each well and incubated for 1 hour. The secondary antibody HPR-labeled goat anti-human IgG Fc was added. After being developed by the TMB solution. The reaction was stopped with the concentrated sulfuric acid and the absorbance was read at 450 nm. FIG. 14 shows that both CD3 humanized antibodies hVH9/VL5 (aCD3-hVH9/VL5) and hVH9/VL2 (aCD3-hVH9/VL2) bind both human CD3 εγ and monkey CD3 εγ proteins.

### 5. Construction of humanized GPC3 × CD3 κλ bispecific antibody

Humanized antibody 49G8VHv6VKv3 and humanized CD3 antibody hVH9/VL5 containing λ light chain (aCD3-hVH9/VL5) were used to construct a novel GPC3-CD3κλ humanized bispecific antibody with natural IgG configuration. At same time, the GPC3 antigen arm and CD3 arm were introduced with charge variants (Vκ GPC3: Gln₄₃Lys; VH_{GPC3}: Gln₃₉Glu; Vλ_{CD3}: Gln₄₀Glu; VH_{CD3}: Gln₃₉Lys) (Table 11). The Fc portion of the bispecific antibody adopts a human IgG4 knob-into-hole structure to achieve heterodimer pairing (Atwell et al. 1997). Through mutations Ser228Pro, Leu235Glu, and Pro329Ala, the hinge region was kept stable while completely eliminating interactions with Fcγ receptors and C1q.

Plasmids encoding the corresponding antibody fragments were mixed in a ratio of kappa light chains: λ light chain: heavy Chain 1: heavy chain 2 = 2:2:1:1. The mixture was mixed with 3 mg/mL PEI, co-transfected into CHO-S cells, and cultured in 500mL CD CHO AGT medium (Gibco # 12490-001) at 37°C, 5% CO₂, and 150 rpm. 4% CHO Feed C+ supplement (Gibco #A25031-05) was added on days 2, 4, and 6 of instant transfection, respectively. When the cell viability decreased to about 85%, the fermentation broth was harvested, filtered, and purified by the Protein A affinity chromatography. The SEC-HPLC monomer content was higher than 92%. The monomer content was further increased to above 99.5% by Butyl HP hydrophobic chromatography and Capto Q anion chromatography (Table 12).

**Table 11. GPC3-CD3 κλ humanized bispecific antibody**

| | GPC3 arm light chain | GPC3 arm heavy chain | CD3 arm light chain | CD3 arm heavy chain |
|---|---|---|---|---|
| GPC3 x CD3 κλ002 | SEQ ID NO. 162 | SEQ ID NO. 164 | SEQ ID NO. 166 | SEQ ID NO. 168 |
| GPC3×CD3 κλ003 | SEQ ID NO. 170 | SEQ ID NO. 172 | SEQ ID NO. 166 | SEQ ID NO. 168 |

GPC3 x CD3 κλ002:
GPC3 arm light chain SEQ ID NO. 162 
DVVMTQSPLSLPVTPGEPASISCRSSQSIVHSNGNTYLEWYLKKPGQSPQLLIYKVS
NRFSGVPDRFSGSGSGTDFTLKISRVEAEDVGVYFCLQVTHVPLTFGQGTKLEIKR
TVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
Nucleotide SEQ ID NO. 163 
GACGTGGTCATGACACAGAGCCCTCTGAGCCTGCCTGTGACACCTGGCGAACC
TGCCAGCATCAGCTGTAGAAGCAGCCAGAGCATCGTGCACAGCAACGGCAAC
ACATACCTGGAGTGGTATCTGAAGAAGCCCGGCCAGTCTCCTCAGCTGCTGATC
TACAAGGTGTCCAACAGATTCAGCGGCGTGCCCGACAGATTCTCTGGCTCTGG
ATCTGGCACCGACTTCACCCTGAAGATCTCCAGAGTGGAAGCCGAGGACGTGG
GCGTGTACTTCTGTCTCCAGGTCACACACGTGCCCCTGACATTTGGCCAGGGCA
CCAAGCTGGAAATCAAGCGAACTGTGGCTGCACCATCTGTCTTCATCTTCCCGC
CATCTGATGAGCAGTTGAAATCTGGAACTGCCTCTGTTGTGTGCCTGCTGAATA
ACTTCTATCCCAGAGAGGCCAAAGTACAGTGGAAGGTGGATAACGCCCTCCAA
TCGGGTAACTCCCAGGAGAGTGTCACAGAGCAGGACAGCAAGGACAGCACCT
 
ACAGCCTCAGCAGCACCCTGACGCTGAGCAAAGCAGACTACGAGAAACACAA
AGTCTACGCCTGCGAAGTCACCCATCAGGGCCTGAGCTCGCCCGTCACAAAGA
GCTTCAACAGGGGAGAGTGT
GPC3 arm heavy chain SEQ ID NO. 164 
QVQLVQSGAEVKKPGSSVKVSCKASGYTFADYEIHWVREAPGQGLEWMGAIHPG
SGGTAYAQKFQGRVTLTADESSTTAYMELSSLRSEDTAVYYCTRYYSFAYWGQGTL
VTVSSASTKGPSVFPLAPCSRSTSESTAALGCLVKDYFPEPVTVSWNSGALTSGVH
TFPAVLQSSGLYSLSSVVTVPSSSLGTKTYTCNVDHKPSNTKVDKRVESKYGPPCP
PCPAPEFEGGPSVFLFPPKPKDTLMISRTPEVTCVVVDVSQEDPEVQFNWYVDGVE
VHNAKTKPREEQFNSTYRVVSVLTVLHQDWLNGKEYKCKVSNKGLASSIEKTISK
AKGQPREPQVCTLPPSQEEMTKNQVSLSCAVKGFYPSDIAVEWESNGQPENNYKT
TPPVLDSDGSFFLVSKLTVDKSRWQEGNVFSCSVMHEALHNHYTQKSLSLSLGK
Nucleotide SEQ ID NO. 165 
CAGGTTCAGCTGGTTCAGTCTGGCGCCGAAGTGAAGAAACCTGGCAGCAGCG
TGAAGGTGTCCTGCAAGGCTAGCGGCTACACCTTCGCCGACTACGAGATCCAC
TGGGTCCGAGAGGCTCCAGGACAGGGACTTGAATGGATGGGCGCTATCCATCC
TGGCTCTGGCGGCACAGCTTACGCCCAGAAATTCCAGGGCAGAGTGACCCTGA
CCGCCGACGAGTCTAGCACCACCGCCTACATGGAACTGAGCAGCCTGAGAAGC
GAGGACACCGCCGTGTACTACTGCACCCGGTACTACAGCTTCGCCTACTGGGG
ACAGGGAACCCTGGTCACAGTCAGCTCTGCTAGCACCAAGGGCCCCAGCGTGT
TCCCCCTGGCCCCTTGCAGCAGAAGCACCAGCGAGAGCACAGCCGCCCTGGG
CTGCCTGGTGAAGGACTACTTCCCCGAGCCCGTGACCGTGTCCTGGAACAGCG
GCGCTCTGACCAGCGGCGTGCATACCTTCCCCGCCGTGCTCCAGAGCAGCGGA
CTGTACTCCCTGAGCAGCGTGGTGACCGTGCCTTCCAGCAGCCTGGGCACCAA
GACCTACACCTGCAACGTGGACCACAAGCCCAGCAACACCAAGGTGGACAAG
AGAGTGGAGAGCAAGTACGGCCCTCCCTGCCCCCCTTGCCCTGCCCCCGAGTT
CGAGGGCGGACCTAGCGTGTTCCTGTTCCCCCCCAAGCCCAAGGACACCCTGA
TGATCAGCAGAACCCCCGAGGTGACCTGCGTGGTGGTGGACGTGTCCCAGGA
GGACCCCGAGGTCCAGTTTAATTGGTACGTGGACGGCGTGGAAGTGCATAACG
CCAAGACCAAGCCCAGAGAGGAGCAGTTCAACAGCACCTACAGAGTGGTGTC
 
CGTGCTGACCGTGCTGCACCAGGACTGGCTGAACGGCAAGGAATACAAGTGC
AAGGTCTCCAACAAGGGCCTGGCCAGCAGCATCGAGAAGACCATCAGCAAGG
CCAAGGGCCAGCCACGGGAGCCCCAGGTCTGCACCCTGCCACCTAGCCAAGA
GGAGATGACCAAGAACCAGGTGTCCCTGAGCTGTGCCGTGAAAGGCTTCTATC
CCAGCGATATCGCCGTGGAGTGGGAGAGCAACGGCCAGCCCGAGAACAACTA
CAAGACCACCCCCCCTGTGCTGGACAGCGACGGCAGCTTCTTCCTGGTTTCCA
AGCTGACCGTGGACAAGTCCAGATGGCAGGAGGGCAACGTCTTCAGCTGCTCC
GTGATGCACGAGGCCCTGCACAACCACTACACCCAGAAGTCCCTGAGCCTGAG
CCTGGGCAAG
CD3 arm light chain SEQ ID NO. 166 
QAVVTQEPSLTVSPGGTVTLTCRSSTGAVTTSNYANWVQEKPGQAPRGLIGGTNK
RAPWTPARFSGSLLGGKAALTITGAQAEDEAEYYCVLWYSNLWVFGGGTKLTVL
GQPKAAPSVTLFPPSSEELQANKATLVCLISDFYPGAVTVAWKADSSPVKAGVETT
TPSKQSNNKYAASSYLSLTPEQWKSHRSYSCQVTHEGSTVEKTVAPTECS
Nucleotide SEQ ID NO. 167 
CAGGCTGTGGTCACACAAGAGCCTAGCCTGACAGTGTCTCCTGGCGGCACAGT
GACCCTGACCTGTAGATCTTCTACAGGCGCCGTGACCACCAGCAACTACGCTAA
TTGGGTGCAGGAGAAGCCCGGCCAGGCTCCTAGAGGACTGATCGGCGGAACA
AACAAGAGAGCCCCTTGGACACCCGCCAGATTCTCTGGATCTCTGCTCGGCGG
AAAGGCCGCTCTGACAATCACTGGTGCTCAGGCTGAGGACGAGGCCGAGTACT
ATTGTGTGCTGTGGTACAGCAACCTGTGGGTGTTCGGCGGAGGCACCAAACTG
ACAGTTCTGGGTCAGCCCAAGGCGGCGCCCTCGGTCACTCTGTTCCCGCCCTC
CTCTGAGGAGCTTCAAGCCAACAAGGCCACACTGGTGTGTCTCATAAGTGACT
TCTATCCGGGAGCCGTGACAGTGGCCTGGAAGGCAGATAGCAGCCCCGTCAAG
GCGGGAGTGGAGACCACCACACCCTCCAAACAAAGCAACAACAAGTACGCGG
CCAGCAGCTACCTGAGCCTGACGCCTGAGCAGTGGAAGTCCCACAGAAGCTAC
AGCTGCCAGGTCACGCATGAAGGGAGCACCGTGGAGAAGACAGTGGCCCCTA
CAGAATGTTCA
CD3 arm heavy chain SEQ ID NO. 168 EVQLVESGGGLVQPGGSLRLSCAASGFTFNTYAMNWVRKAPGKGLEWVGRIRSK 
YNNYATYYADSVKDRFTISRDDSKNSLYLQMNSLKTEDTAVYYCVRHGNFGNSY
VSWFAYWGQGTLVTVSSASTKGPSVFPLAPCSRSTSESTAALGCLVKDYFPEPVTV
SWNSGALTSGVHTFPAVLQSSGLYSLSSVVTVPSSSLGTKTYTCNVDHKPSNTKVD
KRVESKYGPPCPPCPAPEFEGGPSVFLFPPKPKDTLMISRTPEVTCVVVDVSQEDPE
VQFNWYVDGVEVHNAKTKPREEQFNSTYRVVSVLTVLHQDWLNGKEYKCKVSN
KGLASSIEKTISKAKGQPREPQVYTLPPCQEEMTKNQVSLWCLVKGFYPSDIAVEW
ESNGQPENNYKTTPPVLDSDGSFFLYSKLTVDKSRWQEGNVFSCSVMHEALHNHY
TQKSLSLSLGK
Nucleotide SEQ ID NO. 169 
GAGGTGCAGCTGGTTGAATCTGGCGGAGGACTGGTTCAGCCTGGCGGATCTCT
GAGACTGTCTTGTGCCGCCAGCGGCTTCACCTTCAACACCTACGCTATGAACTG
GGTCCGAAAGGCCCCTGGCAAAGGACTGGAATGGGTGGGAAGAATCAGGTCC
AAGTACAACAACTACGCCACCTACTACGCCGACAGCGTGAAGGACAGATTCAC
CATCAGCAGGGACGACAGCAAGAACAGCCTGTACCTGCAGATGAACAGCCTG
AAAACCGAGGACACCGCCGTGTACTACTGTGTCAGACACGGCAACTTCGGCAA
CAGCTATGTGTCTTGGTTTGCCTACTGGGGCCAGGGCACACTGGTCACAGTTAG
CTCTGCTAGCACCAAGGGCCCCAGCGTGTTCCCCCTGGCCCCTTGCAGCAGAA
GCACCAGCGAGAGCACAGCCGCCCTGGGCTGCCTGGTGAAGGACTACTTCCCC
GAGCCCGTGACCGTGTCCTGGAACAGCGGCGCTCTGACCAGCGGCGTGCATAC
CTTCCCCGCCGTGCTCCAGAGCAGCGGACTGTACTCCCTGAGCAGCGTGGTGA
CCGTGCCTTCCAGCAGCCTGGGCACCAAGACCTACACCTGCAACGTGGACCAC
AAGCCCAGCAACACCAAGGTGGACAAGAGAGTGGAGAGCAAGTACGGCCCTC
CCTGCCCCCCTTGCCCTGCCCCCGAGTTCGAGGGCGGACCTAGCGTGTTCCTGT
TCCCCCCCAAGCCCAAGGACACCCTGATGATCAGCAGAACCCCCGAGGTGACC
TGCGTGGTGGTGGACGTGTCCCAGGAGGACCCCGAGGTCCAGTTTAATTGGTA
CGTGGACGGCGTGGAAGTGCATAACGCCAAGACCAAGCCCAGAGAGGAGCAG
TTCAACAGCACCTACAGAGTGGTGTCCGTGCTGACCGTGCTGCACCAGGACTG
GCTGAACGGCAAGGAATACAAGTGCAAGGTCTCCAACAAGGGCCTGGCCAGC
AGCATCGAGAAGACCATCAGCAAGGCCAAGGGCCAGCCACGGGAGCCCCAGG
TCTACACCCTGCCACCTTGTCAAGAGGAGATGACCAAGAACCAGGTGTCCCTG
 
TGGTGTCTGGTGAAAGGCTTCTATCCCAGCGATATCGCCGTGGAGTGGGAGAG
CAACGGCCAGCCCGAGAACAACTACAAGACCACCCCCCCTGTGCTGGACAGC
GACGGCAGCTTCTTCCTGTACTCCAAGCTGACCGTGGACAAGTCCAGATGGCA
GGAGGGCAACGTCTTCAGCTGCTCCGTGATGCACGAGGCCCTGCACAACCACT
ACACCCAGAAGTCCCTGAGCCTGAGCCTGGGCAAG
 GPC3 × CD3 κλ003
GPC3 arm light chain SEQ ID NO. 170 
DVVMTQSPLSLPVTPGEPASISCRSSQSIVHSNGNTYLEWYLKKPGQSPQLLIYKVS
NRFSGVPDRFSGSGSGTDFTLKISRVEAEDVGVYFCLQVTHVPLTFGQGTKLEIKR
TVAAPSVFIFPPSDKKLKSGTASVVCLLNNFYPREAKVQWKVDNALQSGNSQESV
TEQDSKDSTYSLSSTLTLSKADYEKHKVYACEVTHQGLSSPVTKSFNRGEC
Nucleotide SEQ ID NO. 171 
GACGTGGTCATGACACAGAGCCCTCTGAGCCTGCCTGTGACACCTGGCGAACC
TGCCAGCATCAGCTGTAGAAGCAGCCAGAGCATCGTGCACAGCAACGGCAAC
ACATACCTGGAGTGGTATCTGAAGAAGCCCGGCCAGTCTCCTCAGCTGCTGATC
TACAAGGTGTCCAACAGATTCAGCGGCGTGCCCGACAGATTCTCTGGCTCTGG
ATCTGGCACCGACTTCACCCTGAAGATCTCCAGAGTGGAAGCCGAGGACGTGG
GCGTGTACTTCTGTCTCCAGGTCACACACGTGCCCCTGACATTTGGCCAGGGCA
CCAAGCTGGAAATCAAGCGAACTGTGGCTGCACCATCTGTCTTCATCTTCCCGC
CATCTGATAAGAAATTGAAATCTGGAACTGCCTCTGTTGTGTGCCTGCTGAATA
ACTTCTATCCCAGAGAGGCCAAAGTACAGTGGAAGGTGGATAACGCCCTCCAA
TCGGGTAACTCCCAGGAGAGTGTCACAGAGCAGGACAGCAAGGACAGCACCT
ACAGCCTCAGCAGCACCCTGACGCTGAGCAAAGCAGACTACGAGAAACACAA
AGTCTACGCCTGCGAAGTCACCCATCAGGGCCTGAGCTCGCCCGTCACAAAGA
GCTTCAACAGGGGAGAGTGT
GPC3 arm heavy chain SEQ ID NO. 172 
QVQLVQSGAEVKKPGSSVKVSCKASGYTFADYEIHWVREAPGQGLEWMGAIHPG
SGGTAYAQKFQGRVTLTADESSTTAYMELSSLRSEDTAVYYCTRYYSFAYWGQGTL
VTVSSASTKGPSVFPLAPCSRSTSESTAALGCLVEDYFPEPVTVSWNSGALTSGVH
TFPAVLQSSGLYSLSSVVTVPSSSLGTKTYTCNVDHKPSNTKVDERVESKYGPPCPP
 
CPAPEFEGGPSVFLFPPKPKDTLMISRTPEVTCVVVDVSQEDPEVQFNWYVDGVE
VHNAKTKPREEQFNSTYRVVSVLTVLHQDWLNGKEYKCKVSNKGLASSIEKTISK
AKGQPREPQVCTLPPSQEEMTKNQVSLSCAVKGFYPSDIAVEWESNGQPENNYKT
TPPVLDSDGSFFLVSKLTVDKSRWQEGNVFSCSVMHEALHNHYTQKSLSLSLGK
Nucleotide SEQ ID NO. 173 
CAGGTTCAGCTGGTTCAGTCTGGCGCCGAAGTGAAGAAACCTGGCAGCAGCG
TGAAGGTGTCCTGCAAGGCTAGCGGCTACACCTTCGCCGACTACGAGATCCAC
TGGGTCCGAGAGGCTCCAGGACAGGGACTTGAATGGATGGGCGCTATCCATCC
TGGCTCTGGCGGCACAGCTTACGCCCAGAAATTCCAGGGCAGAGTGACCCTGA
CCGCCGACGAGTCTAGCACCACCGCCTACATGGAACTGAGCAGCCTGAGAAGC
GAGGACACCGCCGTGTACTACTGCACCCGGTACTACAGCTTCGCCTACTGGGG
ACAGGGAACCCTGGTCACAGTCAGCTCTGCTAGCACCAAGGGCCCCAGCGTGT
TCCCCCTGGCCCCTTGCAGCAGAAGCACCAGCGAGAGCACAGCCGCCCTGGG
CTGCCTGGTGGAGGACTACTTCCCCGAGCCCGTGACCGTGTCCTGGAACAGCG
GCGCTCTGACCAGCGGCGTGCATACCTTCCCCGCCGTGCTCCAGAGCAGCGGA
CTGTACTCCCTGAGCAGCGTGGTGACCGTGCCTTCCAGCAGCCTGGGCACCAA
GACCTACACCTGCAACGTGGACCACAAGCCCAGCAACACCAAGGTGGACGAG
AGAGTGGAGAGCAAGTACGGCCCTCCCTGCCCCCCTTGCCCTGCCCCCGAGTT
CGAAGGCGGACCTAGCGTGTTCCTGTTCCCCCCCAAGCCCAAGGACACCCTGA
TGATCAGCAGAACCCCCGAGGTGACCTGCGTGGTGGTGGACGTGTCCCAGGA
GGACCCCGAGGTCCAGTTTAATTGGTACGTGGACGGCGTGGAAGTGCATAACG
CCAAGACCAAGCCCAGAGAGGAGCAGTTCAACAGCACCTACAGAGTGGTGTC
CGTGCTGACCGTGCTGCACCAGGACTGGCTGAACGGCAAGGAATACAAGTGC
AAGGTCTCCAACAAGGGCCTGGCCAGCAGCATCGAGAAGACCATCAGCAAGG
CCAAGGGCCAGCCACGGGAGCCCCAGGTCTGCACCCTGCCACCTAGCCAAGA
GGAGATGACCAAGAACCAGGTGTCCCTGAGCTGTGCCGTGAAAGGCTTCTATC
CCAGCGATATCGCCGTGGAGTGGGAGAGCAACGGCCAGCCCGAGAACAACTA
CAAGACCACCCCCCCTGTGCTGGACAGCGACGGCAGCTTCTTCCTGGTTTCCA
AGCTGACCGTGGACAAGTCCAGATGGCAGGAGGGCAACGTCTTCAGCTGCTCC
GTGATGCACGAGGCCCTGCACAACCACTACACCCAGAAGTCCCTGAGCCTGAG
 CCTGGGCAAG
CD3 arm light chain SEQ ID NO. 166 
QAVVTQEPSLTVSPGGTVTLTCRSSTGAVTTSNYANWVQEKPGQAPRGLIGGTNK
RAPWTPARFSGSLLGGKAALTITGAQAEDEAEYYCVLWYSNLWVFGGGTKLTVL
GQPKAAPSVTLFPPSSEELQANKATLVCLISDFYPGAVTVAWKADSSPVKAGVETT
TPSKQSNNKYAASSYLSLTPEQWKSHRSYSCQVTHEGSTVEKTVAPTECS
Nucleotide SEQ ID NO. 167 
CAGGCTGTGGTCACACAAGAGCCTAGCCTGACAGTGTCTCCTGGCGGCACAGT
GACCCTGACCTGTAGATCTTCTACAGGCGCCGTGACCACCAGCAACTACGCTAA
TTGGGTGCAGGAGAAGCCCGGCCAGGCTCCTAGAGGACTGATCGGCGGAACA
AACAAGAGAGCCCCTTGGACACCCGCCAGATTCTCTGGATCTCTGCTCGGCGG
AAAGGCCGCTCTGACAATCACTGGTGCTCAGGCTGAGGACGAGGCCGAGTACT
ATTGTGTGCTGTGGTACAGCAACCTGTGGGTGTTCGGCGGAGGCACCAAACTG
ACAGTTCTGGGTCAGCCCAAGGCGGCGCCCTCGGTCACTCTGTTCCCGCCCTC
CTCTGAGGAGCTTCAAGCCAACAAGGCCACACTGGTGTGTCTCATAAGTGACT
TCTATCCGGGAGCCGTGACAGTGGCCTGGAAGGCAGATAGCAGCCCCGTCAAG
GCGGGAGTGGAGACCACCACACCCTCCAAACAAAGCAACAACAAGTACGCGG
CCAGCAGCTACCTGAGCCTGACGCCTGAGCAGTGGAAGTCCCACAGAAGCTAC
AGCTGCCAGGTCACGCATGAAGGGAGCACCGTGGAGAAGACAGTGGCCCCTA
CAGAATGTTCA
CD3 arm heavy chain SEQ ID NO. 168 
EVQLVESGGGLVQPGGSLRLSCAASGFTFNTYAMNWVRKAPGKGLEWVGRIRSK
YNNYATYYADSVKDRFTISRDDSKNSLYLQMNSLKTEDTAVYYCVRHGNFGNSY
VSWFAYWGQGTLVTVSSASTKGPSVFPLAPCSRSTSESTAALGCLVKDYFPEPVTV
SWNSGALTSGVHTFPAVLQSSGLYSLSSVVTVPSSSLGTKTYTCNVDHKPSNTKVD
KRVESKYGPPCPPCPAPEFEGGPSVFLFPPKPKDTLMISRTPEVTCVVVDVSQEDPE
VQFNWYVDGVEVHNAKTKPREEQFNSTYRVVSVLTVLHQDWLNGKEYKCKVSN
KGLASSIEKTISKAKGQPREPQVYTLPPCQEEMTKNQVSLWCLVKGFYPSDIAVEW
ESNGQPENNYKTTPPVLDSDGSFFLYSKLTVDKSRWQEGNVFSCSVMHEALHNHY
TQKSLSLSLGK
Nucleotide SEQ ID NO. 169

GAGGTGCAGCTGGTTGAATCTGGCGGAGGACTGGTTCAGCCTGGCGGATCTCT
GAGACTGTCTTGTGCCGCCAGCGGCTTCACCTTCAACACCTACGCTATGAACTG
GGTCCGAAAGGCCCCTGGCAAAGGACTGGAATGGGTGGGAAGAATCAGGTCC
AAGTACAACAACTACGCCACCTACTACGCCGACAGCGTGAAGGACAGATTCAC
CATCAGCAGGGACGACAGCAAGAACAGCCTGTACCTGCAGATGAACAGCCTG
AAAACCGAGGACACCGCCGTGTACTACTGTGTCAGACACGGCAACTTCGGCAA
CAGCTATGTGTCTTGGTTTGCCTACTGGGGCCAGGGCACACTGGTCACAGTTAG
CTCTGCTAGCACCAAGGGCCCCAGCGTGTTCCCCCTGGCCCCTTGCAGCAGAA
GCACCAGCGAGAGCACAGCCGCCCTGGGCTGCCTGGTGAAGGACTACTTCCCC
GAGCCCGTGACCGTGTCCTGGAACAGCGGCGCTCTGACCAGCGGCGTGCATAC
CTTCCCCGCCGTGCTCCAGAGCAGCGGACTGTACTCCCTGAGCAGCGTGGTGA
CCGTGCCTTCCAGCAGCCTGGGCACCAAGACCTACACCTGCAACGTGGACCAC
AAGCCCAGCAACACCAAGGTGGACAAGAGAGTGGAGAGCAAGTACGGCCCTC
CCTGCCCCCCTTGCCCTGCCCCCGAGTTCGAGGGCGGACCTAGCGTGTTCCTGT
TCCCCCCCAAGCCCAAGGACACCCTGATGATCAGCAGAACCCCCGAGGTGACC
TGCGTGGTGGTGGACGTGTCCCAGGAGGACCCCGAGGTCCAGTTTAATTGGTA
CGTGGACGGCGTGGAAGTGCATAACGCCAAGACCAAGCCCAGAGAGGAGCAG
TTCAACAGCACCTACAGAGTGGTGTCCGTGCTGACCGTGCTGCACCAGGACTG
GCTGAACGGCAAGGAATACAAGTGCAAGGTCTCCAACAAGGGCCTGGCCAGC
AGCATCGAGAAGACCATCAGCAAGGCCAAGGGCCAGCCACGGGAGCCCCAGG
TCTACACCCTGCCACCTTGTCAAGAGGAGATGACCAAGAACCAGGTGTCCCTG
TGGTGTCTGGTGAAAGGCTTCTATCCCAGCGATATCGCCGTGGAGTGGGAGAG
CAACGGCCAGCCCGAGAACAACTACAAGACCACCCCCCCTGTGCTGGACAGC
GACGGCAGCTTCTTCCTGTACTCCAAGCTGACCGTGGACAAGTCCAGATGGCA
GGAGGGCAACGTCTTCAGCTGCTCCGTGATGCACGAGGCCCTGCACAACCACT
ACACCCAGAAGTCCCTGAGCCTGAGCCTGGGCAAG

**Table 12. Purification of GPC3-CD3 humanized bispecific antibody**

| HPLC | HPLC-SEC (post-Protein A) | HPLC-SEC | (post-anion | | | |
|---|---|---|---|---|---|---|
| detection | | | | chromatography) | | |
| | Multimer | Monomers | Fragments | Multimer | Monomer | Fragments |
| GPC3 x CD3 κλ002 | 2.6% | 94.2% | 3.1% | 0.0% | 100.0% | 0.0% |
| GPC3 x CD3 κλ003 | 2.2% | 93.2% | 4.5% | 0.1% | 99.5% | 0.4% |

### EXAMPLES 4. Binding activity of bispecific antibody GPC3 × CD3 κλ

The affinity of the bispecific antibody GPC3 antigen arm was determined by measuring the binding to recombinant GPC3 protein, overexpressed stably-transfected cells, or GPC3+ tumor cells, respectively. The affinity of the bispecific antibody CD3 arm was determined by measuring the binding to Jurkat cells.

### 1. Determination of affinity of GPC3 × CD3 κλ bispecific antibody to GPC3

The antibodies with a concentration of 1 µg/ml were captured on the Protein A chip (GE healthcare). Different concentrations of recombinant human or cynomolgus monkey GPC3 protein (5 µg/ml as the initial concentration, 2-fold gradient, a total of 6 concentration points) as the analyte was flowed through the chip at a flow rate of 30 µL/min, with an association time of 120 seconds, and a dissociation time of 400 seconds. The changes in the SPR signal during the association and dissociation of the protein were recorded. The kinetic constants were obtained by fitting with 1: 1 binding model using Biacore T200 evaluation software.

Human or monkey CD3 εγ recombinant antigen at 10 µg/mL was bound to a CM5 chip (GE healthcare) by amino coupling, and the antigen binding capacity was controlled to approximately 200 RU. After the baseline was stable, gradient diluted antibodies (initially with 10 µg/mL, 2-fold dilution, 7 gradients) were flowed through the chip at a flow rate of 30 µL/min, with an association time of 350 seconds and a dissociation time of 600 seconds. The kinetic constants were obtained by fitting with 1: 1 binding model using Biacore T200 evaluation software. The results of the affinity determination are shown in Table 13.

**Table 13. Affinities of GPC3 × CD3 κλ bispecific antibodies to GPC3 and CD3 recombinant antigens**

| KD (nM) | Human GPC3 | Monkey GPC3 | Human CD3εγ | Monkey CD3εγ |
|---|---|---|---|---|
| GPC3 x CD3 κλ002 | 1.35 | 1.60 | 15.9 | 23.9 |
| GPC3 x CD3 κλ003 | 2.11 | 1.91 | 12.1 | 17.8 |

### 2. Binding of GPC3 × CD3 κλ bispecific antibody to GPC3 stably-transfected cells

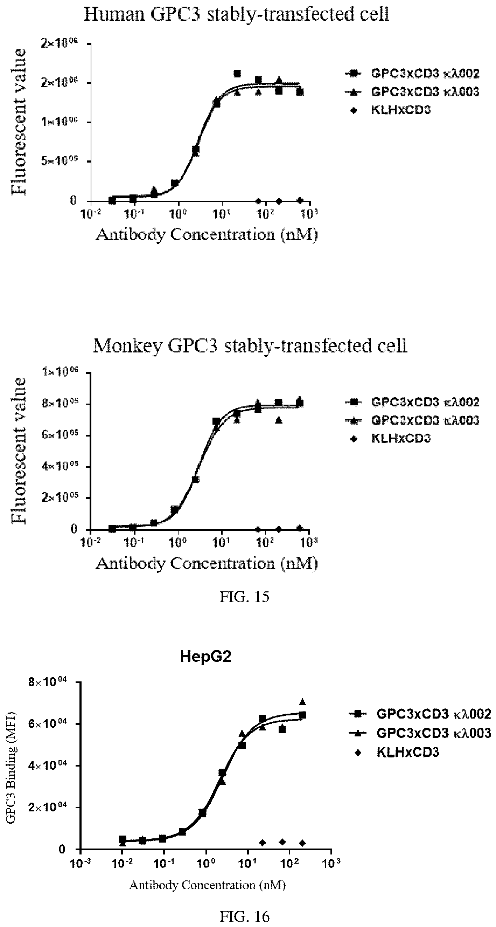

CHO-human GPC3 and CHO-cynomolgus monkey GPC3 stably-transfected cells in the logarithmic growth phase were taken, adjusted to 5 × 10⁵ cells/ml using 4% calf serum (Hyclone, SH30626.06), and added to a 96-well U-shaped plate at 100 µl/well cell suspension. The centrifugation was performed at 300 g for 5 min. The supernatant was discarded. 100 µL of gradient diluted antibody (initial concentration: 1800nM, 3-fold dilution, 10 gradients) was added to each well, and incubated at 4°C for 60 min. The secondary antibody Alexa Fluro647 labeled goat anti-human IgG Fc (1: 300 dilution) was added at 50 µL/well and incubated on ice for 20 min. After washing once, propidium iodide solution (1: 300) was added at 50 µL/well. Incubation was performed for 5 min before detection by flow cytometry. FIG. 15 and Table 14 show that the GPC3 × CD3 κλ bispecific antibody can bind with high affinity to the cell GPC3 receptor and that the affinity to monkey GPC3 stably-transfected cell is comparable to the affinity on human GPC3 stably-transfected cell.

**Table 14. Binding of GPC3 × CD3 κλ bispecific antibody to stably-transfected cell**

| EC₅₀ (nM) | Human GPC3-CHO | Monkey GPC3-CHO |
|---|---|---|
| GPC3 x CD3 κλ002 | 2.8 | 3.0 |
| GPC3 x CD3 κλ003 | 2.9 | 3.0 |
| KLH x CD3 | - | - |

### 3. Binding of GPC3 × CD3 κλ bispecific antibody to GPC3+ tumor cells

HepG2 cells in the logarithmic growth phase were taken, added with 200 µg/mL of murine IgG (Jackson ImmunoResearch, 115-005-03), and blocked on an ice bath for 30 min. The cells were adjusted to 5 × 10⁵ cells/mL with 4% calf serum and added to a 96-well U-shaped plate at 100 µl/well. The centrifugation was performed at 300 g for 5 min. The supernatant was discarded. 100 µL of gradient diluted antibody (initial concentration: 1800 nM, 3-fold dilution, 10 gradients) was added to each well, and incubated at 4°C for 60 min. The primary antibody was washed, added with Alexa Fluro647 labeled goat anti-human IgG Fc (1: 300 dilution) 50 µL/well, and incubated on ice for 20 min. After washing once, PI solution was added at 50 µL/well. Incubation was performed for 5 min before detection by flow cytometry. The detection results are shown in FIG. 16 and Table 15. The GPC3 × CD3 κλ bispecific antibody can bind to GPC3+ tumor cell HepG2 with high affinity.

**Table 15. Binding of GPC3 × CD3 κλ bispecific antibody and GPC3+ tumor cell**

| EC₅₀ (nM) | HepG2 |
|---|---|
| GPC3 x CD3 κλ002 | 2.1 |
| GPC3 x CD3 κλ003 | 2.5 |
| KLH x CD3 | - |

### 4. Binding of GPC3 × CD3 κλ bispecific antibody to Jurkat cell

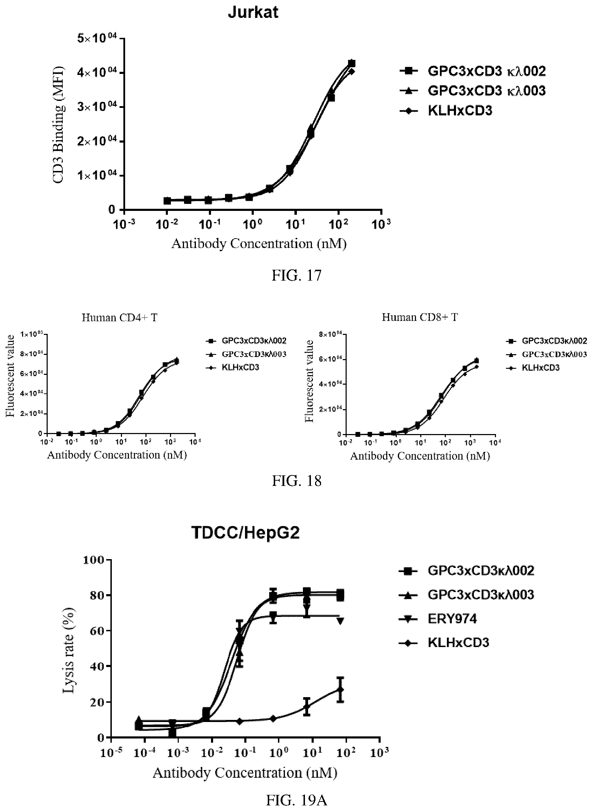

Jurkat cells in the logarithmic growth phase were taken, added with 200 µg/mL of murine IgG (Jackson ImmunoResearch, 115-005-03), and incubated on ice for 30 minutes. The cells were adjusted to 5 × 10⁵ cells/mL with 4% calf serum and added to a 96-well U-shaped plate at 100 µl/well. The centrifugation was performed at 300 g. The supernatant was discarded. 100 µL of gradient diluted antibody (initial concentration: 1800 nM, 3-fold dilution, 10 gradients) was added to each well, and incubated at 4°C for 60 min. The secondary antibody Alexa Fluro647 labeled goat anti-human IgG Fc (1: 300 dilution) was added at 50 µL/well and incubated on ice for 20 min. After washing once, PI solution was added at 50 µL/well. Incubation was performed for 5 min before detection by flow cytometry (BD C6). As shown in FIG. 17 and Table 16, the GPC3 × CD3 κλ bispecific antibody can bind with moderate affinity to human leukemia T cell line Jurkat cell with an EC₅₀ of about 20-40 nM.

**Table 16. Binding of GPC3 × CD3 κλ bispecific antibody to Jurkat cell**

| EC₅₀ (nM) | Jurkat |
|---|---|
| GPC3 x CD3 κλ002 | 36 |
| GPC3 x CD3 κλ003 | 26 |
| KLH x CD3 | 27 |

### 5. Binding of GPC3 × CD3 κλ bispecific antibody to peripheral blood T cell

Fresh human peripheral blood was taken and PBMCs were isolated by Ficoll-Paque Plus (GE, 17-1440-03). The PBMCs were adjusted to 5 × 10⁵ cells/mL with 4% calf serum (Hyclone, SH30626.06), and added to a 96-well U-plate at 100 µL/well. The centrifuged supernatant was discarded. 100 µL of gradient diluted antibody (initial concentration: 1800 nM, 3-fold dilution, 11 gradients) was added to each well and incubated at 4°C for 60 minutes. The secondary antibody Alexa Fluro647 labeled goat anti-human IgG Fc (1: 300 dilution) was added at 50 µL/well and incubated on an ice bath for 20 min. After washing once, PI solution was added at 50 µL/well. Incubation was performed for 5 min before detection by flow cytometry (BD Celesta). The detection results are shown in FIG. 18. The GPC3 × CD3 κλ bispecific antibody can bind to the human peripheral blood T cell with low affinity.

**Table 17. Binding of GPC3 × CD3 κλ bispecific antibody to peripheral blood T cell**

| EC₅₀ (nM) | CD4+ T cell | CD8+ T cell |
|---|---|---|
| GPC3 x CD3 κλ002 | 65 | 65 |
| GPC3 x CD3 κλ003 | 56 | 69 |
| KLH x CD3 | 77 | 84 |

### Example 5. GPC3 x CD3 κλ bispecific antibody-mediated TDCC effect

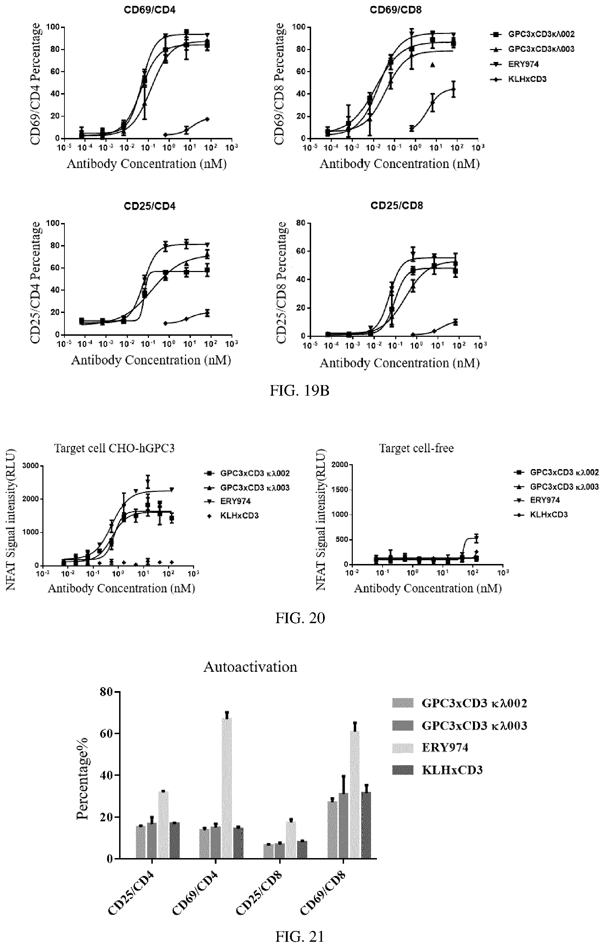

Freshly isolated PBMCs were mixed with target cells, HepG2 cells, in the logarithmic growth phase, respectively, at an effector/target cell ratio = 10: 1. 50 µL of gradient diluted antibody (antibody concentration initially with 66.7nM, 10-fold dilution, 7 gradients) was added to each well and incubated at 37°C, 5% CO₂, for 24 hours. At the end of the incubation, 50 µL of the supernatant was transferred to a new black ELISA plate. 50 µL/well of LDH detection substrate was added. The reaction was stopped after 10 minutes and LDH release was detected; The remaining cells in the wells were washed twice with 4% calf serum, added with 100 µg/mL human IgG, and incubated for 10 minutes. T-cell activation detection antibodies (CD25-BV421, CD4-FITC, CD69-BV605, and CD8-APC) were added and incubated on ice for 20 minutes. After washing, the supernatant was discarded. PI was added at 60 µL/well, and the plate was incubated on ice for 5 minutes and detected by flow cytometry. FIG. 19 shows that the GPC3 x CD3 κλ bispecific antibody can produce slightly higher or equivalent maximal killing of the HepG2 cell than the control antibody in the in vitro activity study (FIG. 19A), but the changes in CD69 (early activation) and CD25 (late activation) of T cells during TDCC suggested that the GPC3 x CD3 κλ bispecific antibody can activate T cells more gently than the control antibody.

### Example 6 Activation of T cell activation pathway by GPC3 x CD3 κλ bispecific antibody

Target cells, CHO-human GPC3, in the logarithmic growth phase, were harvested, and centrifuged. The supernatant was discarded. The cells were resuspended to 2 × 10⁵ cells/ml. The target cells were inoculated into a 96-well plate at 50 µl/well and incubated overnight at 37°C, 5% CO₂. The Jurkat-NFAT-luc reporter cells in the logarithmic growth phase were taken and centrifuged at 300g for 5 min. The supernatant was discarded. The cells were resuspended to 4 × 10⁶ cells/ml. The 96-well plate was taken out and the supernatant was discarded. Jurkat-NFAT-luc reporter cells were inoculated into the 96-well plate at 25 µL/well. 25 µL gradient diluted GPC3 x CD3 κλ bispecific antibody or control antibody KLH x CD3 (initial concentration: 20 µg/ml, 3-fold dilution, 10 gradients) were added into each well, and incubated at 37°C, 5% CO₂ for 6 hours. After the incubation, 100 µL of detection reagent was added to each well according to ONE-Glo Luciferase Assay System instructions for detection in a microplate reader (MD SpectraMax i 3x). The detection results are shown in FIG. 20. When the GPC3 x CD3 κλ bispecific antibody uses CHO-hGPC3 as the target cell, it can activate the NFAT signaling pathway of the T cell, with the activation degree being slightly lower than that of the control antibody.

### Example 7. Non-specific activation of PBMC by GPC3 x CD3 κλ bispecific antibody

Freshly isolated PBMCs were added with 100 µL of antibody (10 µg/mL) and incubated at 37°C, 5% CO₂, for 24 hours. The cells in the wells were washed twice with 4% calf serum, added with 100 µg/mL human IgG, and incubated for 10 minutes. T-cell activation detection antibodies (CD25-BV421, CD4-FITC, CD69-BV605, and CD8-APC) were added and incubated on ice for 20 minutes. After washing, the supernatant was discarded. PI was added at 60 µL/well, and the plate was incubated on ice for 5 minutes and detected by flow cytometry. The results are shown in FIG. 21. In the absence of the target cell, the GPC3 x CD3 κλ bispecific antibody has no activation of peripheral blood T cell, comparable to the negative control KLH x CD3.

### Example 8 Binding of GPC3 x CD3 κλ bispecific antibody to Fc receptor

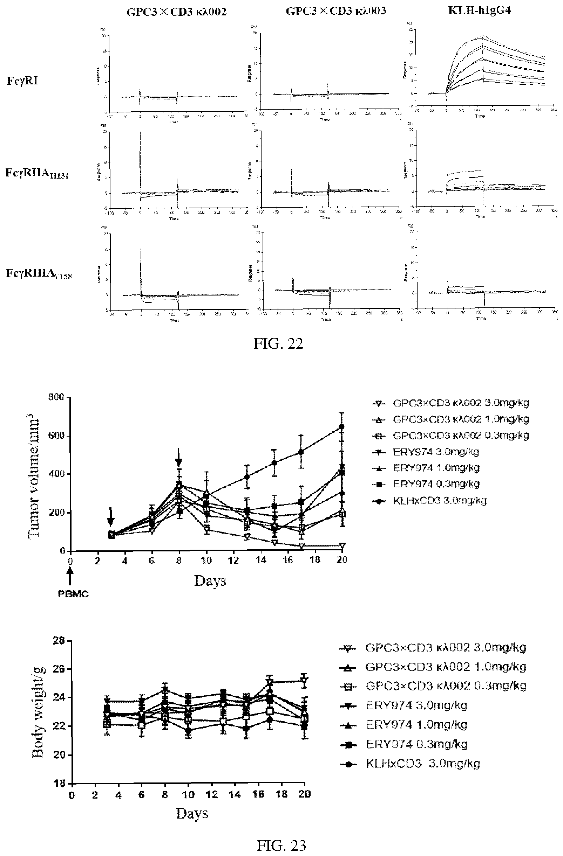

50 µg/mL His-Tag antibodies were conjugated to a CM5 chip through amino groups, and recombinant proteins FcyRI, FcγRIIAH131, and FcγRIIIAV158 with His tags (Sino Biological, #10256-H08H/10374-H08H1/10389-H08H1) were respectively captured for a capture time of 40 s at a flow rate of 10 µL/min. After the baseline was stable, the gradient-diluted antibody (the initial concentration of 37.5 µg/mL, 2-fold dilution) flowed through the chip at a flow rate of 30 µL/min, with a binding time of 120 s and a dissociation time of 200 s. The affinity constants were obtained by fitting them with Biacore evaluation software. It can be seen from FIG. 22 that the GPC3 x CD3 κλ bispecific antibody has no binding to FcyRI, FcγRIIAH131, and FcγRIIIAV158; The wild-type IgG4 control antibody can bind to FcyRI with high affinity and weakly bind to FCγ RIIAH131.

### Example 9. Immune reconstituted mouse models of subcutaneous HepG2 transplantation tumors

6-8-week-old female B-NGD mice (Biocytogen) were selected and subcutaneously inoculated with HepG2 cells (7 × 10⁶/mouse). After the tumor grew to 60-100 mm³, the mice were randomly divided into groups that were respectively set as a 3.0 mg/kg administration group, a 1.0 mg/kg administration group, a 0.3mg/kg administration group, and a negative control group KLH x CD3, 3 mg/kg. 1 × 10⁷ PBMC cells were injected into each mouse via the tail vein. The mice were subjected to administration for the first time 3 days later. The administration was performed once every 5 days and a total of 2 times. The tumor volume and body weight of the mice were monitored. After the experiment was completed, the mice were killed by neck dislocation. The tumor was taken out, weighed, and recorded. The results are shown in FIG. 23. The in vivo efficacy of GPC3 x CD3 κλ bispecific antibody was dose-related, and the tumor inhibition rates (low-dose to high-dose) were: 76.7%, 81.3%, and 95.9%. The tumor-bearing mice tolerated the doses above well, without adverse reactions such as weight loss.

### Example 10. CD3 humanized murine Hepal-6/hGPC3 xenograft model

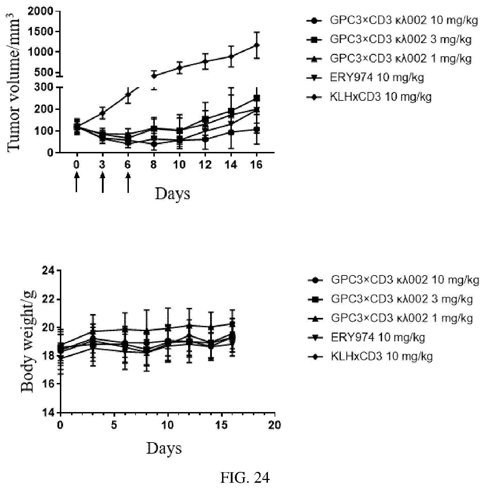

6-week-old female C57/BL6-hCD3 mice (Biocytogen Co. Ltd.) were selected and inoculated subcutaneously with Hepal-6/hGPC3 (6 × 10⁶/mouse). When the tumor volume reached 60-100 mm³, the mice were randomized into groups. The dose groups were set as 10 mg/kg, 3 mg/kg, 1 mg/kg, and negative control group KLH x CD3 10mg/kg, respectively. The dosing interval was once every 3 days for a total of 3 doses. The tumor volume and body weight of the mice were monitored. After the experiment was completed, the mice were killed by neck dislocation. The tumor was taken out, weighed, and recorded. The results are shown in FIG. 24. GPC3 x CD3 κλ bispecific antibody can significantly inhibit Hepal-6/hGPC3 tumor growth and reduce the tumor-bearing volume in the CD3 humanized murine model.

## Claims

1. A bispecific antibody or antigen-binding portion thereof, comprising:
(a) a first antigen-binding portion of a GPC3 antigen or an antigen-binding fragment thereof that binds to the surface of a target cell, the first antigen-binding portion comprising a first heavy chain and a first light chain, the first antigen-binding portion comprising a first binding domain that binds to a first antigen, wherein the first binding domain comprises a heavy chain CDR selected from amino acid sequences of SEQ ID NO. 11, 12, 13, 21, 32, 33, 64, 65, 66, 72, 73, 79, 80, 81, 101, 106 or any variant thereof, and/or a light chain CDR selected from amino acid sequences of SEQ ID NO. 16, 17, 18, 26, 29, 38, 39, 46, 47, 48, 51, 54, 57, 60, 61, 69, 92, 95, 98 or any variant; and
(b) a second antigen binding portion or an antigen binding fragment thereof that binds to a CD3 antigen on the surface of a T cell, the second antigen binding portion comprising a second heavy chain and a second light chain, the second antigen binding portion comprising a second binding domain that binds to a second antigen.

2. The bispecific antibody or antigen binding portion thereof according to claim 1, wherein the first binding domain comprises a heavy chain CDR1 selected from amino acid sequences of SEQ ID NO. 11, 32, 64, 79 or any variant thereof, a heavy chain CDR2 selected from amino acid sequences of SEQ ID NO. 12, 21, 33, 65, 72, 80, 101, 106 or any variant thereof, a heavy chain CDR3 selected from amino acid sequences of SEQ ID NO. 13, 66, 73, 81 or any variant thereof; and/or the first binding domain comprises a light chain CDR1 selected from amino acid sequences of SEQ ID NO. 16, 38, 46, 60 or any variant thereof, a light chain CDR2 selected from amino acid sequences of SEQ ID NO. 17, 47 or any variant thereof, a light chain CDR3 selected from amino acid sequences of SEQ ID NO. 18, 26, 29, 39, 48, 51, 54, 57, 61, 69, 76 or any variant thereof.

3. The bispecific antibody or antigen binding portion thereof according to claim1 or 2, wherein:
the heavy chain CDR1, CDR2, CDR3 sequences of the first binding domain are selected from: a first heavy chain CDR1, CDR2, CDR3 sequences of the amino acid sequences of SEQ ID NO. 11, 12, 13, a first heavy chain CDR1, CDR2, CDR3 sequences of amino acid sequences of SEQ ID NO. 11, 21, 13, a first heavy chain CDR1, CDR2, CDR3 sequences of amino acid sequences of SEQ ID NO. 32, 33, 13, a first heavy chain CDR1, CDR2, CDR3 sequences of amino acid sequences of SEQ ID NO. 64, 65, 66, a first heavy chain CDR1, CDR2, CDR3 sequences of amino acid sequences of SEQ ID NO. 11, 72, 73, a first heavy chain CDR1, CDR2, CDR3 sequences of amino acid sequences of SEQ ID NO. 79, 80, 81, a first heavy chain CDR1, CDR2, CDR3 sequences of amino acid sequences of SEQ ID NO. 11, 101, 13, a first heavy chain CDR1, CDR2, CDR3 sequences of amino acid sequences of SEQ ID NO. 11, 106, 13; and the light chain CDR1, CDR2, CDR3 sequences of the first binding domain are selected from a first light chain CDR1, CDR2, CDR3 sequences of the amino acid sequences SEQ ID NO. 16, 17, 18, a first light chain CDR1, CDR2, CDR3 sequences of amino acid sequences of SEQ ID NO. 16, 17, 26, a first light chain CDR1, CDR2, CDR3 sequences of amino acid sequences of SEQ ID NO. 16, 17, 29, a first light chain CDR1, CDR2, CDR3 sequences of amino acid sequences of SEQ ID NO. 38, 17, 39, a first light chain CDR1, CDR2, CDR3 sequences of amino acid sequences SEQ ID NO. 46, 47, 48, a first light chain CDR1, CDR2, CDR3 sequences of amino acid sequences of SEQ ID NO. 16, 17, 51, a first light chain CDR1, CDR2, CDR3 sequences of amino acid sequences of SEQ ID NO. 16, 17, 54, a first light chain CDR1, CDR2, CDR3 sequences of amino acid sequences of SEQ ID NO. 16, 17, 57, a first light chain CDR1, CDR2, CDR3 sequences of amino acid sequences of SEQ ID NO. 60, 17, 61, a first light chain CDR1, CDR2, CDR3 sequences of amino acid sequences of SEQ ID NO. 16, 17, 69, a first light chain CDR1, CDR2, CDR3 sequences of amino acid sequences of SEQ ID NO. 16, 17, 76, a first light chain CDR1, CDR2, CDR3 sequences of amino acid sequences of SEQ ID NO. 92, 17, 61, a first light chain CDR1, CDR2, CDR3 sequences of amino acid sequences of SEQ ID NO. 95, 17, 61, a first light chain CDR1, CDR2, CDR3 sequences of amino acid sequences of SEQ ID NO. 98, 17, 61;
preferably, the first binding domain comprises the first heavy chain CDR1, CDR2, CDR3 sequences of amino acid sequences of SEQ ID NO. 11, 12, 13 and the first light chain CDR1, CDR2, CDR3 sequences of amino acid sequences of SEQ ID NO. 16, 17, 18;
preferably, the first binding domain comprises the first heavy chain CDR1, CDR2, CDR3 sequences of amino acid sequences of SEQ ID NO. 11, 21, 13 and the first light chain CDR1, CDR2, CDR3 sequences of amino acid sequences of SEQ ID NO. 16, 17, 18; preferably, the first binding domain comprises the first heavy chain CDR1, CDR2, CDR3 sequences of amino acid sequences of SEQ ID NO. 11, 12, 13 and the first light chain CDR1, CDR2, CDR3 sequences of amino acid sequences of SEQ ID NO. 16, 17, 26; preferably, the first binding domain comprises the first heavy chain CDR1, CDR2, CDR3 sequences of amino acid sequences of SEQ ID NO. 11, 12, 13 and the first light chain CDR1, CDR2, CDR3 sequences of amino acid sequences of SEQ ID NO. 16, 17, 29; preferably, the first binding domain comprises the first heavy chain CDR1, CDR2, CDR3 sequences of amino acid sequences of SEQ ID NO. 32, 33, 13 and the first light chain CDR1, CDR2, CDR3 sequences of amino acid sequences of SEQ ID NO. 16, 17, 18; preferably, the first binding domain comprises the first heavy chain CDR1, CDR2, CDR3 sequences of amino acid sequences of SEQ ID NO. 11, 21, 13 and the first light chain CDR1, CDR2, CDR3 sequences of amino acid sequences of SEQ ID NO. 38, 17, 39; preferably, the first binding domain comprises the first heavy chain CDR1, CDR2, CDR3 sequences of amino acid sequences of SEQ ID NO. 11, 21, 13 and the first light chain CDR1, CDR2, CDR3 sequences of amino acid sequences of SEQ ID NO. 16, 17, 18; preferably, the first binding domain comprises the first heavy chain CDR1, CDR2, CDR3 sequences of amino acid sequences of SEQ ID NO. 11, 12, 13 and the first light chain CDR1, CDR2, CDR3 sequences of amino acid sequences of SEQ ID NO. 46, 47, 48; preferably, the first binding domain comprises the first heavy chain CDR1, CDR2, CDR3 sequences of amino acid sequences of SEQ ID NO. 11, 12, 13 and the first light chain CDR1, CDR2, CDR3 sequences of amino acid sequences of SEQ ID NO. 16, 17, 51; preferably, the first binding domain comprises the first heavy chain CDR1, CDR2, CDR3 sequences of amino acid sequences of SEQ ID NO. 11, 12, 13 and the first light chain CDR1, CDR2, CDR3 sequences of amino acid sequences of SEQ ID NO. 16, 17, 54; preferably, the first binding domain comprises the first heavy chain CDR1, CDR2, CDR3 sequences of amino acid sequences of SEQ ID NO. 11, 12, 13 and the first light chain CDR1, CDR2, CDR3 sequences of amino acid sequences of SEQ ID NO. 16, 17, 57; preferably, the first binding domain comprises the first heavy chain CDR1, CDR2, CDR3 sequences of amino acid sequences of SEQ ID NO. 11, 12, 13 and the first light chain CDR1, CDR2, CDR3 sequences of amino acid sequences of SEQ ID NO. 60, 17, 61;
preferably, the first binding domain comprises the first heavy chain CDR1, CDR2, CDR3 sequences of amino acid sequences of SEQ ID NO. 64, 65, 66 and the first light chain CDR1, CDR2, CDR3 sequences of amino acid sequences of SEQ ID NO. 16, 17, 69;
preferably, the first binding domain comprises the first heavy chain CDR1, CDR2, CDR3 sequences of amino acid sequences of SEQ ID NO. 11, 72, 73 and the first light chain CDR1, CDR2, CDR3 sequences of amino acid sequences of SEQ ID NO. 16, 17, 76;
preferably, the first binding domain comprises the first heavy chain CDR1, CDR2, CDR3 sequences of amino acid sequences of SEQ ID NO. 79, 80, 81 and the first light chain CDR1, CDR2, CDR3 sequences of amino acid sequences of SEQ ID NO. 16, 17, 69;
preferably, the first binding domain comprises the first heavy chain CDR1, CDR2, CDR3 sequences of amino acid sequences of SEQ ID NO. 11, 106, 13 and the first light chain CDR1, CDR2, CDR3 sequences of amino acid sequences of SEQ ID NO. 60, 17, 61;
preferably, the first binding domain comprises the first heavy chain CDR1, CDR2, CDR3 sequences of amino acid sequences of SEQ ID NO. 11, 106, 13 and the first light chain CDR1, CDR2, CDR3 sequences of amino acid sequences of SEQ ID NO. 92, 17, 61;
preferably, the first binding domain comprises the first heavy chain CDR1, CDR2, CDR3 sequences of amino acid sequences of SEQ ID NO. 11, 106, 13 and the first light chain CDR1, CDR2, CDR3 sequences of amino acid sequences of SEQ ID NO. 95, 17, 61;
preferably, the first binding domain comprises the first heavy chain CDR1, CDR2, CDR3 sequences of amino acid sequences of SEQ ID NO. 11, 106, 13 and the first light chain CDR1, CDR2, CDR3 sequences of amino acid sequences of SEQ ID NO. 98, 17, 61.

4. The bispecific antibody or antigen binding portion thereof according to any one of claims 1-3, wherein the first binding domain comprises a first heavy chain variable region selected from amino acid sequences of SEQ ID NO. 9, 19, 30, 40, 62, 70, 77, 99, 102, 104, 107, 109, 111 or any variant thereof and/or comprises a first light chain variable region selected from amino acid sequences of SEQ ID NO. 14, 22, 24, 27, 34, 36, 42, 44, 49, 52, 55, 58, 67, 74, 82, 84, 86, 88, 90, 93, 96 or any variant thereof;
preferably, the first binding domain comprises the first heavy chain variable region of the amino acid sequence of SEQ ID NO. 9 or any variant thereof, and the first light chain variable region of the amino acid sequence of SEQ ID NO. 14 or any variant thereof; preferably, the first binding domain comprises the first heavy chain variable region of the amino acid sequence of SEQ ID NO. 19 or any variant thereof, and the first light chain variable region of the amino acid sequence of SEQ ID NO. 22 or any variant thereof; preferably, the first binding domain comprises the first heavy chain variable region of the amino acid sequence of SEQ ID NO. 9 or any variant thereof, and the first light chain variable region of the amino acid sequence of SEQ ID NO. 24 or any variant thereof; preferably, the first binding domain comprises the first heavy chain variable region of the amino acid sequence of SEQ ID NO. 9 or any variant thereof, and the first light chain variable region of the amino acid sequence of SEQ ID NO. 27 or any variant thereof; preferably, the first binding domain comprises the first heavy chain variable region of the amino acid sequence of SEQ ID NO. 30 or any variant thereof, and the first light chain variable region of the amino acid sequence of SEQ ID NO. 34 or any variant thereof; preferably, the first binding domain comprises the first heavy chain variable region of the amino acid sequence of SEQ ID NO. 19 or any variant thereof, and the first light chain variable region of the amino acid sequence of SEQ ID NO. 36 or any variant thereof; preferably, the first binding domain comprises the first heavy chain variable region of the amino acid sequence of SEQ ID NO. 40 or any variant thereof, and the first light chain variable region of the amino acid sequence of SEQ ID NO. 42 or any variant thereof; preferably, the first binding domain comprises the first heavy chain variable region of the amino acid sequence of SEQ ID NO. 9 or any variant thereof, and the first light chain variable region of the amino acid sequence of SEQ ID NO. 44 or any variant thereof; preferably, the first binding domain comprises the first heavy chain variable region of the amino acid sequence of SEQ ID NO. 9 or any variant thereof, and the first light chain variable region of the amino acid sequence of SEQ ID NO. 49 or any variant thereof; preferably, the first binding domain comprises the first heavy chain variable region of the amino acid sequence of SEQ ID NO. 9 or any variant thereof, and the first light chain variable region of the amino acid sequence of SEQ ID NO. 52 or any variant thereof; preferably, the first binding domain comprises the first heavy chain variable region of the amino acid sequence of SEQ ID NO. 9 or any variant thereof, and the first light chain variable region of the amino acid sequence of SEQ ID NO. 55 or any variant thereof;
preferably, the first binding domain comprises the first heavy chain variable region of the amino acid sequence of SEQ ID NO. 9 or any variant thereof, and the first light chain variable region of the amino acid sequence of SEQ ID NO. 58 or any variant thereof;
preferably, the first binding domain comprises the first heavy chain variable region of the amino acid sequence of SEQ ID NO. 62 or any variant thereof, and the first light chain variable region of the amino acid sequence of SEQ ID NO. 67 or any variant thereof;
preferably, the first binding domain comprises the first heavy chain variable region of the amino acid sequence of SEQ ID NO. 70 or any variant thereof, and the first light chain variable region of the amino acid sequence of SEQ ID NO. 74 or any variant thereof;
preferably, the first binding domain comprises the first heavy chain variable region of the amino acid sequence of SEQ ID NO. 77 or any variant thereof, and the first light chain variable region of the amino acid sequence of SEQ ID NO. 82 or any variant thereof;
preferably, the first binding domain comprises the first heavy chain variable region of the amino acid sequence of SEQ ID NO. 111 or any variant thereof, and the first light chain variable region of the amino acid sequence of SEQ ID NO. 86 or any variant thereof;
preferably, the first binding domain comprises the first heavy chain variable region of the amino acid sequence of SEQ ID NO. 111 or any variant thereof, and the first light chain variable region of the amino acid sequence of SEQ ID NO. 88 or any variant thereof;
preferably, the first binding domain comprises the first heavy chain variable region of the amino acid sequence of SEQ ID NO. 111 or any variant thereof, and the first light chain variable region of the amino acid sequence of SEQ ID NO. 90 or any variant thereof;
preferably, the first binding domain comprises the first heavy chain variable region of the amino acid sequence of SEQ ID NO. 111 or any variant thereof, and the first light chain variable region of the amino acid sequence of SEQ ID NO. 93 or any variant thereof;
preferably, the first binding domain comprises the first heavy chain variable region of the amino acid sequence of SEQ ID NO. 111 or any variant thereof, and the first light chain variable region of the amino acid sequence of SEQ ID NO. 96 or any variant thereof.

5. The bispecific antibody or antigen binding portion thereof according to any one of claims 1-4, wherein the second binding domain is selected from a heavy chain CDR selected from amino acid sequences of SEQ ID NO. 134, 135, 136, 139, 142, 145, 148, 151, 154, 155 or any variant thereof; and/or comprising a light chain CDR selected from amino acid sequences of SEQ ID NO. 115, 116, 117, 122, 123, 128, 131 or any variant thereof;
preferably, the second binding domain comprises the second heavy chain CDR1 selected from the amino acid sequences of SEQ ID NO. 134, 139, 154 or any variant thereof, the second heavy chain CDR2 selected from the amino acid sequences of SEQ ID NO. 135,155 or any variant thereof, the second heavy chain CDR3 selected from the amino acid sequences of SEQ ID NO. 136, 142, 145, 148, 151 or any variant thereof; and/or the second light chain CDR1 selected from the amino acid sequences of SEQ ID NO. 115, 122 or any variant thereof, the second light chain CDR2 selected from the amino acid sequences of SEQ ID NO. 116, 123, 131 or any variant thereof, the second light chain CDR3 selected from the amino acid sequences of SEQ ID NO. 117, 128 or any variant thereof;
preferably, the heavy chain CDR1, CDR2, CDR3 sequences of the second binding domain are selected from: the second heavy chain CDR1, CDR2, CDR3 sequences of the amino acid sequences of SEQ ID NO. 134, 135, 136, the second heavy chain CDR1, CDR2, CDR3 sequences of the amino acid sequences of SEQ ID NO. 139, 135, 136, the second heavy chain CDR1, CDR2, CDR3 sequences of the amino acid sequences of SEQ ID NO.139, 135, 142, the second heavy chain CDR1, CDR2, CDR3 sequences of the amino acid sequences SEQ ID NO. 139, 135, 145, the second heavy chain CDR1, CDR2, CDR3 sequences of the amino acid sequences of SEQ ID NO. 139, 135, 148, the second heavy chain CDR1, CDR2, CDR3 sequences of the amino acid sequences SEQ ID NO. 139, 135, 151, the second heavy chain CDR1, CDR2, CDR3 sequences of the amino acid sequences SEQ ID NO. 154, 155, 136; and the light chain CDR1, CDR2, CDR3 sequences of the second binding domain are selected from: the second light chain CDR1, CDR2, CDR3 sequences of the amino acid sequences of SEQ ID NO. 115, 116, 117, the second light chain CDR1, CDR2, CDR3 sequences of the amino acid sequences of SEQ ID NO. 122, 123, 117, the second light chain CDR1, CDR2, CDR3 sequences of the amino acid sequences SEQ ID NO. 115, 116, 128, the second light chain CDR1, CDR2, CDR3 sequences of the amino acid sequences of SEQ ID NO. 115, 131, 128;
preferably, the second binding domain comprises the second heavy chain CDR1, CDR2, CDR3 sequences of the amino acid sequences of SEQ ID NO. 134, 135, 136, the second light chain CDR1, CDR2, CDR3 sequences of the amino acid sequences of SEQ ID NO. 115, 116, 128;
preferably, the second binding domain comprises the second heavy chain CDR1, CDR2, CDR3 sequences of the amino acid sequences of SEQ ID NO. 134, 135, 136, the second light chain CDR1, CDR2, CDR3 sequences of the amino acid sequences of SEQ ID NO. 115, 116, 117;
preferably, the second binding domain comprises the second heavy chain CDR1, CDR2, CDR3 sequences of the amino acid sequences of SEQ ID NO. 134, 135, 136, the second light chain CDR1, CDR2, CDR3 sequences of the amino acid sequences of SEQ ID NO. 122, 123, 117;
preferably, the second binding domain comprises the second heavy chain variable region selected from the amino acid sequences of SEQ ID NO. 132, 137, 140, 143, 146, 149, 152, 156, 158, 160 or any variant thereof, and/or comprises the second light chain variable region selected from the amino acid sequences of SEQ ID NO. 113, 118, 120, 124, 126, 129 or any variant thereof;
preferably, the second binding domain comprises the second heavy chain variable region of the amino acid sequence of SEQ ID NO. 132 or any variant thereof, and the second light chain variable region of the amino acid sequences of SEQ ID NO. 126 or any variant thereof;
preferably, the second binding domain comprises the second heavy chain variable region of the amino acid sequence of SEQ ID NO. 156 or any variant thereof, and the second light chain variable region of the amino acid sequences of SEQ ID NO. 113 or any variant thereof;
preferably, the second binding domain comprises the second heavy chain variable region of the amino acid sequence of SEQ ID NO. 156 or any variant thereof, and the second light chain variable region of the amino acid sequences of SEQ ID NO. 126 or any variant thereof;
preferably, the second binding domain comprises the second heavy chain variable region of the amino acid sequence of SEQ ID NO. 158 or any variant thereof, and the second light chain variable region of the amino acid sequences of SEQ ID NO. 113 or any variant thereof;
preferably, the second binding domain comprises the second heavy chain variable region of the amino acid sequence of SEQ ID NO. 158 or any variant thereof, and the second light chain variable region of the amino acid sequences of SEQ ID NO. 118 or any variant thereof;
preferably, the second binding domain comprises the second heavy chain variable region of the amino acid sequence of SEQ ID NO. 158 or any variant thereof, and the second light chain variable region of the amino acid sequences of SEQ ID NO. 120 or any variant thereof;
preferably, the second binding domain comprises the second heavy chain variable region of the amino acid sequence of SEQ ID NO. 158 or any variant thereof, and the second light chain variable region of the amino acid sequences of SEQ ID NO. 126 or any variant thereof.

6. The bispecific antibody or antigen binding portion thereof according to any one of claims 1-4, wherein the first light chain of the first antigen binding portion is a kappa type light chain and the second light chain of the second antigen binding portion is a lambda type light chain,
preferably, the first light chain variable region of the first antigen binding portion has a Gln₄₃Lys mutation (Vκ_{GPC3}: Gln₄₃Lys); preferably, the first heavy chain variable region of the first antigen-binding portion has a Gln₃₉Glu (VH_{GPC3}: Gln₃₉Glu) mutation;
preferably, the second light chain variable region of the second antigen-binding portion has a Gln₄₀Glu mutation (Vλ_{CD3}: Gln₄oGlu); the second heavy chain variable region of the second antigen-binding portion has a Gln₃₉Lys mutation (VH_{CD3}: Gln₃₉Lys);
preferably, the Fc portion of the first antigen-binding portion and the second antigen-binding portion of the bispecific antibody adopts a knob-into-hole structure; preferably, a human IgG4 knob-into-hole structure is adopted.

7. The bispecific antibody or antigen binding portion thereof according to any one of claims 1-6, wherein the first heavy chain comprises a heavy chain selected from SEQ ID NO. 164, 172 or any variant thereof and the first light chain comprises a light chain selected from SEQ ID NO. 162, 170 or any variant thereof;
preferably, the first heavy chain comprises the heavy chain selected from SEQ ID NO. 164 or any variant thereof and the first light chain comprises the light chain selected from SEQ ID NO. 162 or any variant thereof;
preferably, the first heavy chain comprises the heavy chain selected from SEQ ID NO. 172 or any variant thereof and the first light chain comprises the light chain selected from SEQ ID NO. 170 or any variant thereof.

8. The bispecific antibody or antigen binding portion thereof according to any one of claims 1-7, wherein the second binding domain comprises the second heavy chain variable region of the amino acid sequence of SEQ ID NO. 158 or any variant thereof, and the second light chain variable region of the amino acid sequence of SEQ ID NO. 126 or any variant thereof;
preferably, the second heavy chain comprises the heavy chain of the amino acid sequence of SEQ ID NO. 168 or any variant thereof and the second light chain comprises the light chain of the amino acid sequence of SEQ ID NO. 166 or any variant thereof;
preferably, the first heavy chain of the bispecific antibody comprises the heavy chain of the amino acid sequence of SEQ ID NO. 164 or any variant thereof and the first light chain of the bispecific antibody comprises the light chain of the amino acid sequence of SEQ ID NO. 162 or any variant thereof; the second heavy chain comprises the heavy chain of the amino acid sequence of SEQ ID NO. 168 or any variant thereof and the second light chain comprises the light chain of the amino acid sequence of SEQ ID NO. 166 or any variant thereof;
preferably, the first heavy chain comprises the heavy chain of the amino acid sequence of SEQ ID NO. 172 or any variant thereof and the first light chain of the bispecific antibody comprises the light chain of the amino acid sequence SEQ ID NO. 170 or any variant thereof; the second heavy chain comprises the heavy chain of the amino acid sequence of SEQ ID NO. 168 or any variant thereof and the second light chain comprises the light chain of the amino acid sequence SEQ ID NO. 166 or any variant thereof.

9. An antibody or antigen-binding portion thereof that binds to GPC3, the antibody comprising a first antigen-binding portion according to any one of claims 1-8.

10. A nucleic acid encoding a bispecific antibody or antigen binding portion thereof according to any one of claims 1-8 or an antibody or antigen binding portion thereof that binds to GPC3according to claim 9;
preferably, the nucleic acid encoding the first heavy chain variable region of the first antigen binding portion is selected from nucleotide sequences of SEQ ID NO. 10, 20, 31, 41, 63, 71, 78, 100, 103, 105, 108, 110, 112 or any variant thereof; and/or the nucleic acid encoding the first light chain variable region of the first antigen binding portion is selected from nucleotide sequences of SEQ ID NO. 15, 23, 25, 28, 35, 37, 43, 45, 50, 53, 56, 59, 68, 75, 83, 85, 87, 89, 91, 94, 97 or any variant thereof;
more preferably, the nucleic acid encoding the first heavy chain variable region of the first antigen-binding portion is the nucleotide sequence of SEQ ID NO. 10; and the nucleic acid encoding the first light chain variable region is the nucleotide sequence of SEQ ID NO. 15;
more preferably, the nucleic acid encoding the first heavy chain variable region of the first antigen-binding portion is the nucleotide sequence of SEQ ID NO. 20; and the nucleic acid encoding the first light chain variable region is the nucleotide sequence of SEQ ID NO. 23;
more preferably, the nucleic acid encoding the first heavy chain variable region of the first antigen-binding portion is the nucleotide sequence of SEQ ID NO. 10; and the nucleic acid encoding the first light chain variable region is the nucleotide sequence of SEQ ID NO. 25;
more preferably, the nucleic acid encoding the first heavy chain variable region of the first antigen-binding portion is the nucleotide sequence of SEQ ID NO. 10; and the nucleic acid encoding the first light chain variable region is the nucleotide sequence of SEQ ID NO. 28;
more preferably, the nucleic acid encoding the first heavy chain variable region of the first antigen-binding portion is the nucleotide sequence of SEQ ID NO. 31; and the nucleic acid encoding the first light chain variable region is the nucleotide sequence of SEQ ID NO. 35;
more preferably, the nucleic acid encoding the first heavy chain variable region of the first antigen-binding portion is the nucleotide sequence of SEQ ID NO. 20; and the nucleic acid encoding the first light chain variable region is the nucleotide sequence of SEQ ID NO. 37;
more preferably, the nucleic acid encoding the first heavy chain variable region of the first antigen-binding portion is the nucleotide sequence of SEQ ID NO. 41; and the nucleic acid encoding the first light chain variable region is the nucleotide sequence of SEQ ID NO. 43;
more preferably, the nucleic acid encoding the first heavy chain variable region of the first antigen-binding portion is the nucleotide sequence of SEQ ID NO. 10; and the nucleic acid encoding the first light chain variable region is the nucleotide sequence of SEQ ID NO. 45;
more preferably, the nucleic acid encoding the first heavy chain variable region of the first antigen-binding portion is the nucleotide sequence of SEQ ID NO. 10; and the nucleic acid encoding the first light chain variable region is the nucleotide sequence of SEQ ID NO. 50;
more preferably, the nucleic acid encoding the first heavy chain variable region of the first antigen-binding portion is the nucleotide sequence of SEQ ID NO. 10; and the nucleic acid encoding the first light chain variable region is the nucleotide sequence of SEQ ID NO. 53;
more preferably, the nucleic acid encoding the first heavy chain variable region of the first antigen-binding portion is the nucleotide sequence of SEQ ID NO. 10; and the nucleic acid encoding the first light chain variable region is the nucleotide sequence of SEQ ID NO. 56;
more preferably, the nucleic acid encoding the first heavy chain variable region of the first antigen-binding portion is the nucleotide sequence of SEQ ID NO. 10; and the nucleic acid encoding the first light chain variable region is the nucleotide sequence of SEQ ID NO. 59;
more preferably, the nucleic acid encoding the first heavy chain variable region of the first antigen-binding portion is the nucleotide sequence of SEQ ID NO. 63; and the nucleic acid encoding the first light chain variable region is the nucleotide sequence of SEQ ID NO. 68;
more preferably, the nucleic acid encoding the first heavy chain variable region of the first antigen-binding portion is the nucleotide sequence of SEQ ID NO. 71; and the nucleic acid encoding the first light chain variable region is the nucleotide sequence of SEQ ID NO. 75;
more preferably, the nucleic acid encoding the first heavy chain variable region of the first antigen-binding portion is the nucleotide sequence of SEQ ID NO. 78; and the nucleic acid encoding the first light chain variable region is the nucleotide sequence of SEQ ID NO. 83;
more preferably, the nucleic acid encoding the first heavy chain variable region of the first antigen-binding portion is the nucleotide sequence of SEQ ID NO. 112; and the nucleic acid encoding the first light chain variable region is the nucleotide sequence of SEQ ID NO. 87;
more preferably, the nucleic acid encoding the first heavy chain variable region of the first antigen-binding portion is the nucleotide sequence of SEQ ID NO. 112; and the nucleic acid encoding the first light chain variable region is the nucleotide sequence of SEQ ID NO. 89;
more preferably, the nucleic acid encoding the first heavy chain variable region of the first antigen-binding portion is the nucleotide sequence of SEQ ID NO. 112; and the nucleic acid encoding the first light chain variable region is the nucleotide sequence of SEQ ID NO. 91;
more preferably, the nucleic acid encoding the first heavy chain variable region of the first antigen-binding portion is the nucleotide sequence of SEQ ID NO. 112; and the nucleic acid encoding the first light chain variable region is the nucleotide sequence of SEQ ID NO. 94;
more preferably, the nucleic acid encoding the first heavy chain variable region of the first antigen-binding portion is the nucleotide sequence of SEQ ID NO. 112; and the nucleic acid encoding the first light chain variable region is the nucleotide sequence of SEQ ID NO. 97;
preferably, the nucleic acid encoding the first heavy chain of the first antigen-binding portion is selected from the nucleotide sequences of SEQ ID NO. 165, 173 or any variant thereof; and/or the nucleic acid encoding the first light chain of the first antigen binding portion is selected from the nucleotide sequences of SEQ ID NO. 163, 171 or any variant thereof;
more preferably, the nucleic acid encoding the first heavy chain of the first antigen-binding portion is the nucleotide sequence of SEQ ID NO. 165 and the nucleic acid encoding the first light chain of the first antigen-binding portion is the nucleotide sequence of SEQ ID NO. 163;
more preferably, the nucleic acid encoding the first heavy chain of the first antigen-binding portion is the nucleotide sequence of SEQ ID NO. 173 and the nucleic acid encoding the first light chain of the first antigen-binding portion is the nucleotide sequence of SEQ ID NO. 171;
preferably, the nucleic acid encoding the second heavy chain variable region of the second antigen-binding portion is selected from the nucleotide sequences of SEQ ID NO. 133, 138, 141, 144, 147, 150, 153, 157, 159, 161 or any variant thereof; and/or the nucleic acid encoding the second light chain variable region of the second antigen binding portion is selected from the nucleotide sequences of SEQ ID NO. 114, 119, 121, 125, 127, 130 or any variant thereof;
more preferably, the nucleic acid encoding the second heavy chain variable region of the second antigen-binding portion is the nucleotide sequence of SEQ ID NO. 133; and the nucleic acid encoding the second light chain variable region is the nucleotide sequence of SEQ ID NO. 127;
more preferably, the nucleic acid encoding the second heavy chain variable region of the second antigen-binding portion is the nucleotide sequence of SEQ ID NO. 157; and the nucleic acid encoding the second light chain variable region is the nucleotide sequence of SEQ ID NO. 114;
more preferably, the nucleic acid encoding the second heavy chain variable region of the second antigen-binding portion is the nucleotide sequence of SEQ ID NO. 157; and the nucleic acid encoding the second light chain variable region is the nucleotide sequence of SEQ ID NO. 127;
more preferably, the nucleic acid encoding the second heavy chain variable region of the second antigen-binding portion is the nucleotide sequence of SEQ ID NO. 159; and the nucleic acid encoding the second light chain variable region is the nucleotide sequence of SEQ ID NO. 114;
more preferably, the nucleic acid encoding the second heavy chain variable region of the second antigen-binding portion is the nucleotide sequence of SEQ ID NO. 159; and the nucleic acid encoding the second light chain variable region is the nucleotide sequence of SEQ ID NO. 119;
more preferably, the nucleic acid encoding the second heavy chain variable region of the second antigen-binding portion is the nucleotide sequence of SEQ ID NO. 159; and the nucleic acid encoding the second light chain variable region is the nucleotide sequence of SEQ ID NO. 121;
more preferably, the nucleic acid encoding the second heavy chain variable region of the second antigen-binding portion is the nucleotide sequence of SEQ ID NO. 159; and the nucleic acid encoding the second light chain variable region is the nucleotide sequence of SEQ ID NO. 127;
preferably, the nucleic acid encoding the second heavy chain of the second antigen-binding portion is the nucleotide sequence of SEQ ID NO. 169 or any variant thereof; and/or the nucleic acid encoding the second light chain of the second antigen-binding portion is the nucleotide sequence SEQ ID NO. 167 or any variant thereof;
more preferably, the nucleic acid encoding the second heavy chain of the second antigen-binding portion is the nucleotide sequence of SEQ ID NO. 169 and the nucleic acid encoding the second light chain of the second antigen-binding portion is the nucleotide sequence of SEQ ID NO. 167;
more preferably, the nucleic acid encoding the first heavy chain of the first antigen-binding portion of the bispecific antibody is the nucleotide sequence of SEQ ID NO. 165, and the nucleic acid encoding the first light chain of the first antigen-binding portion is the nucleotide sequence of SEQ ID NO. 163; the nucleic acid encoding the second heavy chain of the second antigen-binding portion is the nucleotide sequence of SEQ ID NO. 169, and the nucleic acid encoding the second light chain of the second antigen-binding portion is the nucleotide sequence of SEQ ID NO. 167;
more preferably, the nucleic acid encoding the first heavy chain of the first antigen-binding portion of the bispecific antibody is the nucleotide sequence of SEQ ID NO. 173, and the nucleic acid encoding the first light chain of the first antigen-binding portion is the nucleotide sequence of SEQ ID NO. 171; the nucleic acid encoding the second heavy chain of the second antigen-binding portion is the nucleotide sequence of SEQ ID NO. 169, and the nucleic acid encoding the second light chain of the second antigen-binding portion is the nucleotide sequence of SEQ ID NO. 167.

11. A vector containing the nucleic acid according to claim 10.

12. A cell containing the nucleic acid according to claim 9 or the vector according to claim 11.

13. A composition comprising the bispecific antibody or antigen binding portion thereof according to any one of claims 1-8, the antibody or antigen binding portion thereof that binds to GPC3 according to claim 9, the nucleic acid according to claim 10, the vector according to claim 11, and/or the cell according to claim 12.

14. An antibody-drug conjugate comprising the bispecific antibody or antigen-binding portion thereof according to any one of claims 1-8 or the antibody or antigen binding portion thereof that binds to GPC3 according to claim 9 covalently attached to a therapeutic moiety;
preferably, the therapeutic moiety is selected from a cytotoxic moiety, a chemotherapeutic agent, a cytokine, an immunosuppressant, an immunostimulant, a lytic peptide, or a radioisotope;
preferably, the cytotoxic moiety is selected from: paclitaxel; cytochalasin B; gramicidin D; ethidium bromide; emetine; mitomycin; etoposide; teniposide; vincristine; vinblastine; colchicine; doxorubicin; daunorubicin; dihydroxy anthrenedione; a tubulin inhibitor such as maytansine and an analog or derivative thereof; an antimitotic agent such as monomethyl auristatins E and F and analogs or derivatives thereof; aplysiatoxins 10 and 15 and analogs thereof; irinotecan and an analog thereof; mitoxantrone; mithramycin; actinomycin D; 1-dehydrotestosterone; a glucocorticoid; procaine; tetracaine; lidocaine; propranolol; puromycin; calicheamicin and an analog or derivative thereof; an anti-metabolite such as methotrexate, 6-mercaptopurine, 6-thioguanine, cytarabine, fludarabine, 5-fluorouracil, decanedizine, hydroxycarbamide, asparaginase, gemcitabine, and cladribine; an alkylating agent such as dichloromethyldiethylamine, a thiopurine, chlorambucil, melphalan, carmustine (BSNU), lomustine (CCNU), cyclophosphamide, busulfan, mitobronitol, streptozotocin, dacarbazine (DTIC), procarbazine, and mitomycin C; a platinum derivative such as cisplatin or carboplatin; duocarmycin A, duocarmycin SA, rapamycin (CC-1065), and analogs or derivatives thereof; an antibiotic such as actinomycin, bleomycin, daunorubicin, doxorubicin, idarubicin, photomycin, mitomycin, mitoxantrone, perimycin, and diazepam (AMC); pyrrolo[2,1-c][1,4]-benzodiazepine (PDB); diphtheria toxin and related molecules such as diphtheria A chain and an active fragment and a hybrid molecule thereof, ricin such as ricin A and deglycosylated ricin A chain toxin, cholera toxin, a Shiga-like toxin such as SLT I, SLT II and SLT IIV, LT toxin, C3 toxin, a Shiga toxin, pertussis toxin, tetanus toxin, a soybean Bowman-Birk protease inhibitor, Pseudomonas exotoxin, aroline, saporin, Adenia heterophylla root toxin, gelsolin, abrin A chain, Adenia heterophylla root A chain, α-sarcin, Aleurites fordii protein, caryophyllin protein, a Phytolacca americana protein such as PAPI, PAPII and PAP-S, a Momordica charantia inhibitor, curcin, crotin, a Sapaonaria officinalis inhibitor, gelonin, mitomycin, restrictocin, phenomycin, and enomycin toxin; ribonuclease (RNase); DNase I and Staphylococcus aureus endotoxin A; Phytolacca acinosa antiviral protein; and diphtheria toxin and Pseudomonas endotoxin;
preferably, the cytokine is selected from IL-2, IL-4, IL-6, IL-7, IL-10, IL-12, IL-13, IL-15, IL-18, IL-23, IL-24, IL-27, IL-28a, IL-28b, IL-29, KGF, IFN α, IFN β, IFN γ, GM-CSF, CD40L, Flt3 ligand, stem cell factor, ancestine, and TNF α;
preferably, the radioisotope is selected from ³H, ¹⁴C, ¹⁵N, ³⁵S, ⁶⁷Cu, ⁹⁰Y, ⁹⁹Tc, ¹²⁵I, ¹³¹I, ¹⁸⁶Re, ¹⁸⁸Re , ²¹¹At, ²¹²Bi, ²¹²Pb , ²¹³Bi, ²²⁵Ac and ²²⁷Th.

15. A kit comprising the specific antibody or antigen binding portion thereof according to any one of claims 1-8, the antibody or antigen binding portion thereof that binds to GPC3 according to claim 9, the nucleic acid molecule according to claim 10, the vector according to claim 11, the cell according to claim 12, the composition according to claim 13 and/or the antibody-drug conjugate according to claim 14.

16. Use of the specific antibody or antigen binding portion thereof according to any one of claims 1-8, an antibody or antigen binding portion thereof that binds to GPC3 according to claim 9, a nucleic acid molecule according to claim 10, a vector according to claim 11, a cell according to claim 12, a composition according to claim 13 and/or an antibody-drug conjugate according to claim 14 in the manufacture of a medicament or kit for the diagnosis, treatment or prevention of a GPC3-associated disease;
preferably, the GPC3-associated disease includes tumors;
preferably, the tumor is a cancer; More preferably, the cancer is a GPC3 positive cancer, e.g. a GPC3 positive liver cancer, a GPC3 positive hepatocellular carcinoma, a GPC positive pancreatic cancer, a GPC positive lung cancer, a GPC positive colon cancer, a GPC positive breast cancer, a GPC positive prostate cancer, a GPC positive leukemia, or a GPC positive lymphoma.
